# EUROPEAN PATENT SPECIFICATION

(11) **EP 2 291 525 B1**
(45) Date of publication and mention of the grant of the patent: **15.04.2015**
(21) Application number: 09749936.2
(22) Date of filing: 25.05.2009
(51) Int. Cl.: C12N 15/82

(54) **TRANSGENIC SUGAR BEET PLANTS**
TRANSGENE ZUCKERRÜBEN
MARQUEURS DE POLYNUCLÉOTIDES

(30) Priority: 23.05.2008 WO PCT/EP2008/056390
(43) Date of publication of application: 09.03.2011
(73) Proprietor: Syngenta Participations AG, 4058 Basel (CH)
(72) Inventor: GIELEN, Johannes Jacobus Ludgerus, F-31620 Bouloc (FR); KRAFT, Thomas, S-22733 Lund (SE); PIN, Pierre, S-21741 Malmö (SE)
(74) Representative: Syngenta International AG
(86) International application number: PCT/EP2009/056262
(87) International publication number: WO 2009/141446

(56) References cited:
- WO-A-2008/142167
- DATABASE EMBL/GENBANK/DDBJ 24 September 2004 (2004-09-24), MCGRATH, J. MITCHELL: "MM5_H06 young roots probed with 3 and 7 week old root enriched transcripts, Beta vulgaris cDNA, mRNA sequence." XP002535976 retrieved from EBI Database accession no. CV301305
- GAAFAR R M ET AL: "Bacterial artificial chromosome-derived molecular markers for early bolting in sugar beet" THEORETICAL AND APPLIED GENETICS ; INTERNATIONAL JOURNAL OF PLANT BREEDING RESEARCH, SPRINGER-VERLAG, BE, vol. 110, no. 6, 1 April 2005 (2005-04-01), pages 1027-1037, XP019321879 ISSN: 1432-2242 cited in the application
- HOHMANN U ET AL: "A bacterial artificial chromosome (BAC) library of sugar beet and a physical map of the region encompassing the bolting gene B." MGG MOLECULAR GENETICS AND GENOMICS, vol. 269, no. 1, April 2003 (2003-04), pages 126-136, XP002487656 ISSN: 1617-4615
- EL-MEZAWY A ET AL: "High-resolution mapping of the bolting gene B of sugar beet" THEORETICAL AND APPLIED GENETICS, vol. 105, no. 1, July 2002 (2002-07), pages 100-105, XP002487538 ISSN: 0040-5752
- PIERREA PIN ET AL: "The Role of a Pseudo-Response Regulator Gene in Life Cycle Adaptation and Domestication of Beet", CURRENT BIOLOGY, CURRENT SCIENCE, GB, vol. 22, no. 12, 2 April 2012 (2012-04-02) , pages 1095-1101, XP028494550, ISSN: 0960-9822, DOI: 10.1016/J.CUB.2012.04.007 [retrieved on 2012-04-06]

## Description

The present invention relates generally to the field of plant molecular biology, plant transformation, and plant breeding. More specifically, the invention relates to transgenic sugar beet plants having a phenotype of delayed bolting. The invention further relates to polynucleotide markers that are closely linked to or residing within the bolting gene or *B* gene within the sugar beet genome and can be used for the discrimination between the annual and biennial genotype or between different haplotypes within plant groupings of sugar beet plants exhibiting a biennial genotype.

The cultivated sugar beet (*Beta vulgaris* ssp. *vulgaris* L.) is a biennial plant which forms a storage root and a leaf rosette in the first year. Shoot elongation (bolting) and flower formation starts after a period of low temperature. In contrast, many wild beets of the genus *B. vulgaris* ssp. *maritima* show an annual growing habit due to the presence of the bolting gene *B* at the *B* locus, which was mapped to the central region of chromosome II. The *BOLTING* gene (*B* gene) is responsible for the determination of the annual habit in sugar beet. Annuality in the *Beta* species is considered a monogenic and dominant trait. Plants carrying the dominant *B* allele are able to switch from juvenile to reproductive stages in a vernalization-independent manner, contrary to biennial plants carrying the b allele that obligatory require vernalization for bolting and subsequent flowering to occur. The dominant allele of locus B is abundant in wild beets and causes bolting under long days without the cold requirement usually essential for biennial cultivars (Abe *et al.,* 1997) carrying the recessive allele.

Bolting (stem elongation) is the first step clearly visible in the transition from vegetative to reproductive growth.

Traditionally, the biennial cultivated sugar beet is grown in spring and harvested in autumn. However, an extension of the growing season by sowing in autumn and cultivation over winter is expected to increase yield substantially and would allow to extend the sugar beet processing campaign addressing one demand of the sugar industry. However, cultivation of present sugar beet in central Europe over winter (i.e., as winter crop) is currently not possible because vernalization (inducted by the exposure to the cold temperatures during winter) would result in bolting and yield loss. It is thus highly desirable to develop non-bolting winter beet in which the vernalization response is modified to confer resistance or significant delay of bolting after cold-induction. Since the *B* gene plays a key role in the vernalization response in sugar beet, it represents a promising candidate for engineering bolting resistance by modulating the vernalization response.

Further, in cultivated sugar beet, bolting is an undesirable phenomenon, as it results in a drastic reduction of yield and gives rise to problems during harvesting and sugar extraction. Commercial seed productions for sugar beet are often done in regions, where annual weed beets are growing, which can cause pollen contamination in the seed productions, resulting in annuals in the commercial seed. This is not acceptable to the customers. To identify contaminations with annuals, commercial seed lots are grown in regions where no wild annual beets are growing directly after harvesting the seed. The plants are not vernalized and contaminations are identified by the presence of bolters. Replacing this test with a marker-based screening assay would be highly desirable, as results could be obtained earlier, which would lead to cost savings in seed processing.

A marker-based approach could also be advantageously used in sugar beet breeding, *e.g.,* to speed up the breeding process, or to introduce new variation from wild sea beets. Owing to the incomplete penetration of the *B* allele and its environmental dependence, closely linked molecular markers are also needed to screen its presence in breeding lines. For all these cases, it is important to have a marker tightly linked to the *B* gene to be able to identify annuals or biennials accurately.

For the foregoing reasons, there is a need for transgenic means to modulate the vernalization response of sugar beet and also for marker-assisted means to discriminate between the annual and biennial alleles of the *B* gene in seed production and in sugar beet breeding.

The present invention now provides such transgenic means as well as marker-assisted means addressing the above needs.

GAAFAR R M ET AL (2005, Tags, vol. 110, p. 1027-1037) discloses the process of map-based cloning of the gene responsible for bolting in *Beta vulgaris* and states that the two genetic markers left and right of the B-gene are located on two different contigs, with a gap in between the two contigs.

### SUMMARY OF THE INVENTION

This specification does not relate to essentially biological processes for the production of plants in the sense of Article 53(b) EPC. The present disclosure relates to nucleic acid sequences, which have a sequence identity of at least 70% to a nucleic acid sequence selected from the group of nucleic acid sequences as set forth in any one of SEQ ID NOs: 1, 4, 5, 6, 7, 8, 9, 10, 53 or 54, to nucleic acid sequences which comprise at least 15 consecutive nucleotides of a nucleic acid sequence selected from the group of nucleic acid sequences as set forth in any one of SEQ ID NOs: 1, 4, 5, 6, 7, 8, 9, 10, 53, or 54, to said one ; or to nucleic acid sequences which hybridize under stringent conditions or to a nucleic acid sequence selected from the group of nucleic acid sequences as set forth in any one of SEQ ID NOs: 1, 4, 5, 6, 7, 8, 9, 10, 53 or 54.

The nucleic acid sequence of the present disclosure described hereinbefore is an isolated nucleic acid. With regard to the homology all individual numerical values, which fall into the range starting with at least 70% as mentioned herein before, *i.e.,* 71%, 72%, 73%, 74%, 75%, ... ,80%, 81%, 82%, 83%, 84%, 85%, 86%, 87%, 88%, 89%, 90%, 91%, 92%, 93%, 94%, 95%, 96%, 97%, 98%, 99% should likewise be covered by the present invention. Preferably the length of the nucleic acid sequence of the present invention comprises at least 15, 20, 25, 30, 35, 40, 45, or at least 50 contiguous nucleotides of a nucleic acid sequence selected from the group of nucleic acid sequences as set forth in any one of SEQ ID NOs: 1, 4, 5, 6, 7, 8, 9, 10, 53, or 54. It is to be understood that the term "at least x nucleotides" encompasses nucleic acid molecules having any numerical value starting with x and above. For example, the term "at least 15 nucleotides" is intended to encompass nucleic acid molecules with 15, 16, 17, 18, 19, 20, and more nucleotides. In a further preferred embodiment, the nucleic acid sequence of the present invention described hereinbefore hybridizes under stringent conditions, more preferred under highly stringent conditions to a nucleic acid sequence selected from the group of nucleic acid sequences as set forth in any one of SEQ ID NOs: 1, 4, 5, 6, 7, 8, 9, 10, 53, or 54.

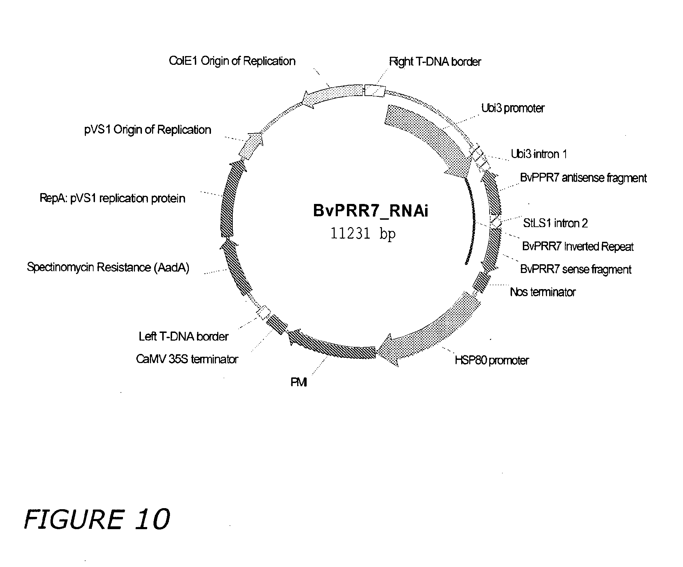

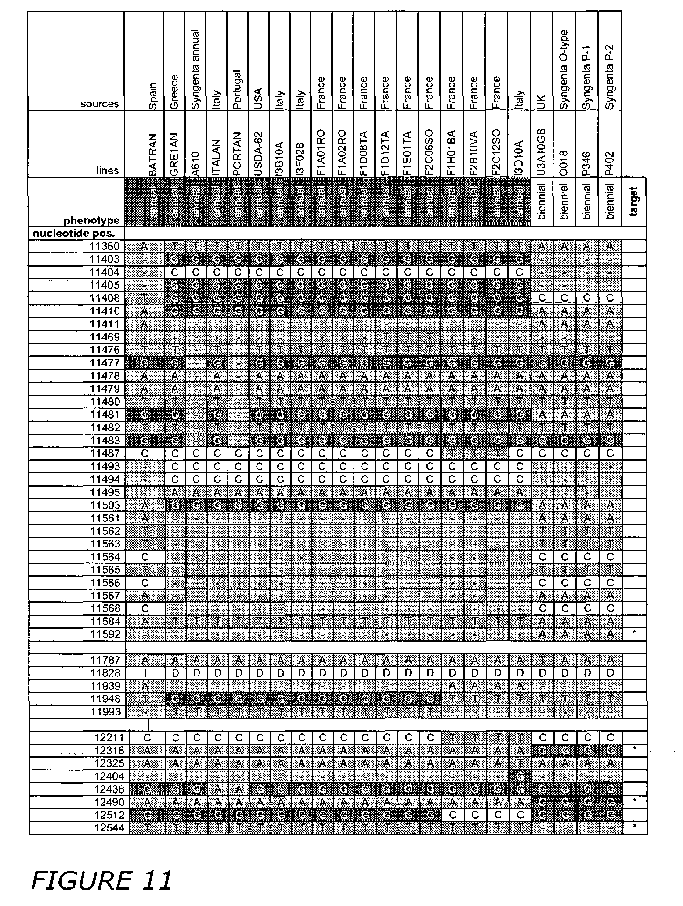

In a further embodiment of this aspect, the nucleic acid molecule comprises a nucleotide sequence selected from the group consisting of SEQ ID NOs: 4, 5, 6, 7, 8, 9, 10, 53, or 54. In another preferred embodiment, the nucleic acid sequence of the present invention described hereinbefore comprises a nucleic acid sequence as depicted in SEQ ID NO: 8, wherein said sequence comprises one or more nucleic acid substitutions, deletions, or.additions as shown in Tables 7-1 and 7-2, wherein the polymorphisms shown in Tables 7-1 and 7-2 are representing 18 annual and 2 biennial alleles of the sequence depicted in SEQ ID NO: 8, respectively. The Tables 7-1 and 7-2 are also shown as Figures 10 and 11.

The invention further relates to the use of the *BvPRR7* gene, in a transgenic approach for producing plants exhibiting a non-bolting phenotype. In particular, the invention relates to chimeric constructs comprising an expression cassette comprising a nucleic acid sequence of the present invention as described above under the control of regulatory elements, particularly under the control of regulatory elements functional in plants.

In one embodiment of the invention, the chimeric construct as described hereinbefore may further contain a selection marker gene which allows discriminating between transformed and non-transformed plant material in a selection procedure.

In one embodiment, the chimeric construct of the invention comprises a negative selection marker, particularly a selection marker encoding a resistance to plant toxic compounds such as antibiotics or herbicides. In another embodiment, the chimeric construct of the invention comprises a positive selection marker, particularly a selection marker encoding an enzyme that provides the transformed plant with a selective advantage over the non-transformed plants, particularly a nutritional advantage such as, for example, a phosphomannose isomerase gene, or a xylose isomerase gene.

In a preferred embodiment the chimeric construct of the present invention is provided for transgenic downregulation of *BvPRR7* gene expression, particularly through an antisense or an RNAi approach. In this context the term "downregulation" or "suppression" is meant to refer to any reduction of the expression level of the BvPPR7 gene compared to the expression of the gene in non-transformed plants under the same conditions. This includes "silencing" of a gene such that no expression of the gene can be detected. In another preferred embodiment the chimeric construct of the present invention for transgenic downregulation of *BvPRR7* gene expression comprises a nucleic acid molecule encoding a dsRNA which is capable of targeting mRNAs produced by transcription of the DNA sequence encoding the *B* gene protein, preferably the *BvPRR7* protein, for degradation. In another preferred embodiment, the chimeric construct of the present invention described hereinbefore comprises a nucleic acid molecule encoding said dsRNA, wherein the nucleic acid molecule has a length of at least 21 nucleotides and is substantially identical with at least a part of the coding sequence of the *BvPRR7* gene. Said coding sequences of the *BvPRR7* gene are preferably the nucleic acid sequences of the present invention described hereinbefore. More preferably said coding sequences of the *BvPRR7* gene are the nucleic acid molecules having as sequence as set forth as SEQ ID NOs: 4, 5, 6, 9, 10, 53, or 54 in the present invention. The "substantially identical" refers two nucleic acid molecules which are able to hybridize to each other under stringent conditions. Generally identify or homology between the dsRNA and the coding sequence of the *BvPRR7* gene or parts thereof is not required over the whole length of the dsRNA. It is sufficient if stretches of at least 21 nucleotides have identity, but preferably nucleic acid sequences are selected encoding dsRNA having homology to the target RNA molecule over a stretch of more than 21 nucleotides. Preferably, the chimeric construct of the present invention described hereinbefore comprises a nucleic acid molecule encoding the dsRNA and having a length of more than 21 nucleotides, more preferred of more than 50, 100, 250, 500, 600, 750, 1000 or more nucleotides.

In the disclosure the nucleic acid molecule encoding the dsRNA which is comprised in the chimeric construct of the present invention has the nucleotide sequence as depicted in SEQ ID NO: 1 under the control of a constitutive promoter, preferably the *Ubi3* promoter from *Arabidopsis.* In another embodiment, the chimeric construct of the present invention further comprises the sequence of the second intron from the potato *StLS1* gene (Eckes *et al,* 1986; Vancanneyt *et al,* 1990). The chimeric construct of the present invention comprises an inverted repeat targeting *BvPRR7* consisting of the nucleotide sequence as depicted in SEQ ID NO: 1 that was cloned between the *Ubi3* promoter (Norris *et al,* 1993) and the *Nos* terminator in both the antisense and sense orientation, separated by the second intron of the *StLS1* gene from potato.

In one embodiment of the invention, a transformation vector and/or an expression vector is provided, particularly a plant transformation vector and/or an expression vector, comprising the chimeric construct of the invention as described herein before. In a further embodiment the plant expression vector is an RNAi expression vector comprising the chimeric construct of the invention described hereinbefore. In a more preferred embodiment the RNAi expression vector comprises the chimeric construct of the present invention shown in Figure 11.

In a further aspect of the present invention a plant cell is provided comprising a chimeric construct or a vector molecule (*e.g.,* a transformation vector or an expression vector) according to the invention and as described herein before. In a preferred embodiment said plant cell comprising a chimeric construct or a vector molecule of the present invention is a plant cell of a sugar beet plant.

Further provided are transgenic plants, particularly sugar beet plants, having a phenotype of delayed bolting, or cells, tissues or seeds thereof, each comprising a plant cell of the present invention and/or a chimeric construct according of the present invention and/or a nucleic acid sequence of the present invention as described above, wherein said transgenic plant is expressing the dsRNA such that bolting is delayed, particularly suppressed, and the plant exhibits a phenotype of delayed bolting, preferably a non-bolting phenotype. "Delay of bolting" has to be understood as a modulation of the natural bolting reaction of sugar beet plants. During bolting the stem is elongated as a first step and during transition from vegetative to reproductive growth after vernalization of the plants (i.e., exposure to cold temperatures) and finally results in flower development. Delay of the bolting reaction is meant to refer to stem elongation which starts later compared to normal plants; those plants exhibit a phenotype of delayed bolting. The bolting reaction can be delayed by a few days (*i.e.,* by 5, 6, 7, 8, 9, 10, 11, 12, 13 or 14 days) and up to several weeks (*i.e.,* by 2, 3, 4 weeks) or several months (*i.e.,* 1, 2, 3, 5, or 6 months). In a preferred embodiment the bolting response is completely suppressed; such plants do not start to bolt after vernalization and exhibit a non-bolting phenotype. In a further preferred embodiment, the present invention provides transgenic plants, particularly sugar beet plants, which are produced from the cells, tissues or seeds of the present invention and described above.

In another aspect, there is herein described a method for producing hybrid seeds from which plants, particularly sugar beet plants, with a phenotype of modulated bolting can be grown. Such methods preferably: (a) providing a plant line, particularly a sugar beet line with a phenotype of modulated bolting, particularly a transgenic sugar beet plant of the present invention and as describe above as a first parent line, (b) providing a second plant line, particularly a sugar beet line having a different genotype as a second parent line; wherein one of the parent lines of step a) or step b) is a male sterile CMS line and wherein the other parent line is male fertile, and (c) allowing the plants of the male fertile parent line to pollinate the flowers of the second male sterile parent line, let the seed develop, and harvest the hybrid seed; wherein the harvested hybrid seeds are seeds of a hybrid plant, particularly a sugar beet hybrid plant, having a phenotype of delayed bolting. In a preferred embodiment both parent lines are sugar beet plant lines, wherein at least one of the sugar beet parent lines is a transgenic sugar beet line of the present invention. The at least one sugar beet parent line with a phenotype of modulated bolting can preferably also be a plant without any transgene, which is then obtained by other methods of genetic manipulation as described below. In an embodiment of this aspect the sugar beet parent line provided in step (a) is a male sterile CMS inbred sugar beet line comprising one or more of the a nucleic acid sequences of the present invention or fragments thereof, and the second sugar parent line provided in step (b) is a male fertile inbred sugar beet line. In another preferred embodiment the sugar beet parent line provided in step (a) is a male fertile sugar beet plant comprising one or more of the a nucleic acid sequences of the present invention or fragments thereof, and the second sugar parent line provided in step (b) is a male sterile CMS inbred sugar beet line.

A further aspect herein described relates to hybrid seed of a plant, particularly a sugar beet plant, exhibiting a phenotype of delayed bolting. In yet another aspect herein described the hybrid seed is produced by a method as described above. In a further preferred embodiment a hybrid plant, particularly a hybrid sugar beet plant, having a phenotype of delayed bolting is produced by growing the hybrid seed described above. A further preferred embodiment relates to plant parts selected from the group consisting of seeds, embryos, microspores, zygotes, protoplasts, cells, ovules, pollen, taproots, cotyledons, extracts or biological samples, which are derived from the transgenic sugar beet plant or seeds thereof or derived from hybrid plants or seeds as described above.

Another aspect of the present invention relates to the use of a nucleic acid sequence of the present invention or fragments thereof for the transformation of plant cells, particularly of cells of sugar beet plants. The purpose of transforming plant cells, particularly of cells of a sugar beet plant, with a nucleic acid sequence of the present invention or fragments thereof is to modulate the bolting behaviour of the plant as described above. Another embodiment of this aspect relates to a method of transforming plant cells, particularly of cells of a sugar beet plant, wherein the method comprises the use of a nucleic acid sequence of the present invention or of a chimeric construct of the present invention or of vector of the present invention and as described above.

In another aspect, the present invention provides the use of the transgenic plant of the present invention, of the hybrid plant of the present invention, or of the plant parts of the present invention and as described above in a method selected from the group comprising of methods of sugar production, methods of aerobic fermentation and methods of anaerobic fermentation. Preferably, the transgenic plant of the present invention, of the hybrid plant of the present invention, or of the plant parts of the present invention and as described above is used in a method of producing sugar.

In a further aspect the present invention relates to polynucleotide marker developed based on a nucleic acid sequence which is obtainable from a genomic DNA region that shows perfect co-segregation with the bolting gene (*B* gene) associated phenotype in sugar beet and wherein the marker allows to discriminate between the annual and biennial genotype or between different haplotypes within a plant grouping of sugar beet plants exhibiting a biennial or annual genotype. In a preferred embodiment the polynucleotide markers of the present invention have a nucleic acid sequence obtainable from one or more of the nucleic acid sequences of the present invention and as described above. In one embodiment the polynucleotide markers of the present invention further comprise one or more polymorphisms, particularly a polymorphism that is based on an SNP, an SSR, a deletion or an insertion of at least one nucleotide, but especially a polymorphism based on an SNP, which polymorphism is diagnostic for the *B* allele at the *B* locus. Such polynucleotide markers are preferably capable of detecting at least one of the various SNPs present in the different alleles of the genomic sequence set forth as SEQ ID NO: 8 herein and shown in Tables 7-1 (further depicted in Figure 10) and 7-2 (further depicted in Figure 10), wherein said polynucleotide marker is capable of differentiating between different alleles, particularly between annual and biennial sugar beet lines. In a preferred embodiment the polynucleotide marker of the present invention are capable of detecting at least one SNP selected from the group comprising SNPs at positions #224, #351, #615, #897, #1082, #1841, #1915, #2334, #11592, #12316, #12490, or #12544 of the sequence set forth as SEQ ID NO: 8 and as shown in Tables 7-1 (further depicted in Figure 10) and 7-2 (further depicted in Figure 11). A further aspect of the present invention relates to a set of polynucleotide markers comprising a plurality of the polynucleotide markers of the present invention and described above. In this context, the term "plurality" refers to a set of more than one polynucleotide marker, which preferably consists of two, three or more markers. Another aspect of the present invention relates to a pair of primers consisting of a forward primer and a reverse primer which primers are capable of annealing to a nucleotide sequence within a genomic region of the sugar beet genome DNA that shows perfect co-segregation with BvPPR7. In a preferred embodiment the pair of primers of the present invention anneals to a nucleic acid sequence of the present invention and as describe above and amplifies a polynucleotide, preferably the polynucleotide marker of the present invention, or of an informative part thereof, wherein said polynucleotide comprises one or more polymorphisms, particularly one or more polymorphisms which is/are diagnostic for the B allele at the B locus and allows to discriminate between the annual and biennial genotype. In a preferred embodiment the pair of primers of the present invention is a pair of primers which anneals to the nucleic acid sequence set forth as SEQ ID NO: 8 and amplifies an informative fragment from said sequence comprising a polymorphism selected from the polymorphisms based on a SNP present in the different alleles of said sequence as shown in Tables 7-1 (further depicted in Figure 10) and 7-2 (further depicted in Figure 11). In a further preferred embodiment, the pair of primers of the present invention comprises: (a) forward primer PRR7(T6)-F as depicted in SEQ ID NO: 49 and reverse primer PRR7(T6)-R as depicted in SEQ ID NO: 50 for amplifying a fragment comprising SNP #2334; or (b) forward primer PRR7(T1)-F as depicted in SEQ ID NO: 13 and reverse primer PRR7(T1)-R as depicted in SEQ ID NO: 14 for amplifying a fragment comprising SNP #160; or (c) forward primer 1r22(T1)-F as depicted in SEQ ID NO: 55 and reverse primer 1r22(T1)-R as depicted in SEQ ID NO: 56 for amplifying a fragment and probe molecule 1r22(T1)-VIC as depicted in SEQ ID NO: 57 as a first probe molecule labelled with VIC as first fluorescent dye and probe molecule 1r22(T1)-FAM as depicted in SEQ ID NO: 58 as a second probe molecule labelled with FAM as second fluorescent dye.

The disclosure relates to one or a plurality of probe molecules and/or to one or a plurality of primers, particularly one or a plurality of primer pairs, but especially one or a plurality of primer pairs consisting of a forward primer and a reverse primer, which primers are capable of annealing to a nucleic acid sequence which is obtainable from a genomic DNA region that shows perfect co-segregation with the bolting gene (*B* gene) associated phenotype in sugar beet and wherein the marker allows to discriminate between the annual and biennial genotype or between different haplotypes within plant groupings of sugar beet plants exhibiting a biennial or annual genotype.

The disclosure, relates to a set of probe polynucleotides comprising at least two separate probe molecules that are complementary to a subregion within an informative polynucleotide fragment according to the invention and as described herein before comprising a polymorphic site and amplify partially overlapping fragments which differ only by one or two base mismatches in the area of overlap, wherein a first probe, particularly a probe labelled with a first fluorescent dye, more particularly with a first fluorescent dye and a quencher represents one allele and a second probe, particularly a probe labelled with a second fluorescent dye, which is not identical with the first dye, more particularly with a second fluorescent dye and a quencher, represents the other allele.

The above polynucleotide markers of the present the disclosure, a set of polynucleotide markers of the present the disclosure or a pair of primers of the present the disclosure can be used in an allelic discrimination assay for identifying the absence or presence of an allele associated with annuality in a sugar beet plant.

In another aspect of the present the disclosure an allelic discrimination assay for identifying the absence or presence of an allele associated with annuality in a sugar beet plant is provided, which allows to discriminate between annual and biennial plants. In a preferred embodiment the polynucleotide marker of the present the disclosure, a set of polynucleotide markers of the present the disclosure, or a pair of primers of the present the disclosure is used in this assay.

In a further preferred embodiment the allelic discrimination assay of the present invention comprises the steps of: (a) obtaining a sample of genomic DNA from a sugar beet plant to be analyzed, (b) amplifying a fragment from said sample or genomic DNA using a pair of primers of the present invention, and (c) comparing the amplified fragment with an allelic sequence known to be associated with the biennial phenotype but not with the annual phenotype, respectively. In this assay the sequence of amplified fragment of step (c) is compared with sequences of alleles known to be associated with the biennial phenotype. If the sequence is different from the sequences of the biennial alleles, this is indicative of the presence of annual allele (*i.e.,* an annual plant). In another preferred embodiment the amplified fragment obtained in step c) of the allelic discrimination assay of the present invention is probed with a first fluorescence-labelled probe molecule comprising a sequence specific for the annual allele. If the dye fluorescence of the first probe increases during the reaction this is indicative of the presence of the annual allele.

In a preferred embodiment, the assay of the present invention employs either (a) forward primer PRR7(T6)-F as depicted in SEQ ID NO: 49 and reverse primer PRR7(T6)-R as depicted in SEQ ID NO: 50 for amplifying a fragment comprising SNP #2334 and probe molecule PRR7(T6)-VIC as depicted in SEQ ID NO: 51 as a first probe molecule labelled with VIC as first fluorescent dye and probe molecule PRR7(T6)-FAM as depicted in SEQ ID NO: 52 as a second probe molecule labelled with FAM as second fluorescent dye; or (b) forward primer PRR7(T1)-F as depicted in SEQ ID NO: 13 and reverse primer PRR7(T1)-R as depicted in SEQ ID NO: 14 for amplifying a fragment comprising SNP #160 and probe molecule PRR7(T1)-VIC as depicted in SEQ ID NO: 15 as a first probe molecule labelled with VIC as first fluorescent dye and probe molecule PRR7(T1)-FAM as depicted in SEQ ID NO: 16 as a second probe molecule labelled with FAM as second fluorescent dye; or (c) forward primer 1r22(T1)-F as depicted in SEQ ID NO: 55 and reverse primer 1r22(T1)-R as depicted in SEQ ID NO: 56 for amplifying a fragment and probe molecule 1r22(T1)-VIC as depicted in SEQ ID NO: 57 as a first probe molecule labelled with VIC as first fluorescent dye and probe molecule 1r22(T1)-FAM as depicted in SEQ ID NO: 58 as a second probe molecule labelled with FAM as second fluorescent dye.

The disclosure, relates to a method of identifying annual contaminations in commercial seed using a marker-based allelic discrimination assay according to the invention and as described herein before.

### BRIEF DESCRIPTION OF THE FIGURES AND SEQUENCES

### FIGURES

Figure 1: Amino acid sequence comparison of the REC domains between different species and the putative REC domain of sugar beet EST CV301305. Identical amino acids are in black; conserved in grey; weakly similar in light grey and non-similar in white. Bb, *Bordetella bronchiseptica;* Bs, *Bacillus subtilis;* Bv, *Beta vulgaris;* Ec, *Escherichia coli*; Kp, *Klebsiella pneumoniae*; Pa, *Pseudomonas aeruginosa;* Rc, *Rhodobacter capsulatus*; Sc, *Streptomyces coelicolor*; Sf, *Shigella flexneri*; St, *Salmonella typhimurium.*
Figure 2: Amino acid sequence comparison of the *Arabidopsis PRR7* protein and the predicted partial protein from sugar beet EST CV301305. Identical amino acids are in black; similar in grey and non-similar in white.
Figure 3: Sequence alignment between the genomic and mRNA sequences of the *Arabidopsis PRR7* gene and sugar beet EST CV301305. Conserved nucleotides between *Arabidopsis* and *Beta vulgaris* L. are in grey. Introns are represented by strings of dashes.
Figure 4: Genetic map of sugar beet chromosome II. Marker names are given at the right of the chromosome, at the left the cumulative genetic distance is indicated.
Figure 5: Schematic representation of the gene structure of the *BvPRR7* gene showing putative exons and introns. The region covered by EST CV301305 is shown by the block arrow.
Figure 6: Amino acid sequence comparison of the *Arabidopsis PRR* gene family members and the *BvPRR7* protein. Identical amino acids are in black; conserved in grey; weakly similar in light grey and non-similar in white.

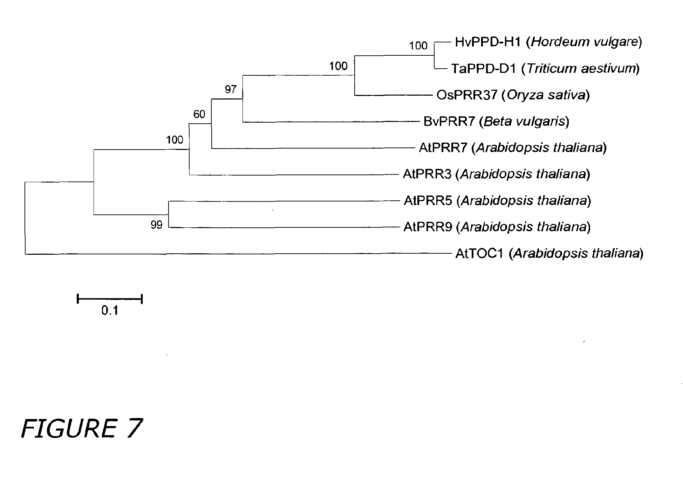

Figure 7: Phylogenetic relationship between *BvPRR7* and related proteins from other flowering plants based on an phylogenetic analysis of multiple members in the PRR gene family from several plant species including the PRR7 homolog from sugar beet, *Arabidopsis thaliana* (TOC1, NP_200946; PRR3, NP_568919; PRR5, NP_568446; PRR7, NP_568107; and PRR9, NP_566085), *Oryza sativa* (PRR37, Q0D3B6), *Hordeum vulgare* (PPD-H1, AAY17586) and *Triticum aestivum* (PPD-D1, ABL09477) by using the Neighbor-Joining method (Saitou and Nei, 1987). The bootstrap consensus tree inferred from 1000 replicates is taken to represent the evolutionary history of the taxa analyzed (Felsenstein, 1985). Branches corresponding to partitions reproduced in less than 50% bootstrap replicates are collapsed. The percentage of replicate trees in which the associated taxa clustered together in the bootstrap test (1000 replicates) are shown next to the branches. The tree is drawn to scale, with branch lengths in the same units as those of the evolutionary distances used to infer the phylogenetic tree. The evolutionary distances were computed using the Poisson correction method (Zuckerkandl and Pauling, 1965) and are in the units of the number of amino acid substitutions per site. All positions containing gaps and missing data were eliminated from the dataset (Complete deletion option). There were a total of 352 positions in the final dataset.
Figure 8: Diurnal expression patterns of *BvPRR7* in annual and biennial sugar beet plants. Leaf tissues were harvested every 2 hours across a period of 24 hours. Wight and dark grey backgrounds represent light respectively dark periods. Data shown are mean values from three independent biological samples. Values are expressed as relative expression levels normalized against the *BvlCDH* reference gene by geometric averaging analysis (Vandesompele *et al.,* 2002). Error bars ± SD. ZT, zeitgeber time.

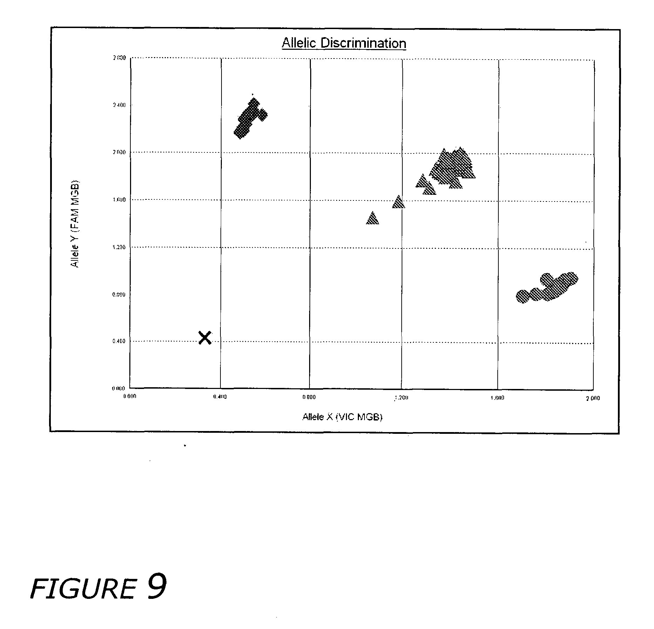

Figure 9: EndPoint reading of an allelic discrimination analysis between a set of annual and biennial individual plants. Values on the Y and X axis represent fluorescence levels of the FAM dye and the VIC dye, respectively. Substantial increase in VIC dye fluorescence (X axis) only indicates homozygosity for the biennial allele (referred to as allele X in this Figure). Substantial increase in FAM dye fluorescence only indicates homozygosity for the annual allele ((referred to as allele Y in this Figure). Substantial increase in both fluorescent signals indicates heterozygosity, i.e. annual plant with heterozygosity for the *B* locus.
Figure 10: Plasmid map of the binary vector used for the transgenic suppression of *BvPRR7* by means of RNAi. The inverted repeat for *BvPRR7* consists of a 0.6 Kb cDNA fragment that was cloned between the Ubi3 promoter (Norris *et al,* 1993) and Nos terminator in both the antisense and sense orientation, separated by the second intron of the StLS1 gene from potato (Eckes *et al,* 1986; Vancanneyt *et al,* 1990). The selectable marker consists of the PMI gene under the control of the HSP80 promoter (Brunke and Wilson, 1993).
Figure 11: Table showing polymorphisms identified in the promoter region of the *BvPRR7 when* comparing 18 annual and 2 biennial alleles of BvPRR7; SNP positions indicated in the table are numbered in accordance to SEQ ID NO: 8.

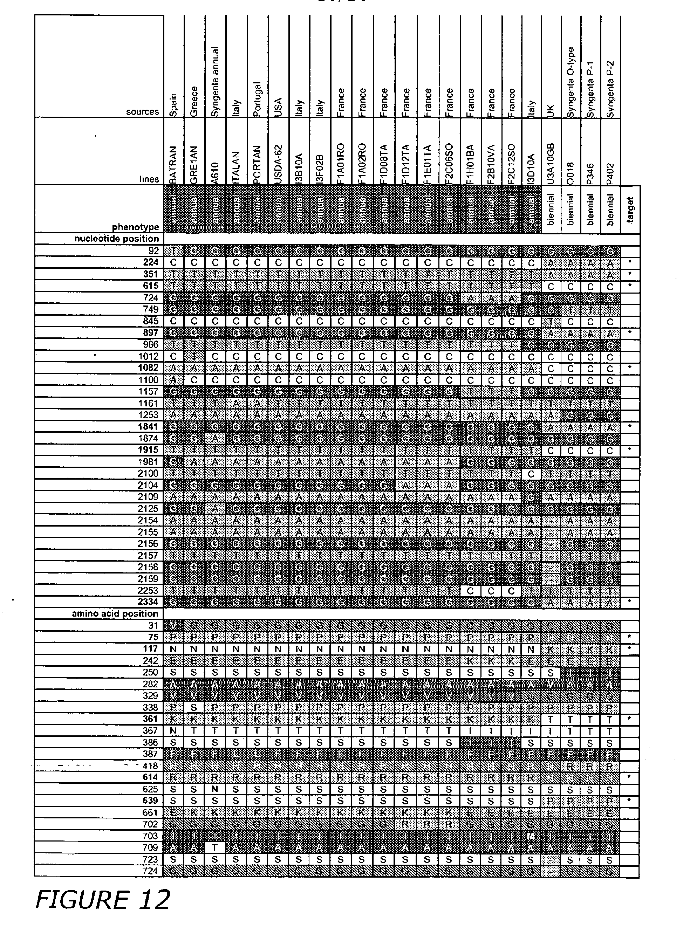

Figure 12: Table showing polymorphisms identified in the coding region of the *BvPRR7 when* comparing 18 annual and 2 biennial alleles of *BvPRR7*; SNP positions indicated in the table are numbered in accordance to SEQ ID NO: 8.

### SEQUENCES

SEQ ID NO: 1 depicts the nucleotide sequence of the sugar beet EST CV301305
SEQ ID NO: 2 depicts the nucleotide sequence of the forward primer PRR7-F
SEQ ID NO: 3 depicts the nucleotide sequence of the reverse primer PRR7-R
SEQ ID NO: 4 depicts the nucleotide sequence of intron 3 of allelic variant 2 of *BvPRR7* (haplotype #2)
SEQ ID NO: 5 depicts the nucleotide sequence of intron 3 of allelic variant 1 of *BvPRR7* (haplotype #1)
SEQ ID NO: 6 depicts the nucleotide sequence of intron 3 of *BvPRR7* and its allelic variability for mapping
SEQ ID NO: 7 depicts the genomic nucleotide sequence of the biennial allele of *BvPRR7*
SEQ ID NO: 8 depicts the nucleotide sequence of genomic nucleotide sequence of *BvPRR7* including the promoter and the terminator regions.
SEQ ID NO: 9 depicts the nucleotide sequence of the coding region of the biennial allele of *BvPRR7*
SEQ ID NO: 10 depicts the nucleotide sequence of the coding region of the annual allele of *BvPRR7*
SEQ ID NO: 11 depicts the putative amino acid sequence of the biennial allele of *BvPRR7*
SEQ ID NO: 12 depicts the putative amino acid sequence of the annual allele of *BvPRR7*
SEQ ID NO: 13 depicts the nucleotide sequence of primer PRR7(T1)-F
SEQ ID NO: 14 depicts the nucleotide sequence of primer PRR7(T1)-R
SEQ ID NO: 15 depicts the nucleotide sequence of probe PRR7(T1)-VIC
SEQ ID NO: 16 depicts the nucleotide sequence of probe PRR7(T1)-FAM
SEQ ID NO: 17 depicts the nucleotide sequence of primer GJ131(T1)-F
SEQ ID NO: 18 depicts the nucleotide sequence of primer GJ131(T1)-R
SEQ ID NO: 19 depicts the nucleotide sequence of probe GJ131(T1)-VIC
SEQ ID NO: 20 depicts the nucleotide sequence of probe GJ131(T1)-FAM
SEQ ID NO: 21 depicts the nucleotide sequence of primer ED031700(T1)-F
SEQ ID NO: 22 depicts the nucleotide sequence of primer ED031700(T1)-R
SEQ ID NO: 23 depicts the nucleotide sequence of probe ED031700(T1)-VIC
SEQ ID NO: 24 depicts the nucleotide sequence of probe ED031700(T1)-FAM
SEQ ID NO: 25 depicts the nucleotide sequence of primer 9_27(T2)-F
SEQ ID NO: 26 depicts the nucleotide sequence of primer 9_27(T2)-R
SEQ ID NO: 27 depicts the nucleotide sequence of probe 9_27(T2)-VIC
SEQ ID NO: 28 depicts the nucleotide sequence of probe 9_27(T2)-FAM
SEQ ID NO: 29 depicts the nucleotide sequence of primer GJ01(T1)-F
SEQ ID NO: 30 depicts the nucleotide sequence of primer GJ01(T1)-R
SEQ ID NO: 31 depicts the nucleotide sequence of probe GJ01(T1)-VIC
SEQ ID NO: 32 depicts the nucleotide sequence of probe GJ01(T1)-FAM
SEQ ID NO: 33 depicts the nucleotide sequence of primer SELA3977
SEQ ID NO: 34 depicts the nucleotide sequence of primer SELA3988
SEQ ID NO: 35 depicts the nucleotide sequence of primer SELA4442
SEQ ID NO: 36 depicts the nucleotide sequence of primer SELA3809
SEQ ID NO: 37 depicts the nucleotide sequence of primer SELA3810
SEQ ID NO: 38 depicts the nucleotide sequence of primer SELA3807
SEQ ID NO: 39 depicts the nucleotide sequence of primer SELA3766
SEQ ID NO: 40 depicts the nucleotide sequence of primer SELA3769
SEQ ID NO: 41 depicts the nucleotide sequence of primer SELA3857
SEQ ID NO: 42 depicts the nucleotide sequence of primer SELA3860
SEQ ID NO: 43 depicts the nucleotide sequence of primer SELA3861
SEQ ID NO: 44 depicts the nucleotide sequence of primer SELA3864
SEQ ID NO: 45 depicts the nucleotide sequence of forward primer *BvPRR7* used for gene expression analysis
SEQ ID NO: 46 depicts the nucleotide sequence of reverse primer *BvPRR7* used for gene expression analysis
SEQ ID NO: 47 depicts the nucleotide sequence of forward primer *BvlCDH* used for gene expression analysis
SEQ ID NO: 48 depicts the nucleotide sequence of reverse primer *BvlCDH* used for gene expression analysis
SEQ ID NO: 49 depicts the nucleotide sequence of primer PRR7(T6)-F
SEQ ID NO: 50 depicts the nucleotide sequence of primer PRR7(T6)-R
SEQ ID NO: 51 depicts the nucleotide sequence of probe PRR7(T6)-VIC
SEQ ID NO: 52 depicts the nucleotide sequence of probe PRR7(T6)-FAM
SEQ ID NO: 53 depicts the nucleotide sequence of the coding region of the annual *PRR7* allele downstream of approximately 1,3 kb of its promoter region
SEQ ID NO: 54 depicts the nucleotide sequence of the coding region of the annual allele of *BvPRR7* including approximately 1,3 kb of its promoter region and approximately 0.7 kb of its terminator region
SEQ ID NO: 55 depicts the nucleotide sequence of primer 1r22(T1)-F
SEQ ID NO: 56 depicts the nucleotide sequence of primer 1r22(T1)-R
SEQ ID NO: 57 depicts the nucleotide sequence of probe 1r22(T1)-VIC
SEQ ID NO: 58 depicts the nucleotide sequence of probe 1r22(T1)-FAM

### DEFINITIONS

The technical terms and expressions used within the scope of this application are generally to be given the meaning commonly applied to them in the pertinent art of plant molecular biology if not otherwise indicated herein below.

As used in this specification and the appended claims, the singular forms "a", "an", and "the" include plural referents unless the context clearly dictates otherwise. Thus, for example, reference to "a plant" includes one or more plants, and reference to "a cell" includes mixtures of cells, tissues, and the like.

"Sugar beet" refers to all species and subspecies within the genus *Beta* as well as all kinds of cultivated beets of *Beta vulgaris.* Cultivated beets have been separated into four groups: leaf beet, garden beet, fodder beet and sugar beet. "Sugar beet" refers also to all cultivated beets including those grown for other purposes than the production of sugar, such as ethanol, plastics or industrial products. In particular, "Sugar beet" refers to fodder beet and sugar beet, but especially to sugar beet. This term also includes sugar beet plants adapted for growth in tropical or subtropical regions.

An "annual sugar beet line" refers to a sugar beet plant containing the dominant allele *B* at the *B* locus in a heterozygous or homozygous state.

A "biennial sugar beet line" refers to a sugar beet plant containing the recessive allele *b* at the *B* locus in a homozygous state

"Bolting" refers to the transition from the vegetative rosette stage to the inflorescence or reproductive growth stage.

"Delayed bolting" or "delay of bolting" as used herein has to be understood as a modulation of the natural bolting reaction of sugar beet plants. In plants with delayed bolting stem elongation as the first visible step of bolting starts later than in normal plants. The bolting reaction can be delayed by just a few days (*i.e.,* by, for example, 5, 6, 7, 8, 9, 10, 11, 12, 13 or 14 days) and up to several weeks (*i.e.,* by 2, 3, 4 weeks) or several months (*i.e.,* 1, 2, 3, 5, or 6 months). Delay of bolting can also result in a complete suppression of the bolting response; such plants do not bolt after vernalization and exhibit a non-bolting phenotype.

"*B* gene" as used herein refers to a gene that is responsible for the determination of the annual habit (early bolting) in sugar beet. Plants carrying the dominant allele *B* are able to switch from juvenile to reproductive stages in a vernalization-independent manner, *i.e.* make shoot elongation followed by flowering without prior exposure to cold temperatures.

"Vernalization" refers to the process by which floral induction in some plants is promoted by exposing the plants to chilling for certain duration.

An "allele" is understood within the scope of the invention to refer to alternative forms of various genetic units associated with different forms of a gene or of any kind of identifiable genetic element, which are alternative in inheritance because they are situated at the same locus in homologous chromosomes. In a diploid cell or organism, the two alleles of a given gene (or marker) typically occupy corresponding loci on a pair of homologous chromosomes.

As used herein, the term "haplotype" refers to the set of alleles an individual inherited from one parent. A diploid individual thus has two haplotypes. The term "haplotype" can be used in a more limited sense to refer to physically linked and/or unlinked genetic markers (*e.g.,* sequence polymorphisms) associated with a phenotypic trait (such as the annual or biennial bolting behavior of sugar beet plants in the context of the present invention). With regard to the *B* gene the haplotype of this gene also directly confers a phenotype. The annual growing habit of sugar beet, for example, is caused by the presence of the dominant allele of locus *B* at chromosome II.

"Locus" is understood within the scope of the invention to refer to a region on a chromosome, which comprises a gene or any other genetic element or factor contributing to a trait.

As used herein, the phrase "genetic marker" refers to a feature of an individual's genome (*e.g.,* a nucleotide or a polynucleotide sequence that is present in an individual's genome) that is associated with one or more loci of interest. In some embodiments, a genetic marker is polymorphic in a population of interest, or the locus occupied by the polymorphism, depending on context. Genetic markers include, for example, single nucleotide polymorphisms (SNPs), indels (*i.e.,* insertions/deletions), simple sequence repeats (SSRs), restriction fragment length polymorphisms (RFLPs), random amplified polymorphic DNAs (RAPDs), cleaved amplified polymorphic sequence (CAPS) markers, Diversity Arrays Technology (DArT) markers, and amplified fragment length polymorphisms (AFLPs), among many other examples. Genetic markers can, for example, be used to locate genetic loci containing alleles that contribute to variability in expression of phenotypic traits on a chromosome. The phrase "genetic marker" can also refer to a polynucleotide sequence complementary to a genomic sequence, such as a sequence of a nucleic acid used as probes.

A genetic marker can be physically located in a position on a chromosome that is within or outside of to the genetic locus with which it is associated (*i.e.,* is intragenic or extragenic, respectively). Stated another way, whereas genetic markers are typically employed when the location on a chromosome of the gene that corresponds to the locus of interest has not been identified and there is a non-zero rate of recombination between the genetic marker and the locus of interest, the presently disclosed subject matter can also employ genetic markers that are physically within the boundaries of a genetic locus (*e.g.,* inside a genomic sequence that corresponds to a gene such as, but not limited to a polymorphism within an intron or an exon of a gene). In some embodiments of the presently disclosed subject matter, the one or more genetic markers comprise between one and ten markers, and in some embodiments the one or more genetic markers comprise more than ten genetic markers.

As used herein, the phrase "phenotypic trait" refers to the appearance or other detectable characteristic of an individual, resulting from the interaction of its genome with the environment.

"Phenotype" is understood within the scope of the invention to refer to a distinguishable characteristic(s) of a genetically controlled trait.

The terms "closely linked" or "genetically closely linked" as used herein in the context of a genomic region of the sugar beet genome linked to the *B* gene are understood to refer to a close association of the genomic region and the *B* gene in inheritance due to location of both in proximity on the same chromosome, measured by percent recombination between loci (centi-Morgan, cM). As used herein, the term "linkage", and grammatical variants thereof, refers to the tendency of alleles at different loci on the same chromosome to segregate together (co-segregate) more often than would be expected by chance if their transmission were independent,

As used herein, the phrase "informative fragment" refers to a polynucleotide fragment with an information content that is a retrievable and can assist in the determination and/or characterization of a genetic locus of interest. This information content may be represented by a polymorphism which is associated with said locus of interest such as, for example, a single nucleotide polymorphisms (SNPs), indels (*i.e.,* insertions/deletions), simple sequence repeats (SSRs), restriction fragment length polymorphisms (RFLPs), random amplified polymorphic DNAs (RAPDs), cleaved amplified polymorphic sequence (CAPS) markers, Diversity Arrays Technology (DArT) markers, and amplified fragment length polymorphisms (AFLPs), among many other examples and may be used for the development of a genetic marker. The information content of such an "informative fragment" may also be represented by a specific sequence that can be detected by a corresponding probe molecule. Such informative fragments could be a primer or a marker or a part thereof. Such fragments have a length of at least 10 nucleotides, preferably of at least 15, 20, 25, 30, 50, or 100 nucleotides.

"Marker-based selection" is understood within the scope of the invention to refer to the use of genetic markers to detect one or more nucleic acids from the plant, where the nucleic acid is associated with a desired trait to identify plants that carry genes for desirable (or undesirable) traits, so that those plants can be used (or avoided) in a selective breeding program.

"PCR (Polymerase chain reaction)" is understood within the scope of the invention to refer to a method of producing relatively large amounts of specific regions of DNA, thereby making possible various analyses that are based on those regions.

"PCR primer" or "primer" is understood within the scope of the invention to refer to short fragments of isolated single-stranded DNA used in the PCR amplification of specific regions of DNA. They are annealed to a complimentary target DNA strand by nucleic acid hybridization to form a hybrid between the primer and the target DNA strand, and then extended along the target DNA strand by a polymerase, such as DNA polymerase. Primer pairs or sets can be used for amplification of a nucleic acid molecule, for example, by the polymerase chain reaction (PCR) or other conventional nucleic-acid amplification methods. Primers are generally between 10 and 15 nucleotides or more in length. Primers can also be at least 20 nucleotides or more in length, or at least 25 nucleotides or more, or at least 30 nucleotides or more in length. Such primers hybridize specifically to a target sequence under high stringency hybridization conditions. Primers according to the present invention may have complete sequence complementarity with the target sequence. It is to be understood that the length of the primers of the present invention can be any numerical value between the values specified herein. Thus, primers being generally between 10 and 15 nucleotides or more in length encompass primer having a length of 10, 11, 12, 13, 14, or 15 nucleotides, whereas the expression "at least 20 nucleotides" further includes primer having a length of 16, 17, 18, 19, or nucleotides. The same applies to the expressions "at least 25 nucleotides or more" and "at least 30 nucleotides or more in length".

As used herein, the term "amplified" means the construction of multiple copies of a nucleic acid molecule or multiple copies complementary to the nucleic acid molecule using at least one of the nucleic acid molecules as a template. Amplification systems include the polymerase chain reaction (PCR) system, ligase chain reaction (LCR) system, nucleic acid sequence based amplification (NASBA, Cangene, Mississauga, Ontario), Q-Beta Replicase systems, transcription-based amplification system (TAS), and strand displacement amplification (SDA). See, e.g., Diagnostic Molecular Microbiology: Principles and Applications, D. H. Persing et al., Ed., American Society for Microbiology, Washington, D.C. (1993). The product of amplification is termed an amplicon.

A "probe" is an isolated nucleic acid to which is attached a conventional detectable label or reporter molecule, such as a radioactive isotope, ligand, chemiluminescent agent, fluorescent label or enzyme. Such a probe is complimentary to a strand of a target nucleic acid. Probes according to the present invention include not only deoxyribonucleic or ribonucleic acids but also polyamides and other probe materials that bind specifically to a target DNA sequence and can be used to detect the presence of that target DNA sequence.

Primers and probes are generally between 10 and 15 nucleotides or more in length. Primers and probes can also be at least 20 nucleotides or more in length, or at least 25 nucleotides or more, or at least 30 nucleotides or more in length. Such primers and probes hybridize specifically to a target sequence under high stringency hybridization conditions. Primers and probes according to the present invention may have complete sequence complementarity with the target sequence, although probes differing from the target sequence and which retain the ability to hybridize to target sequences may be designed by conventional methods. It is to be understood that the length of the primers and probes of the present invention can be any numerical value between the values specified herein. Thus, primers and probes being generally between 10 and 15 nucleotides or more in length encompass primer and probes having a length of 10, 11, 12, 13, 14, or 15 nucleotides, whereas the expression "at least 20 nucleotides" further includes primer and probes having a length of 16, 17, 18, 19, or nucleotides. The same applies to the expressions "at least 25 nucleotides or more" and "at least 30 nucleotides or more in length".

"Polymorphism" is understood within the scope of the invention to refer to the presence in a population of two or more different forms of a gene, genetic marker, or inherited trait.

A "single-nucleotide polymorphism" or "SNP" is understood within the scope of the invention to refer to a DNA sequence variation occurring when a single nucleotide in the genome (or other shared sequence) differs between members of a species or between paired chromosomes in an individual. Two sequenced DNA fragments from different individuals containing a difference in a single nucleotide are called two alleles. Preferably, a SNP has only two alleles.

The term "polynucleotide" is understood herein to refer to polymeric molecule of high molecular weight which can be single-stranded or double-stranded, composed of monomers (nucleotides) containing a sugar, phosphate and a base which is either a purine or pyrimidine. A "polynucleotide fragment" is a fraction of a given polynucleotide molecule. In higher plants, deoxyribonucleic acid (DNA) is the genetic material while ribonucleic acid (RNA) is involved in the transfer of information contained within DNA into proteins. A "genome" is the entire body of genetic material contained in each cell of an organism. The term "polynucleotide" thus refers to a polymer of DNA or RNA which can be single- or double-stranded, optionally containing synthetic, non-natural or altered nucleotide bases capable of incorporation into DNA or RNA polymers. Unless otherwise indicated, a particular nucleic acid sequence of this invention also implicitly encompasses conservatively modified variants thereof (*e.g.,* degenerate codon substitutions) and complementary sequences and as well as the sequence explicitly indicated. Specifically, degenerate codon substitutions may be achieved by generating sequences in which the third position of one or more selected (or all) codons is substituted with mixed-base and/or deoxyinosine residues (Batzer *et al.,* 1991; Ohtsuka *et al.,* 1985; Rossolini *et al.,* 1994). The term polynucleotide is used interchangeably with nucleic acid, nucleotide sequence and may include genes, cDNAs, and mRNAs encoded by a gene, etc.

The term "isolated", when used in the context of the nucleic acid molecules of the present invention, refers to a nucleic acid sequence that is identified within and isolated/separated from its chromosomal nucleic acid sequence context within the respective source organism. An isolated nucleic acid is not a nucleic acid as it occurs in its natural context, if it indeed has a naturally occurring counterpart. In contrast, non-isolated nucleic acids are nucleic acids such as DNA and RNA, which are found in the state they exist in nature. For example, a given DNA sequence (e.g. a gene) is found on the host cell chromosome in proximity to neighboring genes. The isolated nucleic acid sequence may be present in single-stranded or double-stranded form. Alternatively, it may contain both the sense and anti-sense strands (i.e., the nucleic acid sequence may be double-stranded). If claimed in the context of a plant genome, the nucleic acid molecule of the invention is distinguished over naturally occurring counterparts by the insertion side in the genome and the flanking sequences at the insertion site. In a preferred embodiment, the nucleic acid molecules of the present invention are understood to be isolated.

As used herein, the phrase "nucleic acid" refers to any physical string of monomer units that can be corresponded to a string of nucleotides, including a polymer of nucleotides (*e.g.,* a typical DNA or RNA polymer), modified oligonucleotides (*e.g.,* oligonucleotides comprising bases that are not typical to biological RNA or DNA, such as 2'-O-methylated oligonucleotides), and the like. In some embodiments, a nucleic acid can be single-stranded, double-stranded, multi-stranded, or combinations thereof. Unless otherwise indicated, a particular nucleic acid sequence of the presently disclosed subject matter optionally comprises or encodes complementary sequences, in addition to any sequence explicitly indicated.

The term "gene" is used broadly to refer to any segment of nucleic acid associated with a biological function. Thus, genes include coding sequences and/or the regulatory sequences required for their expression. For example, gene refers to a nucleic acid fragment that expresses mRNA or functional RNA, or encodes a specific protein, and which includes regulatory sequences. Genes also include non-expressed DNA segments that, for example, form recognition sequences for other proteins. Genes can be obtained from a variety of sources, including cloning from a source of interest or synthesizing from known or predicted sequence information, and may include sequences designed to have desired parameters.

"Expression cassette" as used herein means a nucleic acid molecule capable of directing expression of a particular nucleotide sequence in an appropriate host cell, comprising a promoter operably linked to the nucleotide sequence of interest which is operably linked to termination signals. It also typically comprises sequences required for proper translation of the nucleotide sequence. The expression cassette may also comprise sequences not necessary in the direct expression of a nucleotide sequence of interest but which are present due to convenient restriction sites for removal of the cassette from an expression vector. The expression cassette comprising the nucleotide sequence of interest may be chimeric, meaning that at least one of its components is heterologous with respect to at least one of its other components. The expression cassette may also be one that is naturally occurring but has been obtained in a recombinant form useful for heterologous expression. Typically, however, the expression cassette is heterologous with respect to the host, i.e., the particular nucleic acid sequence of the expression cassette does not occur naturally in the host cell and must have been introduced into the host cell or an ancestor of the host cell by a transformation process known in the art. The expression of the nucleotide sequence in the expression cassette may be under the control of a constitutive promoter or of an inducible promoter that initiates transcription only when the host cell is exposed to some particular external stimulus. In the case of a multicellular organism, such as a plant, the promoter can also be specific to a particular tissue, or organ, or stage of development. An expression cassette, or fragment thereof, can also be referred to as "inserted sequence" or "insertion sequence" when transformed into a plant.

The term "expression" when used in reference to a nucleic acid sequence, such as a gene, refers to the process of converting genetic information encoded in a gene into RNA (e.g., mRNA, rRNA, tRNA, or snRNA) through "transcription" of the gene (*i.e.,* via the enzymatic action of an RNA polymerase), and into protein where applicable (when a gene encodes a protein), through "translation" of mRNA. Gene expression can be regulated at many stages in the process.

The term "chimeric gene" refers to any gene that contains 1) DNA sequences, including regulatory and coding sequences that are not found together in nature or 2) sequences encoding parts of proteins not naturally adjoined, or 3) parts of promoters that are not naturally adjoined. Accordingly, a chimeric gene may comprise regulatory sequences and coding sequences that are derived from different sources, or comprise regulatory sequences and coding sequences derived from the same source, but arranged in a manner different from that found in nature.

A "transgene" refers to a gene that has been introduced into the genome by transformation and is stably maintained. Transgenes may include, for example, genes that are either heterologous or homologous to the genes of a particular plant to be transformed. Additionally, transgenes may comprise native genes inserted into a non-native organism, or chimeric genes.

"Transformation" is a process for introducing heterologous nucleic acid into a host cell or organism. In particular, "transformation" means the stable integration of a DNA molecule into the genome of an organism of interest.

"Transformed / transgenic / recombinant" refer to a host organism such as a bacterium or a plant into which a heterologous nucleic acid molecule has been introduced. The nucleic acid molecule can be stably integrated into the genome of the host or the nucleic acid molecule can also be present as an extrachromosomal molecule. Such an extrachromosomal molecule can be auto-replicating. Transformed cells, tissues, or plants are understood to encompass not only the end product of a transformation process, but also transgenic progeny thereof. A "non-transformed", "non-transgenic", or "non- recombinant" host refers to a wild-type organism, e.g., a bacterium or plant, which does not contain the heterologous nucleic acid molecule. As used herein, "transgenic" refers to a plant, plant cell, or multitude of structured or unstructured plant cells having integrated, via well known techniques of genetic manipulation and gene insertion, a sequence of nucleic acid representing a gene of interest into the plant genome, and typically into a chromosome of a cell nucleus, mitochondria or other organelle containing chromosomes, at a locus different to, or in a number of copies greater than, that normally present in the native plant or plant cell. Transgenic plants result from the manipulation and insertion of such nucleic acid sequences, as opposed to naturally occurring mutations, to produce a non-naturally occurring plant or a plant with a non-naturally occurring genotype. Techniques for transformation of plants and plant cells are well known in the art and may comprise for example electroporation, microinjection, Agrobacterium-mediated transformation, and ballistic transformation.

The terms "protein," "peptide" and "polypeptide" are used interchangeably herein.

"Coding sequence" refers to a DNA or RNA sequence that codes for a specific amino acid sequence and excludes the non-coding sequences. It may constitute an "uninterrupted coding sequence", *i.e.,* lacking an intron, such as in a cDNA or it may include one or more introns bounded by appropriate splice junctions. An "intron" is a sequence of RNA which is contained in the primary transcript but which is removed through cleavage and re-ligation of the RNA within the cell to create the mature mRNA that can be translated into a protein.

"Promoter" refers to a nucleotide sequence, usually upstream (5') to its coding sequence, which controls the expression of the coding sequence by providing the recognition for RNA polymerase and other factors required for proper transcription. "Promoter" includes a minimal promoter that is a short DNA sequence comprised of a TATA box and other sequences that serve to specify the site of transcription initiation, to which regulatory elements are added for control of expression. "Promoter" also refers to a nucleotide sequence that includes a minimal promoter plus regulatory elements that is capable of controlling the expression of a coding sequence or functional RNA. This type of promoter sequence consists of proximal and more distal upstream elements, the latter elements often referred to as enhancers. Accordingly, an "enhancer" is a DNA sequence which can stimulate promoter activity and may be an innate element of the promoter or a heterologous element inserted to enhance the level or tissue specificity of a promoter. It is capable of operating in both orientations (normal or flipped), and is capable of functioning even when moved either upstream or downstream from the promoter. Both enhancers and other upstream promoter elements bind sequence-specific DNA-binding proteins that mediate their effects. Promoters may be derived in their entirety from a native gene, or be composed of different elements derived from different promoters found in nature, or even be comprised of synthetic DNA segments. A promoter may also contain DNA sequences that are involved in the binding of protein factors which control the effectiveness of transcription initiation in response to physiological or developmental conditions.

The "initiation site" is the position surrounding the first nucleotide that is part of the transcribed sequence, which is also defined as position +1. With respect to this site all other sequences of the gene and its controlling regions are numbered. Downstream sequences (*i.e.,* further protein encoding sequences in the 3' direction) are denominated positive, while upstream sequences (mostly of the controlling regions in the 5' direction) are denominated negative.

Promoter elements, particularly a TATA element, that are inactive or that have greatly reduced promoter activity in the absence of upstream activation are referred to as "minimal or core promoters." In the presence of a suitable transcription factor, the minimal promoter functions to permit transcription. A "minimal or core promoter" thus consists only of all basal elements needed for transcription initiation, *e.g.,* a TATA box and/or an initiator.

"Constitutive expression" refers to expression using a constitutive or regulated promoter. "Conditional" and "regulated expression" refer to expression controlled by a regulated promoter.

"Constitutive promoter" refers to a promoter that is able to express the open reading frame (ORF) that it controls in all or nearly all of the plant tissues during all or nearly all developmental stages of the plant. Each of the transcription-activating elements do not exhibit an absolute tissue-specificity, but mediate transcriptional activation in most plant parts at a level of ≥1% of the level reached in the part of the plant in which transcription is most active.

"Regulated promoter" refers to promoters that direct gene expression not constitutively, but in a temporally- and/or spatially-regulated manner, and includes both tissue-specific and inducible promoters. It includes natural and synthetic sequences as well as sequences which may be a combination of synthetic and natural sequences. Different promoters may direct the expression of a gene in different tissues or cell types, or at different stages of development, or in response to different environmental conditions. New promoters of various types useful in plant cells are constantly being discovered, numerous examples may be found in the compilation by Okamuro *et al.* (1989). Typical regulated promoters useful in plants include but are not limited to safener-inducible promoters, promoters derived from the tetracycline-inducible system, promoters derived from salicylate-inducible systems, promoters derived from alcohol-inducible systems, promoters derived from glucocorticoid-inducible system, promoters derived from pathogen-inducible systems, and promoters derived from ecdysone-inducible systems.

"Tissue-specific promoter" refers to regulated promoters that are not expressed in all plant cells but only in one or more cell types in specific organs (such as leaves or seeds), specific tissues (such as embryo or cotyledon), or specific cell types (such as leaf parenchyma or seed storage cells). These also include promoters that are temporally regulated, such as in early or late embryogenesis, during fruit ripening in developing seeds or fruit, in fully differentiated leaf, or at the onset of senescence.

"Inducible promoter" refers to those regulated promoters that can be turned on in one or more cell types by an external stimulus, such as a chemical, light, hormone, stress, or a pathogen.

"Operably linked" refers to the association of nucleic acid sequences on single nucleic acid fragment so that the function of one is affected by the other. For example, a regulatory DNA sequence is said to be "operably linked to" or "associated with" a DNA sequence that codes for an RNA or a polypeptide if the two sequences are situated such that the regulatory DNA sequence affects expression of the coding DNA sequence (*i.e.,* that the coding sequence or functional RNA is under the transcriptional control of the promoter). Coding sequences can be operably-linked to regulatory sequences in sense or antisense orientation.

"Expression" refers to the transcription and/or translation of an endogenous gene, ORF or portion thereof, or a transgene in plants. For example, in the case of antisense constructs, expression may refer to the transcription of the antisense DNA only. In addition, expression refers to the transcription and stable accumulation of sense (mRNA) or functional RNA. Expression may also refer to the production of protein.

"Overexpression" refers to the level of expression in transgenic cells or organisms that exceeds levels of expression in normal or untransformed (nontransgenic) cells or organisms.

"Antisense inhibition" refers to the production of antisense RNA transcripts capable of suppressing the expression of protein from an endogenous gene or a transgene.

"Gene silencing" refers to homology-dependent suppression of viral genes, transgenes, or endogenous nuclear genes. Gene silencing may be transcriptional, when the suppression is due to decreased transcription of the affected genes, or post-transcriptional, when the suppression is due to increased turnover (degradation) of RNA species homologous to the affected genes. Gene silencing includes virus-induced gene silencing.

"RNA interference" (RNAi) refers to the process of sequence-specific post-transcriptional gene silencing in plants and animals mediated by short interfering RNAs (siRNAs). Various terms such as siRNA, target RNA molecule, dicer or ribonuclease III enzyme are concepts known to those skilled in the art and full descriptions of these terms and other concepts pertinent to RNAi can be found in the literature. It is understood that any particular hypothesis describing the mechanisms of RNAi are not necessary to practice the present invention.

The term "siRNAs" refers to short interfering RNAs. In some embodiments, siRNAs comprise a duplex, or double-stranded region, of about 21-23 nucleotides long; often siRNAs contain from about two to four unpaired nucleotides at the 3' end of each strand. At least one strand of the duplex or double-stranded region of a siRNA is substantially homologous to or substantially complementary to a target RNA molecule. The strand complementary to a target RNA molecule is the "antisense strand;" the strand homologous to the target RNA molecule is the "sense strand," and is also complementary to the siRNA antisense strand. siRNAs may also contain additional sequences; non-limiting examples of such sequences include linking sequences, or loops, as well as stem and other folded structures. siRNAs appear to function as key intermediaries in triggering RNA interference in invertebrates and in vertebrates, and in triggering sequence-specific RNA degradation during posttranscriptional gene silencing in plants.

"dsRNA" or "double-stranded RNA" is RNA with two complementary strands, which directs the sequence-specific degradation of mRNA through a process known as RNA interference (RNAi). dsRNA is cut into siRNAs interfering with the expression of a specific gene.

The term "target RNA molecule" refers to an RNA molecule to which at least one strand of the short double-stranded region of a siRNA (or dsRNA) is homologous or complementary. Typically, when such homology or complementary is about 100% over a stretch of at least 21 nucleotides, the siRNA is able to silence or inhibit expression of the target RNA molecule. Although it is believed that processed mRNA is a target of siRNA, the present invention is not limited to any particular hypothesis, and such hypotheses are not necessary to practice the present invention. Thus, it is contemplated that other RNA molecules may also be targets of siRNA. Such RNA target molecules include unprocessed mRNA, ribosomal RNA, and viral RNA genomes. It is not necessary that there is 100% homology between the target RNA molecule and the dsRNA over the whole length of the dsRNA, but the hairpins of the dsRNA should comprise stretches of at least 21 nucleotides, preferably of at least 23 nucleotides, more preferred of at least 50 nucleotides, even more preferred of at least 500 nucleotides, most preferred of at least 700 nucleotides, and up to 1000 nucleotides having at least 95%, preferred 100% homology between the target RNA molecule.

The term "hybridize" as used herein refers to conventional hybridization conditions, preferably to hybridization conditions at which 5xSSPE, 1% SDS, 1xDenhardts solution is used as a solution and/or hybridization temperatures are between 35°C and 70°C, preferably 65°C. After hybridization, washing is preferably carried out first with 2xSSC, 1% SDS and subsequently with 0.2xSSC at temperatures between 35°C and 75°C, particularly between 45°C and 65°C, but especially at 59°C (regarding the definition of SSPE, SSC and Denhardts solution see Sambrook *et al. loc. cit.*). High stringency hybridization conditions as for instance described in Sambrook *et al*, supra, are particularly preferred. Particularly preferred stringent hybridization conditions are for instance present if hybridization and washing occur at 65°C as indicated above. Non-stringent hybridization conditions for instance with hybridization and washing carried out at 45°C are less preferred and at 35°C even less.

"Sequence Homology or Sequence Identity" is used herein interchangeably. The terms "identical" or percent "identity" in the context of two or more nucleic acid or protein sequences, refer to two or more sequences or subsequences that are the same or have a specified percentage of amino acid residues or nucleotides that are the same, when compared and aligned for maximum correspondence, as measured using one of the following sequence comparison algorithms or by visual inspection. If two sequences which are to be compared with each other differ in length, sequence identity preferably relates to the percentage of the nucleotide residues of the shorter sequence which are identical with the nucleotide residues of the longer sequence. Sequence identity can be determined conventionally with the use of computer programs such as the Bestfit program (Wisconsin Sequence Analysis Package, Version 8 for Unix, Genetics Computer Group, University Research Park, 575 Science Drive Madison, WI 53711). Bestfit utilizes the local homology algorithm of Smith and Waterman, Advances in Applied Mathematics 2 (1981), 482-489, in order to find the segment having the highest sequence identity between two sequences. When using Bestfit or another sequence alignment program to determine whether a particular sequence has for instance 95% identity with a reference sequence of the present invention, the parameters are preferably so adjusted that the percentage of identity is calculated over the entire length of the reference sequence and that homology gaps of up to 5% of the total number of the nucleotides in the reference sequence are permitted. When using Bestfit, the so-called optional parameters are preferably left at their preset ("default") values. The deviations appearing in the comparison between a given sequence and the above-described sequences of the invention may be caused for instance by addition, deletion, substitution, insertion or recombination. Such a sequence comparison can preferably also be carried out with the program "fasta20u66" (version 2.0u66, September 1998 by William R. Pearson and the University of Virginia; see also Pearson, 1990, appended examples and http://workbench.sdsc.edu/). For this purpose, the "default" parameter settings may be used.

Another indication that two nucleic acid sequences are "substantially identical" is that the two molecules hybridize to each other under stringent conditions. The phrase: "hybridizing specifically to" refers to the binding, duplexing, or hybridizing of a molecule only to a particular nucleotide sequence under stringent conditions when that sequence is present in a complex mixture (*e.g.,* total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence.

"Stringent conditions", "stringent hybridization conditions" or "stringent hybridization wash conditions" in the context of nucleic acid hybridization experiments, such as Southern and Northern hybridization, include reference to conditions under which a probe will hybridize to its target sequence, to a detectably greater degree than to other sequences. Stringent conditions are target sequence dependent, are different under different environmental parameters and will differ depending on the structure of the polynucleotide. Longer sequences hybridize specifically at higher temperatures. An extensive guide to the hybridization of nucleic acids is found in Tijssen P., 1993 Laboratory Techniques in Biochemistry and Molecular Biology-Hybridization with Nucleic Acid Probes, Part I, Chapter 2 "Overview of principles of hybridization and the strategy of nucleic acid probe assays" Elsevier, New York; and Current Protocols in Molecular Biology, Chapter 2, Ausubel et al., Eds., Greene Publishing and Wiley-Interscience: New York (1995), and also Sambrook et al. (2001) Molecular Cloning: A Laboratory Manual (5th Ed. Cold Spring Harbor Laboratory, Cold Spring Harbor, NY).

Specificity is typically the function of post-hybridization washes, the critical factors being the ionic strength and temperature of the final wash solution. Generally, highly stringent hybridization and wash conditions are selected to be about 5° lower than the thermal melting point (Tₘ) for the specific sequence at a defined ionic strength and pH. Typically, under "stringent conditions" a probe will hybridize to its target subsequence, but to no other sequences.

The Tₘ is the temperature (under defined ionic strength and pH) at which 50% of the target sequence hybridizes to a perfectly matched probe. Very stringent conditions are selected to be equal to the Tₘ for a particular probe. An example of stringent hybridization conditions for hybridization of complementary nucleic acids which have more than 100 complementary residues on a filter in a Southern or northern blot is 50% formamide with 1 mg of heparin at 42° C, with the hybridization being carried out overnight.

Often, a high stringency wash is preceded by a low stringency wash to remove background probe signal. An example of high stringent wash conditions is a 0.2 x SSC wash at 65° C for 15 minutes (see, Sambrook, *infra,* for a description of SSC buffer), whereas an example of very high stringent wash conditions is 0.15M NaCl at 72° C for about 15 minutes. An example moderate (medium) stringency wash for a duplex of, *e.g.,* more than 100 nucleotides, is 1X SSC at 45° C for 15 minutes. An example low stringency wash for a duplex of, *e.g.,* more than 100 nucleotides, is 4-6X SSC at 40° C for 15 minutes. For short probes (*e.g.,* about 10 to 50 nucleotides), stringent conditions typically involve salt concentrations of less than about 1.0M Na ion, typically about 0.01 to 1.0 M Na ion concentration (or other salts) at pH 7.0 to 8.3, and the temperature is typically at least about 30° C. Stringent conditions can also be achieved with the addition of destabilizing agents such as formamide. In general, a signal to noise ratio of 2 X (or higher) than that observed for an unrelated probe in the particular hybridization assay indicates detection of a specific hybridization. Nucleic acids that do not hybridize to each other under stringent conditions are still substantially identical if the proteins that they encode are substantially identical. This occurs, *e.g.,* when a copy of a nucleic acid is created using the maximum codon degeneracy permitted by the genetic code.

The following are exemplary sets of hybridization/wash conditions that may be used to hybridize nucleotide sequences that are substantially identical to reference nucleotide sequences of the present invention: a reference nucleotide sequence preferably hybridizes to the reference nucleotide sequence in 7% sodium dodecyl sulfate (SDS), 0.5 M NaPO₄, 1 mM EDTA at 50°C with washing in 2X SSC, 0.1% SDS at 50°C, more desirably in 7% sodium dodecyl sulfate (SDS), 0.5 M NaPO₄, 1 mM EDTA at 50°C with washing in 1X SSC, 0.1% SDS at 50°C, more desirably still in 7% sodium dodecyl sulfate (SDS), 0.5 M NaPO₄, 1 mM EDTA at 50°C with washing in 0.5X SSC, 0.1% SDS at 50°C, preferably in 7% sodium dodecyl sulfate (SDS), 0.5 M NaPO₄, 1 mM EDTA at 50°C with washing in 0.1X SSC, 0.1% SDS at 50°C, more preferably in 7% sodium dodecyl sulfate (SDS), 0.5 M NaPO₄, 1 mM EDTA at 50°C with washing in 0.1 X SSC, 0.1% SDS at 65°C. The sequences of the present invention may be detected using all the above conditions. For the purposes of defining the invention, the high stringency conditions are used.

A "plant" is any plant at any stage of development, particularly a seed plant.

A "plant cell" is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant.

"Plant cell culture" means cultures of plant units such as, for example, protoplasts, cell culture cells, cells in plant tissues, pollen, pollen tubes, ovules, embryo sacs, zygotes and embryos at various stages of development.

"Plant material" refers to leaves, stems, roots, flowers or flower parts, fruits, pollen, egg cells, zygotes, seeds, cuttings, cell or tissue cultures, or any other part or product of a plant. This also includes callus or callus tissue as well as extracts (such as extracts from taproots) or samples.

A "plant organ" is a distinct and visibly structured and differentiated part of a plant such as a root, stem, leaf, flower bud, or embryo. "Plant tissue" as used herein means a group of plant cells organized into a structural and functional unit. Any tissue of a plant in planta or in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue.

As used herein, the term "breeding", and grammatical variants thereof, refer to any process that generates a progeny individual. Breeding can be sexual or asexual, or any combination thereof. Exemplary non-limiting types of breeding include crossings, selfings, doubled haploid derivative generation, and combinations thereof.

"Selective breeding" is understood within the scope of the invention to refer to a program of breeding that uses plants that possess or display desirable traits as parents.

"Fermentation" as used herein refers to the process of transforming an organic molecule into another molecule using a micro-organism. For example, "fermentation" can refer to aerobic transforming sugars or other molecules from plant material, such as the plant material of the present invention, to produce alcohols (*e.g.,* ethanol, methanol, butanol); organic acids (*e.g.,* citric acid, acetic acid, itaconic acid, lactic acid, gluconic acid); ketones (*e.g.,* acetone), amino acids (*e.g.,* glutamic acid); gases (*e.g.,* H2 and CO2), antibiotics (*e.g.,* penicillin and tetracycline); enzymes; vitamins (e.g., riboflavin, B12, beta-carotene); and/or hormones. Fermentation include fermentations used in the consumable alcohol industry (e.g., beer and wine). Fermentation also includes anaerobic fermentations, for example, for the production of biofuels. Fermenting can be accomplished by any organism suitable for use in a desired fermentation step, including, but not limited to, bacteria, fungi, archaea, and protists. Suitable fermenting organisms include those that can convert mono-, di-, and trisaccharides, especially glucose and maltose, or any other biomass-derived molecule, directly or indirectly to the desired fermentation product (e.g., ethanol, butanol, etc.). Suitable fermenting organisms also include those which can convert non-sugar molecules to desired fermentation products. Such organisms and fermentation methods are known to the person skilled in the art.

The term "biofuel" as used herein refers to any biofuel produced by aerobic or anaerobic fermentation of plant material. A non-limiting example of a biofuel obtained by aerobic fermentation is bioethanol. Biofuels that can be obtained by anaerobic fermentation include, but are not limited to biogas and/or biodiesel. Methods of aerobic and/or anaerobic fermentation are known to the person skilled in the art.

### DETAILED DESCRIPTION

The present invention discloses transgenic sugar beet plants which have a phenotype of delayed bolting.

The cultivated sugar beet (*Beta vulgaris* ssp. *vulgaris* L.) is a biennial plant which forms a storage root and a leaf rosette in the first year. Shoot elongation (bolting) and flower formation starts after a period of low temperature, whereas many wild beets of the genus *B. vulgaris* ssp. *maritima* show an annual growing habit due to the presence of the bolting gene *B* at the *B* locus. The *BOLTING* gene (*B* gene) is responsible for the determination of the annual habit in sugar beet. Annuality in the *Beta* species is considered a monogenic and dominant trait. Plants carrying the dominant *B* allele are able to switch from juvenile to reproductive stages in a vernalization-independent manner, contrary to biennial plants carrying the b allele that obligatory require vernalization for bolting and subsequent flowering to occur. The dominant allele of locus *B* is abundant in wild beets and causes bolting under long days without the cold requirement usually essential for biennial cultivars (Abe *et al*., 1997) carrying the recessive allele. Although it was known that the *B* gene is a key player in the vernalization response in sugar beet via, the gene as such has not been identified so far.

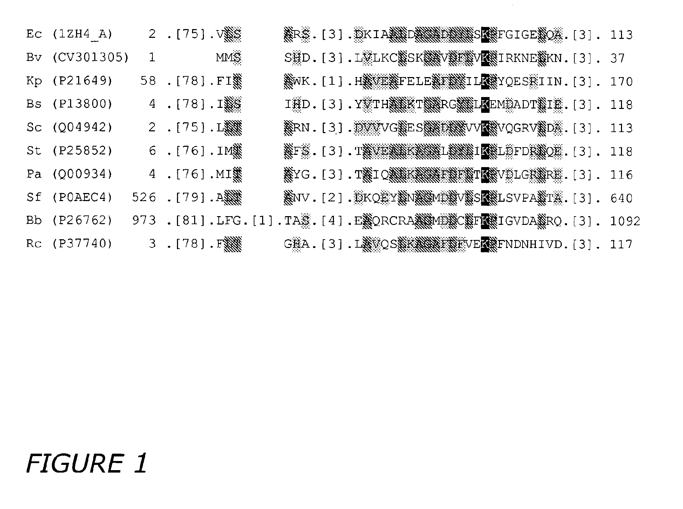

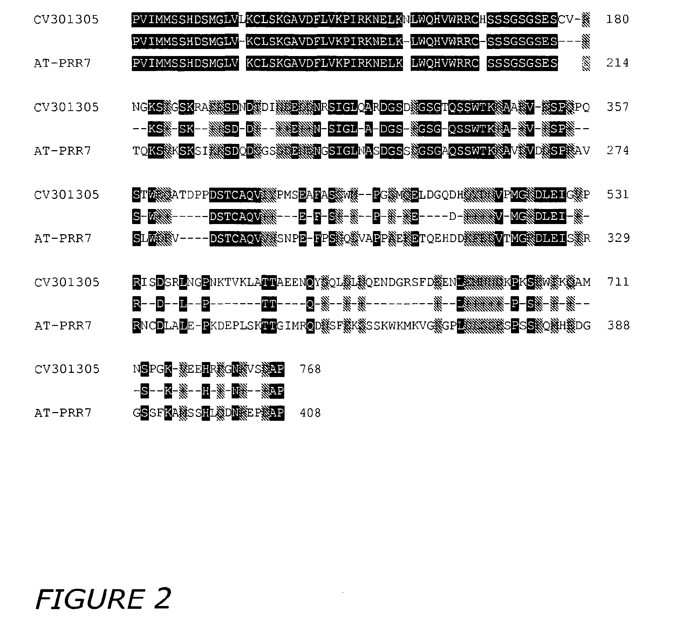

The present inventors now used a candidate gene approach in order to identify and characterize putative bolting control genes in sugar beet. In this approach an EST sequence with accession number CV301305 was identified as the putative beet homologue of *PRR7* by means of homology searches using BLAST (see Example 1.1). The corresponding amino acid sequence shows the partial presence of a Pseudo Response Regulator receiver (PRR, pfam00072) or Signal Receiver (REC, cd00156) domain (Figure 1), a hallmark of the PRR gene family that all play key roles in the circadian clock (Nakamichi *et al.,* 2005). Figure 2 shows the alignment of the amino acid sequence of CV301305 with *PRR7,* its closest *Arabidopsis* homologue. The PSEUDO RESPONSE REGULATOR 7 (PRR7) gene as originally described in *Arabidopsis* is a member of the pseudo-response regulator gene family (PRR1 or TOC1, PRR3, PRR5, PRR7 and PRR9) that all contain two characteristic signatures: the response regulator receiver (REC) and CCT domains. Transcription levels of the PRR family members oscillate in a circadian fashion, which suggest that their proteins are closely associated with the circadian clock. In fact PRR7 is described in *Arabidposis* as a component of the temperature-sensitive circadian system (Nakamichi *et al.,* 2007; Salomé and McClung 2005). In plants, the circadian clock is involved in the regulation of a number of fundamental biological processes, including leaf movement, diurnal changes in photosynthesis activities and photoperiodic control of flowering time (Imaizumi and Kay, 2006; Zhou *et al.,* 2007). Recently, PRR7 homologues were identified and characterized in barley, wheat and rice (HvPPD, TaPPD and OsPRR37) and shown to be major determinants of the photoperiod response in cereals.

In one aspect of the invention the sequences of several annual and biennial alleles of BvPRR7, preferably the sequences as given in SEQ ID NOs: 4, 5, 6, 7, 8, 9, 10, 53, or 54 are thus provided, which encode a protein which is functionally equivalent to the *B* gene.

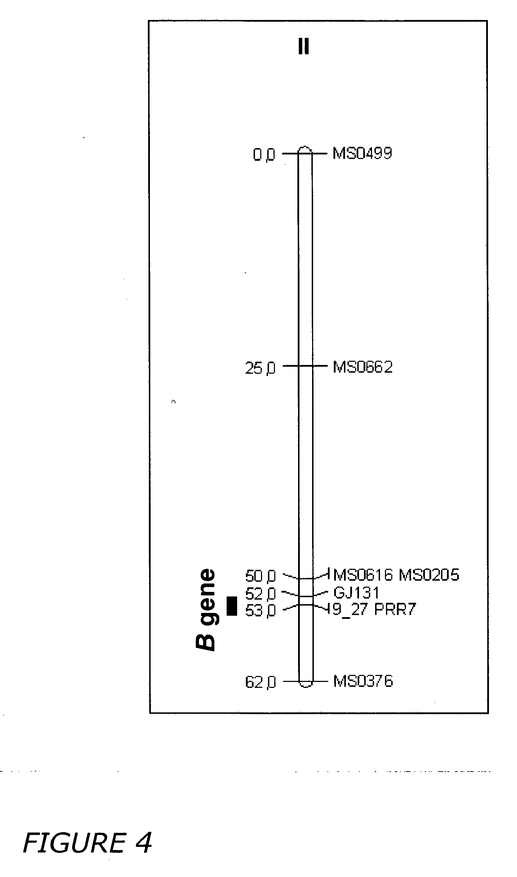

Based on the EST sequence a partial beet *PRR7* fragment of approximately 0.5 Kb as amplified and sequenced (see Example 1.1). Mapping experiments using a F2 population of 198 individuals derived from a cross between the annual line and a biennial line polymorphic for one SNP at position #160 showed that *BvPRR7* maps at chromosome II at an approximate distance of 1 cM downstream of the GJ131 marker (Figure 4), a region known to contain the *B* gene for vernalization-independent flowering (Mohring *et al.,* 2004; Gaafar *et al.,* 2005). The results of the marker assay show a perfect match between the predicted genotype of the *B* gene and the genotype of the *BvPRR7* gene (see Example 1,1). The results of further mapping analysis, *i.e*., its map position, combined with is biological function relating to the temperature-sensitive circadian rhythm (Salomé and McClung, 2005) showed that *BvPRR7* is a strong candidate for the *B* gene (Example 1.1).

In a next step a BAC library was screened using standard PCR techniques well known to those skilled in the art in order to recover the full-length genomic sequence of the sugar beet *PRR7* gene (see Example 1.2). The BAC library used was a BAC library which has been established with DNA from the biennial commercial sugar beet cultivar H20. Partially (Hindlll) digested HMW DNA of fragments in the size of 100-400 kb were size selected two times. The DNA fragments were ligated into the vector pBeloBAC-Kan. The library contains 57,600 clones with an average insert size of approximately 120 kb, corresponding to an 8X coverage of the beet genome. The redundancy has been tested by screening with single-copy probes and the frequency of clones from mitochondrial or plastid DNA was estimated to be lower than 1 %. Subsequent screenings of the DNA pools for fragment *BvPRR7* resulted in the positive identification of a BAC clone carrying the respective fragment.

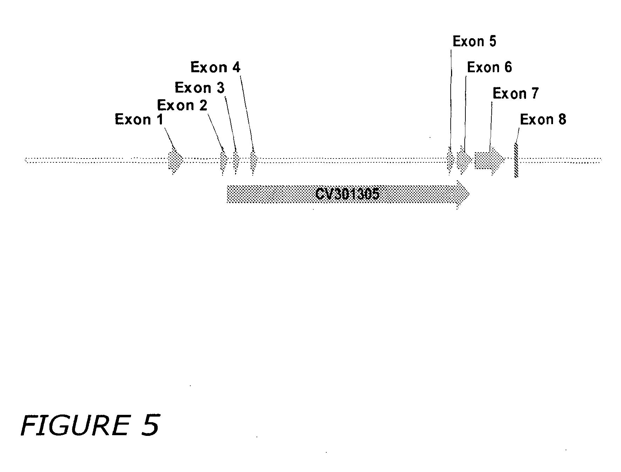

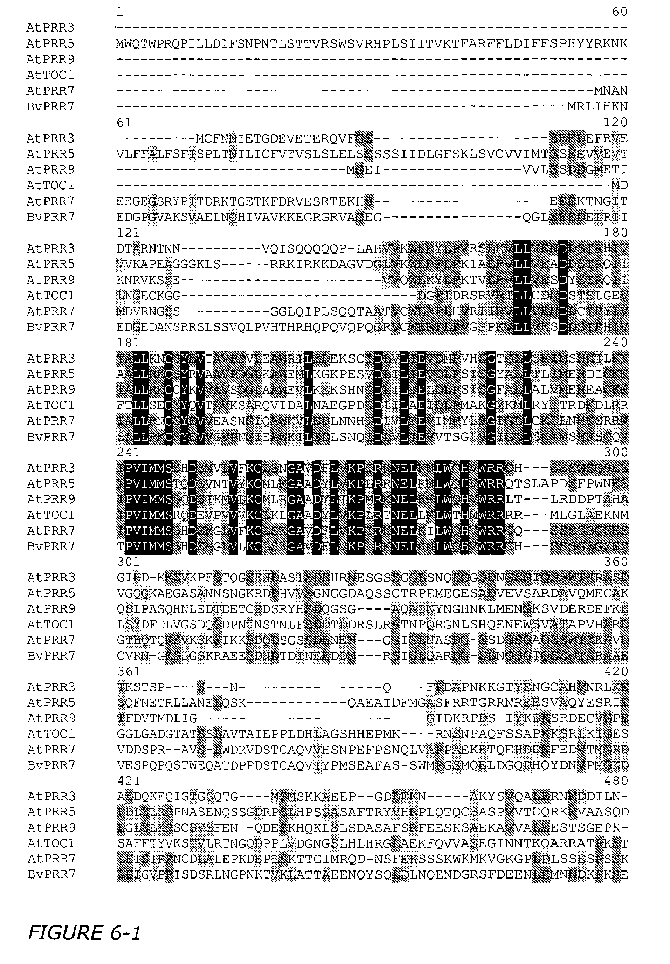

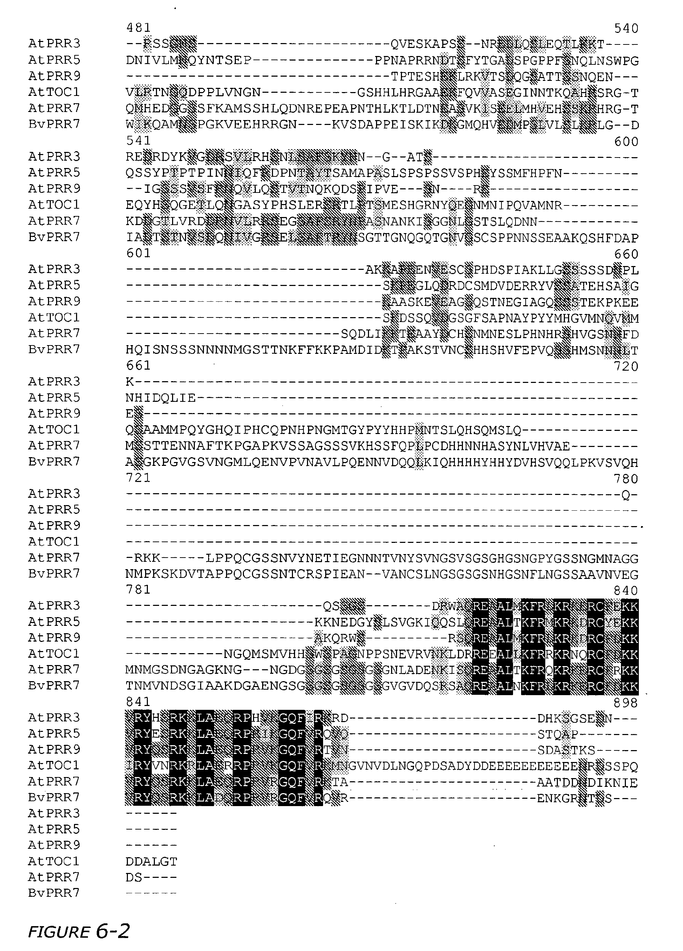

In order to obtain the full-length sequence of the *BvPRR7* gene, the previously identified BAC clone (BAC SBA079-L24) is sequenced using standard sequencing technology. Two non-overlapping contigs that both share sequence homology with EST CV301305 can then be combined into one single sequence (SEQ ID NO 8). Based on the alignment of the BAC sequence contigs to EST CV301305 and on sequence homology to the *PRR7* gene from *Arabidopsis,* the putative gene structure of the beet *BvPRR7* gene comprising introns and exons can be predicted as shown in Figure 5. Based on this prediction the genomic sequence can be shown to span the entire *BvPRR7* gene with 3.6 Kb of sequence upstream of the ATG stop codon and 2.2 Kb downstream of the coding region. The corresponding amino acid sequence of *BvPRR7* is shown under SEQ ID NO 11. Alignment of the amino acid sequence of *BvPRR7* to all members of the PRR gene family from *Arabidopsis* including *TOC1* (*PRR1*), *PRR3, PRR5, PRR7* and *PRR9* illustrates the strong conservation of the Pseudo Response Regulator receiver domain (PRR) motif (pfam00072) near the NH2-terminus and the CCT motif (pfam06203) at the COOH-terminus (Figure 6). In addition to the *PRR* gene family from *Arabidopsis, BvPRR7* also shares strong homology to the *PRR7* homologue in cereals as illustrated by the phylogenetic tree shown in Figure 7. The *PRR7* homologue in cereals, better known as *Ppd,* was shown to represent the major determinant of the photoperiod response (Turner *et al,* 2005; Beales *et al,* 2007). A function in the vernalization response as in sugar beet could not yet be demonstrated.

Based on their homology to known flowering-time control genes or their putative regulatory function as suggested by the presence of conserved domains representative of regulatory proteins, few genes can be identified as potential candidates for the *B* gene. These genes need further validation by allelic variability and/or gene expression studies between annual and biennial genotypes, or by means of complementation or knockout experiments using transgenic approaches. The annual plant habit conferred by the *B* gene behaves as a single dominant trait; the requirement for vernalization in biennial plants accordingly is recessive. The transformation of an annual allele of *BvPRR7* into a biennial genotype thus is predicted to bestow the annual flowering behavior onto the biennial acceptor genotype. To verify this hypothesis, the coding sequence of an annual allele of *BvPRR7* under the control of an annual promoter and terminator fragment is transformed into biennial genotype such as, for example G018 (see Example 2). Transformation can be accomplished by methods known in art such as that disclosed by Chang *et al,* 2002 using sugar beet meristems as explant material and the phosphomannose isomerase (PMI) gene as selectable marker. Transgenic shoots are checked for expression of the selection marker such as, for example, PMI activity (Joersbo *et al,* 1998) and subsequently rooted, potted in soil and transferred to the greenhouse. Negative controls consist of non-transgenic shoots that are subjected to the same *in vitro* regeneration procedure, but without *Agrobacterium* infection and selection. Plants are grown in growth chambers at a constant temperature of 18 °C and a photoperiod of 17 hours light and 7 hours dark. Under these conditions (without induction of bolting by applying cold temperatures) the non-transgenic biennial controls do not show any signs of bolting within an observation period of up to 12 weeks, whereas the annual control plants start to bolt normally within 6 to 8 weeks. Contrary to the non-transgenic biennial control plants, a substantial number of transgenic events starts bolting within four to ten weeks and basically behaves as annual plants despite their biennial genetic background. Transgenic plants that bolted and flowered are cross-pollinated with a biennial maintainer line to produce offspring. Progeny plants are tested for PMI activity and subsequently monitored for bolting and flowering without vernalization. These progeny plants show a one to one segregation ratio and a perfect correlation between PMI activity and the annual habit. These data confirm the causal relationship between *BvPRR7* and vernalization-independent flowering in sugar beet.

The present inventors further found out that *BvPRR7* plays a key role in the vernalization response in sugar beet and can thus be used for engineering bolting resistance into sugar beet plants by suppressing the vernalization response. In one aspect of the invention the *BvPRR7* gene may thus be used in a transgenic approach for producing transgenic sugar beet plants comprising said polynucleotides stably integrated into the sugar beet genome. In particular, upon expression from the genome, the expression product can be used to modulate the vernalization response of the sugar beet plant by suppressing or down-regulating expression of the B gene.

The DNA sequences of interest are assembled into chimeric constructs which contain the nucleic acid sequence to be expressed in the transgenic plant under the control of regulatory elements which function in plants. Methods for assembling such chimeric constructs are well known to the person skilled in the art.

Obtaining sufficient levels of transgene expression in the appropriate plant tissues is an important aspect in the production of genetically engineered crops. Expression of heterologous DNA sequences in a plant host is dependent upon the presence of an operably linked promoter that is functional within the plant host. Choice of the promoter sequence will determine when and where within the organism the heterologous DNA sequence is expressed.

For example, a plant promoter fragment may be employed which will direct expression of the gene in all tissue; of a regenerated plant. Such promoters are referred to herein as "constitutive" promoters and are active under most environmental conditions and states of development or cell differentiation. Examples of constitutive promoters include the cauliflower mosaic virus (CaMV) 35S transcription initiation region, the 1'- or 2'-promoter derived from T-DNA of Agrobacterium tumefaciens, and other transcription initiation regions from various plant genes known to those of skill. Such genes include for example, the AP2 gene, ACT11 from Arabidopsis (Huang et al. Plant Mol. Biol. 33:125-139 (1996)), Cat3 from Arabidopsis (GenBank No. U43147, Zhong et al., Mol. Gen. Genet. 251:196-203 (1996)), the gene encoding stearoyl-acyl carrier protein desaturase from Brassica napus (Genbank No. X74782, Solocombe et al. Plant Physiol. 104:1167-1176 (1994)), GPc1 from maize (GenBank No. X15596, Martinez et al. J. Mol. Biol 208:551-565 (1989)), and Gpc2 from maize (GenBank No. U45855, Manjunath et al., Plant Mol. Biol. 33:97-112 (1997)).

Alternatively, the plant promoter may direct expression of the nucleic acid molecules of the invention in a specific tissue or may be otherwise under more precise environmental or developmental control. Examples of environmental conditions that may affect transcription by inducible promoters include anaerobic conditions, elevated temperature, or the presence of light. Such promoters are referred to here as "inducible" or "tissue-specific" promoters. One of skill will recognize that a tissue-specific promoter may drive expression of operably linked sequences in tissues other than the target tissue. Thus, as used herein a tissue-specific promoter is one that drives expression preferentially in the target tissue, but may also lead to some expression in other tissues as well.

Examples of promoters under developmental control include promoters that initiate transcription only (or primarily only) in certain tissues, such as fruit, seeds, or flowers. Promoters that direct expression of nucleic acids in ovules, flowers or seeds are particularly useful in the present invention. As used herein a seed-specific or preferential promoter is one which directs expression specifically or preferentially in seed tissues, such promoters may be, for example, ovule-specific, embryo-specific, endosperm-specific, integument-specific, seed coat-specific, or some combination thereof. Examples include a promoter from the ovule-specific BEL1 gene described in Reiser et al. Cell 83:735-742 (1995) (GenBank No. U39944). Other suitable seed specific promoters are derived from the following genes: MAC1 from maize (Sheridan et al. Genetics 142:1009-1020 (1996), Cat3 from maize (GenBank No. L05934, Abler et al. Plant Mol. Biol. 22:10131-1038 (1993), the gene encoding oleosin 18 kD from maize (GenBank No, J05212, Lee et al. Plant Mol. Biol. 26:1981-1987 (1994)), vivparous-1 from *Arabidopsis* (Genbank No. U93215), the gene encoding oleosin from *Arabidopsis* (Genbank No. Z17657), Atmycl from *Arabidopsis* (Urao et al. Plant Mol. Biol. 32:571-576 (1996), the 2s seed storage protein gene family from *Arabidopsis* (Conceicao et al. Plant 5:493-505 (1994)) the gene encoding oleosin 20 kD from *Brassica napus* (GenBank No. M63985), napA from *Brassica napus* (GenBank No. J02798, Josefsson et al. JBL 26:12196-1301 (1987), the napin gene family from *Brassica napus* (Sjodahl et al. Planta 197:264-271 (1995), the gene encoding the 2S storage protein from *Brassica napus* (Dasgupta et al. Gene 133:301-302 (1993)), the genes encoding oleosin A (Genbank No. U09118) and oleosin B (Genbank No. U09119) from soybean and the gene encoding low molecular weight sulphur rich protein from soybean (Choi et al. Mol Gen, Genet. 246:266-268 (1995)).

Alternatively, particular sequences which provide the promoter with desirable expression characteristics, or the promoter with expression enhancement activity, could be identified and these or similar sequences introduced into the sequences via mutation. It is further contemplated that one could mutagenize these sequences in order to enhance their expression of transgenes in a particular species.

Furthermore, it is contemplated that promoters combining elements from more than one promoter may be useful. For example, U.S. Patent No. 5,491,288 discloses combining a Cauliflower Mosaic Virus (CaMV) promoter with a histone promoter. Thus, the elements from the promoters disclosed herein may be combined with elements from other promoters.

A variety of 5' and 3' transcriptional regulatory sequences are available for use in the present invention. Transcriptional terminators are responsible for the termination of transcription and correct mRNA polyadenylation. The 3' nontranslated regulatory DNA sequence preferably includes from about 50 to about 1,000, more preferably about 100 to about 1,000, nucleotide base pairs and contains plant transcriptional and translational termination sequences. Appropriate transcriptional terminators and those which are known to function in plants include the CaMV 35S terminator, the *tml* terminator, the nopaline synthase terminator, the pea rbcS E9 terminator, the terminator for the T7 transcript from the octopine synthase gene of *Agrobacterium tumefaciens,* and the 3' end of the protease inhibitor I or II genes from potato or tomato, although other 3' elements known to those of skill in the art can also be employed. Alternatively, one also could use a gamma coixin, oleosin 3 or other terminator from the genus Coix.

Preferred 3' elements include those from the nopaline synthase gene of *Agrobacterium tumefaciens* (Bevan *et al.,* 1983), the terminator for the T7 transcript from the octopine synthase gene of *Agrobacterium tumefaciens,* and the 3' end of the protease inhibitor I or II genes from potato or tomato.

As the DNA sequence between the transcription initiation site and the start of the coding sequence, *i.e*., the untranslated leader sequence, can influence gene expression, one may also wish to employ a particular leader sequence. Preferred leader sequences are contemplated to include those which include sequences predicted to direct optimum expression of the attached gene, *i.e*., to include a preferred consensus leader sequence which may increase or maintain mRNA stability and prevent inappropriate initiation of translation. The choice of such sequences will be known to those of skill in the art in light of the present disclosure. Sequences that are derived from genes that are highly expressed in plants will be most preferred.

Other sequences that have been found to enhance gene expression in transgenic plants include intron sequences (*e.g.,* from *Adh1, bronze1, actin1, actin 2* (WO 00/760067), or the sucrose synthase intron) and viral leader sequences (e.g., from TMV, MCMV and AMV). For example, a number of non-translated leader sequences derived from viruses are known to enhanced expression. Specifically, leader sequences from Tobacco Mosaic Virus (TMV), Maize Chlorotic Mottle Virus (MCMV), and Alfalfa Mosaic Virus (AMV) have been shown to be effective in enhancing expression (*e.g*., Gallie *et al.,* 1987; Skuzeski *et al.,* 1990). Other leaders known in the art include but are not limited to: Picornavirus leaders, for example, EMCV leader (Encephalomyocarditis 5 noncoding region) (Elroy-Stein *et al.,* 1989); Potyvirus leaders, for example, TEV leader (Tobacco Etch Virus); MDMV leader (Maize Dwarf Mosaic Virus); Human immunoglobulin heavy-chain binding protein (BiP) leader, (Macejak *et al.,* 1991); Untranslated leader from the coat protein mRNA of alfalfa mosaic virus (AMV RNA 4), (Jobling *et al.,* 1987; Tobacco mosaic virus leader (TMV), (Gallie *et al.,* 1989; and Maize Chlorotic Mottle Virus leader (MCMV) (Lommel *et al.,* 1991. See also, Della-Cioppa *et al.,* 1987.

Regulatory elements such as *Adh* intron 1 (Callis *et al.,* 1987), sucrose synthase intron (Vasil *et al.,* 1989) or TMV omega element (Gallie, *et al.,* 1989), may further be included where desired.

Examples of enhancers include elements from the CaMV 35S promoter, octopine synthase genes (Ellis el al., 1987), the rice actin I gene, the maize alcohol dehydrogenase gene (Callis *et al.,* 1987), the maize shrunken I gene (Vasil *et al.,* 1989), TMV Omega element (Gallie *et al.,* 1989) and promoters from non-plant eukaryotes (*e.g*., yeast; Ma *et al.,* 1988).

One principal method for the control of expression is underexpression. For underexpression there are two principle methods which are commonly referred to in the art as "antisense downregulation" and "sense downregulation" (sense downregulation is also referred to as "cosuppression"). Generically these processes are referred to as "gene silencing". Both of these methods lead to an inhibition of expression of the target gene.

The invention comprises various strategies for reducing the expression, amount, activity and/or function of the of the nucleic acid molecule of the present invention. The skilled worker appreciates the fact that a number of various methods are available in order to influence the expression, amount, activity and/or function of the nucleic acid molecule of the present invention in the desired way. Examples which may be mentioned but which are not limiting are:

### "Sense" Suppression

Alteration of the expression of a nucleotide sequence of the present invention, preferably reduction of its expression, is obtained by "sense" suppression (referenced in *e.g*., Jorgensen et al. (1996) Plant Mol. Biol. 31, 957-973). In this case, the entirety or a portion of a nucleotide sequence of the present invention is comprised in a DNA molecule. The DNA molecule is preferably operatively linked to a promoter functional in a cell comprising the target gene, preferably a plant cell, and introduced into the cell, in which the nucleotide sequence is expressible. The nucleotide sequence is inserted in the DNA molecule in the "sense orientation", meaning that the coding strand of the nucleotide sequence can be transcribed. In a preferred embodiment, the nucleotide sequence is fully translatable and all the genetic information comprised in the nucleotide sequence, or portion thereof, is translated into a polypeptide. In another preferred embodiment, the nucleotide sequence is partially translatable and a short peptide is translated. In a preferred embodiment, this is achieved by inserting at least one premature stop codon in the nucleotide sequence, which brings translation to a halt. In another more preferred embodiment, the nucleotide sequence is transcribed but no translation product is being made. This is usually achieved by removing the start codon, *e.g*., the "ATG", of the polypeptide encoded by the nucleotide sequence. In a further preferred embodiment, the DNA molecule comprising the nucleotide sequence, or a portion thereof, is stably integrated in the genome of the plant cell. In another preferred embodiment, the DNA molecule comprising the nucleotide sequence, or a portion thereof, is comprised in an extrachromosomally replicating molecule.

In transgenic plants containing one of the DNA molecules described immediately above, the expression of the nucleotide sequence corresponding to the nucleotide sequence comprised in the DNA molecule is preferably reduced. Preferably, the nucleotide sequence in the DNA molecule is at least 80% identical to the nucleotide sequence the expression of which is reduced, more preferably it is at least 90% identical, yet more preferably at least 95% identical, and most preferably at least 99% identical.

### "Anti-sense" Suppression

In another preferred embodiment, the alteration of the expression of a nucleotide sequence of the present invention, preferably the reduction of its expression is obtained by "anti-sense" suppression. The entirety or a portion of a nucleotide sequence of the present invention is comprised in a DNA molecule. The DNA molecule is preferably operatively linked to a promoter functional in a plant cell, and introduced in a plant cell, in which the nucleotide sequence is expressible. The nucleotide sequence is inserted in the DNA molecule in the "anti-sense orientation", meaning that the reverse complement (also called sometimes non-coding strand) of the nucleotide sequence can be transcribed. In a preferred embodiment, the DNA molecule comprising the nucleotide sequence, or a portion thereof, is stably integrated in the genome of the plant cell. In another preferred embodiment the DNA molecule comprising the nucleotide sequence, or a portion thereof, is comprised in an extrachromosomally replicating molecule. Several publications describing this approach are cited for further illustration (Green, P. J. et al., Ann. Rev. Biochem. 55:569-597 (1986); van der Krol, A. R. et al, Antisense Nuc. Acids & Proteins, pp. 125-141 (1991); Abel, P. P. et al., Proc. Natl. Acad. Sci. USA 86:6949-6952 (1989); Ecker, J. R. et al., Proc. Natl. Acad. Sci. USA 83:5372-5376 (Aug. 1986)).

In transgenic plants containing one of the DNA molecules described immediately above, the expression of the nucleotide sequence corresponding to the nucleotide sequence comprised in the DNA molecule is preferably reduced. Preferably, the nucleotide sequence in the DNA molecule is at least 80% identical to the nucleotide sequence the expression of which is reduced, more preferably it is at least 90% identical, yet more preferably at least 95% identical, and most preferably at least 99% identical.

### Homologous Recombination

In another preferred embodiment, at least one genomic copy corresponding to a nucleotide sequence of the present invention is modified in the genome of the plant by homologous recombination as further illustrated in Paszkowski et al., EMBO Journal 7:4021-26 (1988). This technique uses the property of homologous sequences to recognize each other and to exchange nucleotide sequences between each by a process known in the art as homologous recombination. Homologous recombination can occur between the chromosomal copy of a nucleotide sequence in a cell and an incoming copy of the nucleotide sequence introduced in the cell by transformation. Specific modifications are thus accurately introduced in the chromosomal copy of the nucleotide sequence. In one embodiment, the regulatory elements of the nucleotide sequence of the present invention are modified. Such regulatory elements are easily obtainable by screening a genomic library using the nucleotide sequence of the present invention, or a portion thereof, as a probe. The existing regulatory elements are replaced by different regulatory elements, thus altering expression of the nucleotide sequence, or they are mutated or deleted, thus abolishing the expression of the nucleotide sequence. In another embodiment, the nucleotide sequence is modified by deletion of a part of the nucleotide sequence or

the entire nucleotide sequence, or by mutation. Expression of a mutated polypeptide in a plant cell is also contemplated as herein described. More recent refinements of this technique to disrupt endogenous plant genes have been described (Kempin et al., Nature 389:802-803 (1997) and Miao and Lam, Plant J., 7:359-365 (1995).

The skilled worker knows numerous possible processes of how to modify genomic sequences in a targeted manner. These include, in particular, processes such as the generation of knockout mutants by means of targeted homologous recombination, for example, by generating stop codons, shifts in the reading frame etc. (Hohn B and Puchta H (1999) Proc Natl Acad Sci USA 96:8321-8323) or the targeted deletion or inversion of sequences by means of, for example, sequence-specific recombinases or nucleases. In another preferred embodiment, a mutation in the chromosomal copy of a nucleotide sequence is introduced by transforming a cell with a chimeric oligonucleotide composed of a contiguous stretch of RNA and DNA residues in a duplex conformation with double hairpin caps on the ends. An additional feature of the oligonucleotide is for example the presence of 2'-O-methylation at the RNA residues. The RNA/DNA sequence is designed to align with the sequence of a chromosomal copy of a nucleotide sequence of the present invention and to contain the desired nucleotide change. For example, this technique is further illustrated in US patent 5,501,967 and Zhu et al. (1999) Proc. Natl. Acad. Sci. USA 96: 8768-8773.

### Ribozymes

The RNA coding for a polypeptide as herein described is cleaved by a catalytic RNA, or ribozyme, specific for such RNA. The ribozyme is expressed in transgenic plants and results in reduced amounts of RNA coding for the polypeptide as herein described in plant cells, thus leading to reduced amounts of polypeptide accumulated in the cells. This method is further illustrated in US patent 4,987,071.

### Dominant-Negative Mutants

The activity of the polypeptide encoded by the nucleotide sequences as herein described is changed. This is achieved by expression of dominant negative mutants of the proteins in transgenic plants, leading to the loss of activity of the endogenous protein.

### Aptamers

The activity of polypeptide as herein described is inhibited by expressing in transgenic plants nucleic acid ligands, so-called aptamers, which specifically bind to the protein. Aptamers are preferentially obtained by the SELEX (Systematic Evolution of Ligands by Exponential Enrichment) method. In the SELEX method, a candidate mixture of single stranded nucleic acids having regions of randomized sequence is contacted with the protein and those nucleic acids having an increased affinity to the target are partitioned from the remainder of the candidate mixture. The partitioned nucleic acids are amplified to yield a ligand enriched mixture. After several iterations a nucleic acid with optimal affinity to the polypeptide is obtained and is used for expression in transgenic plants. This method is further illustrated in US patent 5,270,163.

### Zinc finger proteins

A zinc finger protein that binds a nucleotide sequence of the present invention or to its regulatory region is also used to alter expression of the nucleotide sequence. Preferably, transcription of the nucleotide sequence is reduced or increased. Zinc finger proteins are for example described in Beerli et al. (1998) PNAS 95:14628-14633., or in WO 95/19431, WO 98/54311, or WO 96/06166.

### dsRNA

Alteration of the expression of a nucleotide sequence of the present invention is also obtained by dsRNA interference (RNAi)The process of gene regulation by means of double-stranded RNA ("double-stranded RNA interference"; dsRNAi) has been described many times for animal and plant organisms (*e.g*., Matzke M A et al. (2000) Plant Mol Biol 43:401-415; Fire A. et al. (1998) Nature 391:806-811; WO 99/32619; WO 99/53050; WO 00/68374; WO 00/44914; WO 00/44895; WO 00/49035; WO 00/63364). The processes and methods described in the references indicated are hereby explicitly referred to dsRNAi processes are based on the phenomenon that simultaneously introducing the complementary strand and contour strand of a gene transcript suppresses expression of the corresponding gene in a highly efficient manner. Preferably, the phenotype caused is very similar to that of a corresponding knockout mutant (Waterhouse P M et al. (1998) Proc Natl Acad Sci USA 95:13959-64). The dsRNAi process has proved to be particularly efficient and advantageous in reducing marker protein expression.

Double-stranded RNA (dsRNA) molecule means within the scope of the invention preferably one or more ribonucleic acid sequences which, owing to complementary sequences, are theoretically (e.g. according to the base pair rules by Watson and Crick) and/or actually (e.g. owing to hybridization experiments *in vitro* and/or *in vivo*) capable of forming double-stranded RNA structures. The skilled worker is aware of the fact that the formation of double-stranded RNA structures represents a state of equilibrium. Preferably, the ratio of double-stranded molecules to corresponding dissociated forms is at least 1 to 10, preferably 1:1, particularly preferably 5:1, most preferably 10:1.

The present disclosure further relates to double-stranded RNA molecules which, when introduced into a plant organism (or into a cell, tissue, organ or propagation material derived therefrom) cause the reduction of expression of at least one target gene. The double-stranded RNA molecule for reducing expression of a target gene here preferably comprises a) a "sense" RNA strand comprising at least one ribonucleotide sequence which is essentially identical to at least a part of the "sense" RNA transcript of a target gene, and b) an "antisense" RNA strand which is essentially, preferably fully, complementary to the RNA sense strand under a).

"Essentially identical" means that the dsRNA sequence may also have insertions, deletions and also individual point mutations in comparison with the target gene sequence and nevertheless causes an efficient reduction in expression. The homology (as defined herein below) between the "sense" strand of an inhibitory dsRNA and at least one part of the "sense" RNA transcript of a target gene nucleic acid sequence (or between the "antisense" strand of the complementary strand of a nucleic acid sequence of a target gene) is preferably at least 75%, preferably at least 80%, very particularly preferably at least 90%, most preferably 100%.

A 100% sequence identity between dsRNA and a marker protein gene transcript is not absolutely necessary in order to cause an efficient reduction in target gene expression. Consequently, the process is advantageously tolerant toward sequence deviations as may be present due to genetic mutations, polymorphisms or evolutionary divergences. Thus it is possible, for example, using the dsRNA which has been generated starting from the sequence of the target gene of the first organism, to suppress target gene expression in a second organism. For this purpose, the dsRNA preferably includes sequence regions of the target gene transcripts which correspond to conserved regions. Said conserved regions may be readily derived from sequence comparisons.

Alternatively, an "essentially identical" dsRNA may also be defined as a nucleic acid sequence capable of hybridizing with part of a target gene transcript.

"Essentially complementary" means that the "antisense" RNA strand may also have insertions, deletions and also individual point mutations in comparison with the complement of this "sense" RNA strand. The homology between the "antisense" RNA strand and the complement of the "sense" RNA strand is preferably at least 80%, preferably at least 90%, very particularly preferably at least 95%, most preferably 100%.

"Part of the "sense" RNA transcript" of a nucleic acid sequence of a target gene means fragments of an RNA or mRNA transcribed or transcribable from a nucleic acid sequence of a target gene. In this context, the fragments have a sequence length of preferably at least 20 bases, preferably at least 50 bases, particularly preferably at least 100 bases, very particularly preferably at least 200 bases, most preferably at least 500 bases. The complete transcribable RNA or mRNA is also included. Included are also sequences such as those which may be transcribed under artificial conditions from regions of a target gene which are otherwise, under natural conditions, not transcribed, such as promoter regions, for example.

The dsRNA may consist of one or more strands of polyribonucleotides. Naturally, in order to achieve the same purpose, it is also possible to introduce a plurality of individual dsRNA molecules which comprise in each case one of the above-defined ribonucleotide sequence sections into the cell or the organism. The double-stranded dsRNA structure may be formed starting from two complementary, separate RNA strands or, preferably, starting from a single, self-complementary RNA strand. In this case, the "sense" RNA strand and the "antisense" RNA strand are preferably connected covalently to one another in the form of an inverted "repeat".

As described in WO 99/53050, for example, the dsRNA may also comprise a hairpin structure by connecting the "sense" and the "antisense" strands by a connecting sequence ("linker"; for example an intron). Preference is given to the self-complementary dsRNA structures, since they require only the expression of an RNA sequence and always comprise the complementary RNA strands in an equimolar ratio. The connecting sequence may is preferably an intron (e.g. an intron of the potato ST-LS1 gene; Vancanneyt G F et al. (1990) Mol Gen Genet 220(2):245-250).

The nucleic acid sequence coding for a dsRNA may include further elements such as, for example, transcription termination signals or polyadenylation signals.

Bringing together, if intended, the two strands of the dsRNA in a cell or plant may be achieved by way of example in the following way: a) transformation of the cell or plant with a vector comprising both expression cassettes, b) cotransformation of the cell or plant with two vectors, one of which comprises the expression cassettes containing the "sense" strand and the other one of which comprises the expression cassettes containing the "antisense" strand. The formation of the RNA duplex may be initiated either outside or inside the cell.

The dsRNA may be synthesized either *in vivo* or *in vitro.* For this purpose, a DNA sequence coding for a dsRNA may be inserted into an expression cassette under the control of at least one genetic control element (such as a promoter, for example). A polyadenylation is not necessary and neither need any elements for initiating a translation be present. Preference is given to the expression cassette for the dsRNA targeting the target gene being present on the transformation construct or the transformation vector. For this purpose, the expression cassettes coding for the "antisense" strand and/or the "sense" strand of a dsRNA targeting the target gene or for the self-complementary strand of the dsRNA are preferably inserted into a transformation vector and introduced into the plant cell by using the processes described below. A stable insertion into the genome may be advantageous for the process of the invention but is not absolutely necessary. Since a dsRNA causes a long-term effect, transient expression is also sufficient in many cases. The dsRNA may also be part of the RNA to be expressed by the nucleic acid sequence to be inserted by fusing it, for example, to the 3'-untranslated part of said RNA.

The dsRNA may be introduced in an amount which makes possible at least one copy per cell. Higher amounts (e.g. at least 5, 10, 100, 500 or 1000 copies per cell) may, if appropriate, cause a more efficient reduction.

For RNAi suppression a *BvPRR7* the present inventors have assembled a cDNA fragment such as, for example the 0.6 Kb fragment depicted in SEQ ID NO: 1, into an RNAi cassette under the control of a constitutive promoter (see Example 3). Suitable constitutive promoters are, for example, the Ubi3 promoter from *Arabidopsis* (Norris *et al,* 1993), the CaMV 35S promoter, or any other promoter known to promote constitutive expression in sugar beet. The expression cassette further contains a selectable marker gene under the control of a suitable promoter. Particularly, the marker gene encodes a positive selection marker such as phosphomannose isomerase or a xylose isomerase. The inverted repeat of the *BvPRR7* fragment is separated by the second intron from the potato StLS1 gene (Eckes *et al,* 1986; Vancanneyt *et al,* 1990) to stabilize the RNAi cassette, but also to improve the efficiency of the RNAi phenomenon (Wang and Waterhouse, 2001; Smith *et al,* 2000).

### Insertion of a DNA molecule (Insertional mutagenesis)

In another preferred embodiment, a DNA molecule is inserted into a chromosomal copy of a nucleotide sequence of the present invention, or into a regulatory region thereof. Preferably, such DNA molecule comprises a transposable element capable of transposition in a plant cell, such as *e.g*., Ac/Ds, Em/Spm, mutator. Alternatively, the DNA molecule comprises a T-DNA border of an Agrobacterium T-DNA. The DNA molecule may also comprise a recombinase or integrase recognition site which can be used to remove part of the DNA molecule from the chromosome of the plant cell. Methods of insertional mutagenesis using T-DNA, transposons, oligonucleotides or other methods known to those skilled in the art are also encompassed. Methods of using T-DNA and transposon for insertional mutagenesis are described in Winkler et al. (1989) Methods Mol. Biol. 82:129-136 and Martienssen (1998) PNAS 95:2021-2026. Further suitable methods are the introduction of nonsense mutations into endogenous target genes, for example, by means of introducing RNA/DNA oligonucleotides into the plant (Zhu et al. (2000) Nat Biotechnol 18(5):555-558). Point mutations may also be generated by means of DNA-RNA hybrids which are also known as "chimeraplasty" (Cole-Strauss et al. (1999) Nucl Acids Res 27(5):1323-1330; Kmiec (1999) Gene therapy American Scientist 87(3):240-247).

### Deletion mutagenesis

In yet another embodiment, a mutation of a nucleic acid molecule of the present invention is created in the genomic copy of the sequence in the cell or plant by deletion of a portion of the nucleotide sequence or regulator sequence. Methods of deletion mutagenesis are known to those skilled in the art. See, for example, Miao et al, (1995) Plant J. 7:359. The activity or amount of the expression of a target gene may also be reduced by a targeted deletion in the target gene, for example by sequence-specific induction of DNA double-strand breaks at a recognition sequence for specific induction of DNA double-strand breaks in or close to the nucleic acid sequence of the target gene.

In yet another embodiment, this deletion is created at random in a large population of plants by chemical mutagenesis or irradiation and a plant with a deletion in a gene of the present invention is isolated by forward or reverse genetics. Irradiation with fast neutrons or gamma rays is known to cause deletion mutations in plants (Silverstone et al, (1998) Plant Cell, 10:155-169; Bruggemann et al., (1996) Plant J., 10:755-760; Redei and Koncz in Methods in Arabidopsis Research, World Scientific Press (1992), pp. 16-82). Deletion mutations in a gene of the present invention can be recovered in a reverse genetics strategy using PCR with pooled sets of genomic DNAs as has been shown in *C. elegans* (Liu et al., (1999), Genome Research, 9:859-867.). A forward genetics strategy would involve mutagenesis of a line displaying PTGS followed by screening the M2 progeny for the absence of PTGS. Among these mutants would be expected to be some that disrupt a gene of the present invention. This could be assessed by Southern blot or PCR for a gene of the present invention with genomic DNA from these mutants.

In still another embodiment, the expression of the nucleotide sequence of the present invention is altered in every cell of a plant. This is for example obtained though homologous recombination or by insertion in the chromosome. This is also for example obtained by expressing a sense or antisense RNA, zinc finger protein or ribozyme under the control of a promoter capable of expressing the sense or antisense RNA, zinc finger protein or ribozyme in every cell of a plant. Constructs for expression of the sense or antisense RNA, zinc finger protein or ribozyme, or for overexpression of a nucleotide sequence of the present invention, are prepared and transformed into a plant cell according to the teachings of the present invention, e.g., as described infra.

A combined application is also conceivable. Further methods are known to the skilled worker and may comprise hindering or stopping the processing of the target gene, the transport of the protein encoded by the target gene or of its mRNA, the inhibition of ribosome attachment, the inhibition of RNA splicing, the induction of an enzyme degrading target gene RNA and/or the inhibition of translational elongation or termination.

The disclosure hence also provides sense and anti-sense nucleic ,acid molecules corresponding to the sequences set forth in SEQ ID NOs: 1, 4, 5, 6, 7, 8, 9, 10, 53, or 54 of the Sequence Listing as well as their orthologs.

The genes and open reading frames according to the present invention which are substantially similar to a nucleotide sequence encoding a polypeptide as given in SEQ ID NO: 6 including any corresponding anti-sense constructs can be operably linked to any promoter that is functional within the plant host including the promoter sequences according to the invention or mutants thereof.

Once completed, the polynucleotide construct of the invention comprising an expression cassette or an RNAi cassette may be mobilized into a suitable vector for plant transformation, such as, for example, a binary vector, which may then be mobilized to sugar beet using one of the well known transformation techniques such as, for example, *Agrobacterium*-mediated transformation.

Transgenic plants (or plant cells, or plant explants, or plant tissues) incorporating and expressing the nucleic acid sequences or dsRNA of the invention can be produced by a variety of well established techniques. Following construction of the chimeric construct of the invention comprising an expression cassette or an RNAi cassette incorporating a nucleic acid sequence according to the invention and as described herein before, standard techniques can be used to introduce the chimeric construct into a plant, a plant cell, a plant explant or a plant tissue of interest. Optionally, the plant cell, explant or tissue can be regenerated to produce a transgenic plant. The plant can be any higher plant, including gymnosperms, monocotyledonous and dicotyledonous plants. Suitable protocols are available for Leguminosae (alfalfa, soybean, clover, etc.), Umbelliferae (carrot, celery, parsnip), Cruciferae (cabbage, radish, rapeseed, broccoli, etc.), Curcurbitaceae (melons and cucumber), Gramineae (wheat, corn, rice, barley, millet, etc.), Solanaceae (potato, tomato, tobacco, peppers, etc.), and various other crops. See protocols described in Ammirato et al., eds., (1984) Handbook of Plant Cell Culture--Crop Species, Macmillan Publ. Co., New York, N.Y.; Shimamoto et al. (1989) Nature 338: 274 276; Fromm et al. (1990) Bio/Technol. 8: 833 839; and Vasil et al. (1990) Bio/Technol. 8: 429 434. Transformation and regeneration of both monocotyledonous and dicotyledonous plant cells is now routine, and the selection of the most appropriate transformation technique will be determined by the practitioner. The choice of method will vary with the type of plant to be transformed; those skilled in the art will recognize the suitability of particular methods for given plant types. Suitable methods can include, but are not limited to: electroporation of plant protoplasts; liposome-mediated transformation; polyethylene glycol (PEG) mediated transformation; transformation using viruses; micro-injection of plant cells; micro-projectile bombardment of plant cells; vacuum infiltration; and *Agrobacterium tumefaciens* mediated transformation.

Transformation of plants can be undertaken with a single DNA molecule or multiple DNA molecules (*i.e*., co-transformation), and both these techniques are suitable for use with the chimeric constructs of the present invention. Numerous transformation vectors are available for plant transformation, and the expression cassettes of this invention can be used in conjunction with any such vectors. The selection of vector will depend upon the preferred transformation technique and the target species for transformation.

A variety of techniques are available and known to those skilled in the art for introduction of constructs into a plant cell host. These techniques generally include transformation with DNA employing *A. tumefaciens* or *A. rhizogenes* as the transforming agent, liposomes, PEG precipitation, electroporation, DNA injection, direct DNA uptake, microprojectile bombardment, particle acceleration, and the like (See, for example, EP 295959 and EP 138341) (see below). However, cells other than plant cells may be transformed with the polynucleotide construct of the invention. The general descriptions of plant expression vectors and reporter genes, and *Agrobacterium* and *Agrobacterium*-mediated gene transfer, can be found in Gruber *et al.* (1993).

Expression vectors containing a nucleic acid sequence according to the invention can be introduced into protoplasts or into intact tissues or isolated cells. Preferably expression vectors are introduced into intact tissue. General methods of culturing plant tissues are provided for example by Maki *et al.,* (1993); and by Phillips *et al.* (1988). Preferably, expression vectors are introduced into maize or other plant tissues using a direct gene transfer method such as microprojectile-mediated delivery, DNA injection, electroporation and the like. More preferably expression vectors are introduced into plant tissues using the microprojectile media delivery with the biolistic device. See, for example, Tomes *et al.* (1995). The vectors of the invention can not only be used for expression of structural genes but may also be used in exon-trap cloning, or promoter trap procedures to detect differential gene expression in varieties of tissues, (Lindsey *et al.,* 1993; Auch & Reth *et al*.).

It is particularly preferred to use the binary type vectors of Ti and Ri plasmids of *Agrobacterium spp.* Ti-derived vectors transform a wide variety of higher plants, including monocotyledonous and dicotyledonous plants, such as soybean, cotton, rape, tobacco, and rice (Pacciotti *et al.,* 1985: Byrne *et al.,* 1987; Sukhapinda *et al.,* 1987; Lorz *et al.,* 1985; Potrykus, 1985; Park *et al.,* 1985: Hiei *et al.,* 1994). The use of T-DNA to transform plant cells has received extensive study and is amply described (EP 120516; Hoekema, 1985; Knauf, *et al.,* 1983; and An *et al.,* 1985). For introduction into plants, the chimeric constructs of the invention can be inserted into binary vectors as described in the examples.

Those skilled in the art will appreciate that the choice of method might depend on the type of plant, *i.e*., monocotyledonous or dicotyledonous, targeted for transformation. Suitable methods of transforming plant cells include, but are not limited to, microinjection (Crossway *et al.,* 1986), electroporation (Riggs *et al.,* 1986), *Agrobacterium*-mediated transformation (Hinchee *et al.,* 1988), direct gene transfer (Paszkowski *et al.,* 1984), and ballistic particle acceleration using devices available from Agracetus, Inc., Madison, Wis. and BioRad, Hercules, Calif. (see, for example, Sanford *et al.,* U.S. Pat. No. 4,945,050; and McCabe *et al.,* 1988). Also see, Weissinger *et al.,* 1988; Sanford *et al.,* 1987 (onion); Christou *et al.,* 1988 (soybean); McCabe *et al.,* 1988 (soybean); Datta *et al.,* 1990 (rice); Klein *et al.,* 1988 (maize); Klein *et al.,* 1988 (maize); Klein *et al.,* 1988 (maize); Fromm *et al.,* 1990 (maize); and Gordon-Kamm *et al.,* 1990 (maize); Svab *et al.,* 1990 (tobacco chloroplast); Koziel *et al*., 1993 (maize); Shimamoto *et al*., 1989 (rice); Christou *et al.,* 1991 (rice); European Patent Application EP 0 332 581 (orchardgrass and other Pooideae); Vasil *et al.,* 1993 (wheat); Weeks *et al.,* 1993 (wheat). In one embodiment, the protoplast transformation method for maize is employed (European Patent Application EP 0 292 435, U. S. Pat. No. 5,350,689).

The main focus of the present invention is on transformation of sugar beet. The experimental procedures for the transformation of sugar beet are well known to those skilled in the art such as that disclosed by Chang *et al,* 2002 using sugar beet meristems as explant material or as described by Joersbo *et al,* 1998.

In a preferred embodiment (as shown in Example 3) the RNAi cassette can be transformed into a biennial sugar beet genotype such as, for example, G018. Transgenic shoots are checked for expression of the selection marker such as, for example, PMI activity (Joersbo *et al,* 1998). Positive shoots and non-transgenic controls are rooted and transferred to the greenhouse for an acclimatization period of two weeks minimum at 18 °C prior to the vernalization treatment. Once well-established, the transgenic plants are exposed to the vernalization treatment consisting of a period of 14 weeks at a constant temperature of 6 °C and 12 hours low artificial light. Prior to applying bolting-inductive conditions, vernalized plants are slowly acclimatized for two weeks in climate chambers by stepwise increasing the temperature from 10 to 18 °C. Plants are subsequently repotted into to larger pots (2 liter), and monitored for bolting while exposed to a constant temperature of 18 °C and a long-day photoperiod of 17 hours light / 7 hours dark.

After transformed plant cells or plants are selected and grown to maturity, those plants showing the trait of interest are identified. The trait can be any of those traits described above. Additionally, to confirm that the trait of interest is due to the expression of the introduced nucleic acid sequence of interest under control of the regulatory nucleotide according to the invention, expression levels or activity of the polypeptide or nucleic acid sequence of interest can be determined by analyzing mRNA expression using Northern blots, RT-PCR or microarrays, or protein expression using immunoblots or Western blots or gel shift assays.

The disclosure relates to plant cells and tissues, to plants derived from such cells and tissues, respectively, to plant material, to the progeny and to seeds derived from such plants, and to agricultural products including processed plant products obtainable by, for example, any one of the transformation methods described below.

Once an expression cassette as described herein before comprising a nucleic acid sequence according to the invention has been transformed into a particular plant species, it may be propagated in that species or moved into other varieties of the same species, particularly including commercial varieties, using traditional breeding techniques. Plants include gymnosperms, monocots, and dicots, especially agronomically important crop plants, such as rice, wheat, barley, rye, rape, corn, potato, carrot, sweet potato, sugar beet, bean, pea, chicory, lettuce, cabbage, cauliflower, broccoli, turnip, radish, spinach, asparagus, onion, garlic, eggplant, pepper, celery, carrot, squash, pumpkin, zucchini, cucumber, apple, pear, quince, melon, plum, cherry, peach, nectarine, apricot, strawberry, grape, raspberry, blackberry, pineapple, avocado, papaya, mango, banana, soybean, tobacco, tomato, sorghum and sugarcane.

The genetic properties engineered into the transgenic plants described above are passed on by sexual reproduction or vegetative growth and can thus be maintained and propagated in progeny plants. Generally said maintenance and propagation make use of known agricultural methods developed to fit specific purposes such as tilling, sowing or harvesting. Specialized processes such as hydroponics or greenhouse technologies can also be applied. Use of the advantageous genetic properties of the transgenic plants according to the invention can further be made in plant breeding that aims at the development of plants with improved properties such as tolerance of pests, herbicides, or stress, improved nutritional value, increased yield, or improved structure causing less loss from lodging or shattering. The various breeding steps are characterized by well-defined human intervention such as selecting the lines to be crossed, directing pollination of the parental lines, or selecting appropriate progeny plants. Depending on the desired properties different breeding measures are taken. The relevant techniques are well known in the art and include but are not limited to hybridization, inbreeding, backcross breeding, multiline breeding, variety blend, interspecific hybridization, aneuploid techniques, etc. Hybridization techniques also include the sterilization of plants to yield male or female sterile plants by mechanical, chemical or biochemical means. Cross pollination of a male sterile plant with pollen of a different line assures that the genome of the male sterile but female fertile plant will uniformly obtain properties of both parental lines. Thus, the transgenic plants according to the invention can be used for the breeding of improved plant lines that for example increase the effectiveness of conventional methods such as herbicide or pesticide treatment or allow to dispense with said methods due to their modified genetic properties. Alternatively new crops with improved stress tolerance can be obtained that, due to their optimized genetic "equipment", yield harvested product of better quality than products that were not able to tolerate comparable adverse developmental conditions.

One skilled in the art will recognize that the transgenic genotype of the present invention can be introgressed by breeding into other plant lines (preferably sugar beet plant lines) comprising different transgenic or non-transgenic genotypes. This different transgenic or non-transgenic genotype could be any genotype, but a genotype comprising at least one trait of interest is preferred. For example, a sugar beet inbred comprising the transgenic genotype of the present invention can be crossed with a sugar beet inbred line comprising the transgenic genotype of an event resistant to a different virus known to infect sugar beet plants. The resulting seed and progeny plants will have the trait of delayed bolting and the resistance traits in stacked form. For example, a sugar beet inbred with the transgenic genotype of the present invention can be crossed with a sugar beet inbred comprising the transgenic genotype of the glyphosate resistant H7-1 event (European patent application EP-A1-1597373). The resulting seed and progeny plants have both the resistance trait and the trait of delayed bolting. Further traits, like herbicide resistance, disease resistance or resistance against viruses (i.e., viruses like, for example, BNYW in either transgenic from or from conventional sources (like Holly or C48) or viruses other than BNYW) can also used for stacking with transgenic genotype of the present invention. It will be further recognized that other combinations or stacks can be made with the transgenic genotype of the invention and thus these examples should not be viewed as limiting.

One skilled in the art will also recognize that transgenic sugar beet seed comprising the transgenic genotype of the present invention can be treated with various seed-treatment chemicals, including various pesticides and insecticides, to further augment the resistance of the present invention.

The transgenic genotype of the present invention can be introgressed in any sugar beet inbred or hybrid using art recognized breeding techniques. The goal of plant breeding is to combine in a single variety or hybrid various desirable traits. For field crops, these traits may include resistance to insects and diseases (e.g., derived from conventional sources including, but not limited to Holly and C48), tolerance to herbicides, tolerance to heat and drought, greater yield, and better agronomic quality. With mechanical harvesting of many crops, uniformity of plant characteristics such as germination and taproot establishment, growth rate, maturity, and root size, is important.

In another aspect, there is herein described a method for producing hybrid seeds from which sugar beet plants with a phenotype of delayed bolting. Such methods comprise: (a) providing a sugar beet line with a phenotype of delayed bolting, particularly a transgenic sugar beet plant as herein described as a first parent line, (b) providing a second sugar beet line having a different genotype as a second parent line; (c) allowing the plants of the first parent line of step (a) and the plants of the second parent line of step (b) to pollinate each other, let the seed develop, and harvest the hybrid seed, wherein the harvested hybrid seeds are seeds of a sugar beet hybrid plant having a phenotype of delayed bolting. In an embodiment of this aspect, the first parental line provided in step (a) is an inbred sugar beet line comprising one or more or all polynucleotides of the present invention. In a further embodiment of this aspect, the second parental line is selected from the group consisting of (a) an inbred sugar beet plant line resistant to at least one virus affecting sugar beet, such as, for example, *Beet necrotic yellow vein virus*; (b) an inbred sugar beet plant line resistant to at least one herbicide; and (c) an inbred sugar beet plant line having resistant to at least one disease. Examples of common viruses and diseases affecting sugar beet and sources for resistance against these viruses or diseases are known to the person skilled in the art. Further, herbicides used on sugar beet and sources of resistance against these herbicides are also known to the person skilled in the art.

Plants that have been self-pollinated and selected for type for many generations become homozygous at almost all gene loci and produce a uniform population of true breeding progeny. A cross between two different homozygous lines produces a uniform population of hybrid plants that may be heterozygous for many gene loci. A cross of two plants each heterozygous at a number of gene loci will produce a population of hybrid plants that differ genetically and will not be uniform.

Plant breeding techniques known in the art and used in a sugar beet plant breeding program include, but are not limited to, recurrent selection, backcrossing, pedigree breeding, restriction length polymorphism enhanced selection, genetic marker enhanced selection and transformation. The development of sugar beet hybrids in a sugar beet plant breeding program requires, in general, the development of homozygous inbred lines, the crossing of these lines, and the evaluation of the crosses. Pedigree breeding and recurrent selection breeding methods are used to develop inbred lines from breeding populations. Sugar beet plant breeding programs combine the genetic backgrounds from two or more inbred lines or various other germplasm sources into breeding pools from which new inbred lines are developed by selfing and selection of desired phenotypes. The new inbreds are crossed with other inbred lines and the hybrids from these crosses are evaluated to determine which of those have commercial potential. Plant breeding and hybrid development, as practiced in a sugar beet plant-breeding program, are expensive and time-consuming processes.

Pedigree breeding starts with the crossing of two genotypes, each of which may have one or more desirable characteristics that is lacking in the other or which complements the other. If the two original parents do not provide all the desired characteristics, other sources can be included into the breeding population. In the pedigree method, superior plants are selfed and selected in successive generations. In the succeeding generations the heterozygous condition gives way to homogeneous lines as a result of self-pollination and selection. Typically in the pedigree method of breeding five or more generations of selfing and selection is practiced: F1 → F2; F2 → F3; F3 → F4; F4 → F5; etc.

Recurrent selection breeding, backcrossing for example, can be used to improve an inbred line and a hybrid that is made using those inbreds. Backcrossing can be used to transfer a specific desirable trait from one inbred or source to an inbred that lacks that trait. This can be accomplished, for example, by first crossing a superior inbred (recurrent parent) to a donor inbred (non-recurrent parent), that carries the appropriate gene(s) for the trait in question. The progeny of this cross is then mated back to the superior recurrent parent followed by selection in the resultant progeny for the desired trait to be transferred from the non-recurrent parent. After five or more backcross generations with selection for the desired trait, the progeny will be homozygous for loci controlling the characteristic being transferred, but will be like the superior parent for essentially all other genes. The last backcross generation is then selfed to give pure breeding progeny for the gene(s) being transferred. A hybrid developed from inbreds containing the transferred gene(s) is essentially the same as a hybrid developed from the same inbreds without the transferred gene(s).

Elite inbred lines, that is, pure breeding, homozygous inbred lines, can also be used as starting materials for breeding or source populations from which to develop other inbred lines. These inbred lines derived from elite inbred lines can be developed using the pedigree breeding and recurrent selection breeding methods described earlier. As an example, when backcross breeding is used to create these derived lines in a sugar beet plant-breeding program, elite inbreds can be used as a parental line or starting material or source population and can serve as either the donor or recurrent parent.

A single cross hybrid results from the cross of two inbred lines, each of which has a genotype that complements the genotype of the other. The hybrid progeny of the first generation is designated F1. In the development of commercial hybrids in a sugar beet plant-breeding program, only the F1 hybrid plants are sought. Preferred F1 hybrids are more vigorous than their inbred parents. This hybrid vigor, or heterosis, can be manifested in many polygenic traits, including increased vegetative growth and increased yield.

The development of a sugar beet hybrid in a sugar beet plant breeding program involves three steps: (1) the selection of plants from various germplasm pools for initial breeding crosses; (2) the selfing of the selected plants from the breeding crosses for several generations to produce a series of inbred lines, which, although different from each other, breed true and are highly uniform; and (3) crossing the selected inbred lines with different inbred lines to produce the hybrid progeny (F1). During the inbreeding process in sugar beet, the vigor of the lines decreases. Vigor is restored when two different inbred lines are crossed to produce the hybrid progeny (F1). An important consequence of the homozygosity and homogeneity of the inbred lines is that the hybrid between a defined pair of inbreds will always be the same. Once the inbreds that give a superior hybrid have been identified, the hybrid seed can be reproduced indefinitely as long as the homogeneity of the inbred parents is maintained.

A single cross hybrid is produced when two inbred lines are crossed to produce the F1 progeny. A double cross hybrid is produced from four inbred lines crossed in pairs (A X B and C X D) and then the two F1 hybrids are crossed again (A X B) X (C X D). A three-way cross hybrid is produced from three inbred lines where two of the inbred lines are crossed (A X B) and then the resulting F1 hybrid is crossed with the third inbred (A X B) X C. Much of the hybrid vigor exhibited by F1 hybrids is lost in the next generation (F2). Consequently, seed from hybrids is not used for planting stock.

In hybrid seed production it is preferred to eliminate or inactivate pollen production by the female parent. Incomplete removal or inactivation of the pollen provides the potential for self-pollination. This inadvertently self-pollinated seed may be unintentionally harvested and packaged with hybrid seed. Once the seed is planted, it is possible to identify and select these self-pollinated plants. These self-pollinated plants will be genetically equivalent to the female inbred line used to produce the hybrid. Typically these self-pollinated plants can be identified and selected due to their decreased vigor. Female selfs are identified by their less vigorous appearance for vegetative and/or reproductive characteristics. Identification of these self-pollinated lines can also be accomplished through molecular marker analyses. However, simple and efficient pollination control systems exist which ensure utilizing heterosis by excluding self-pollination in commercial hybrid seed production. If one of the parents is a self-incompatible (SI), cytoplasmic male sterile (CMS) or nuclear male sterile (NMS) plant that is not able to self-pollinate or is incapable of producing pollen, only cross pollination will occur. By eliminating the pollen of one parental variety in a cross, a plant breeder is assured of obtaining hybrid seed of uniform quality, provided that the parents are of uniform quality and the breeder conducts a single cross. Cytoplasmic male sterility (CMS) is a maternally inherited phenomenon, the genetic determinants of which are located in the genome of the cytoplasmic organelles, the mitochondria. Such plants are severely impaired in their ability to produce functional pollen grains. Restorer genes for CMS systems are dominant nuclear genes, which suppress male sterile effects of the cytoplasm. The expression of male sterility in CMS plants is the result of incompatibility between recessive nuclear gene and male sterile specific cytoplasmic genome. In the disclosure a CMS system is applied for production of the hybrid sugar beet plants of the present invention. In such a system a male sterile CMS line is used as female parent which is pollinated by a male fertile line used as male parent. The trait of delayed bolting according to the present invention can be present in both the CMS male sterile (female) parent line or the male fertile (male) parent line or even both. Preferably, the trait of delayed bolting is kept on the male sterile side in order to avoid GM contaminations via the pollen containing the trait shed by the male parent.

As is readily apparent to one skilled in the art, the foregoing are only some of the various ways by which the inbred of the present invention can be obtained by those looking to introgress the transgenic genotype of the invention into other sugar beet lines. Other means are available and known to the person skilled in the art, and the above examples are illustrative only.

In general, the second parent line used for the hybrid production can also be a sugar beet plant line having the phenotype of delayed bolting like, for example, a sugar beet plant of the present invention. Preferably, the first parent line and the second parent line employed in the production of the hybrid seed are based on genetically diverse backgrounds. Genetic distance can be measured by the use of molecular markers as described for example in Knaak (1996). However, the second parent line could also be a sugar beet inbred comprising another trait of interest like, for example but not limited to glyphosate resistant (e.g. containing the H7-1 event as described in the European patent application EP-A1-1597373).

The resulting hybrid seed will contain the stacked traits of delayed bolting and herbicide glyphosate. Further traits, like herbicide resistance, disease resistance or resistance against BNYW from conventional sources (like Holly or C48) or viruses other than BNYW can also be comprised in the second parent line for stacking with transgenic genotype of the present invention in the hybrid seed. It will be further recognized that other combinations or stacks can be made with the transgenic genotype of the invention and thus these examples should not be viewed as limiting. Another disclosure relates to hybrid seed of a sugar beet plant having a phenotype of delayed bolting. In one aspect said hybrid seed is produced by the method for producing sugar beet hybrid seed of sugar beet plants having a phenotype of delayed bolting. Such methods are known to the person skilled in the art. In yet another aspect of the present invention a hybrid sugar beet plant having a phenotype of delayed bolting is provided that is produced by growing the hybrid seed of the present invention. Preferably, this hybrid plant is not bolting at all, i.e. show complete suppression of the vernalization response. A further preferred embodiment of the present invention relates to a part of said hybrid sugar beet plant of the present invention. Preferably said part is selected from the group comprising seeds, embryos, microspores, zygotes, protoplasts, cells, ovules, pollen, taproots, cotyledons, or other reproductive or vegetative parts or extracts or samples.

According to another aspect of the invention, methods of detecting the presence of a nucleic acid sequence or a chimeric construct of the present invention in a biological sample are provided. Such methods comprise: (a) contacting the sample comprising DNA with a pair of primers that, when used in a nucleic-acid amplification reaction with genomic DNA from a sugar beet carrying a nucleic acid sequence or a chimeric construct of the present invention, produces an amplicon that is diagnostic for a sugar beet of the present invention; (b) performing a nucleic acid amplification reaction, thereby producing the amplicon; and (c) detecting the amplicon. Detection of the amplicon can be conducted by any means well known in the art including but not limited to fluorescent, chemiluminescent, radiological, immunological, or otherwise. In the case in which hybridization is intended to be used as a means for amplification of a particular sequence to produce an amplicon, the production and detection by any means well known in the art of the amplicon is intended to be indicative of the intended hybridization to the target sequence where one probe or primer is utilized, or sequences where two or more probes or primers are utilized.

Further disclosed are methods for producing sugar, wherein a sugar beet plant of the present invention, or cells or tissues, a biological sample or an extract thereof is processed to produce sugar. Further, sugar is provided by the present invention that is produced by the method of producing sugar of the present invention. The method for producing sugar can be any conventional method for producing sugar known to person skilled in the art.

Another preferred aspect relates to a method for producing one or more biofuel(s) selected from the group comprising ethanol, biogas and/or biodiesel, by processing a transgenic sugar beet plant of the present invention, or cells or tissues, or a biological sample or an extract thereof to produce the one or more biofuel(s). The biofuel can be any biofuel produced by aerobic or anaerobic fermentation of plant material. A non-limiting example of a biofuel obtained by aerobic fermentation is bioethanol. Biofuels that can be obtained by anaerobic fermentation include, but are not limited to biogas and/or biodiesel. Methods of aerobic and/or anaerobic fermentation are known to the person skilled in the art. Further encompassed by the present invention are biofuels selected from the group comprising ethanol, biogas and/or biodiesel as produced by the method for producing one or more biofuel(s) or the present invention.

In the disclosure polynucleotide markers are provided which maps at or in close vicinity to the B locus, particularly at a distance of 1 cM upstream of markers MP0176 and GJ01 and co-segregates with marker GJ131 (Möhring S. *et al,* 2004; Gaafar R. M. *et al,* 2005) (Figure 5).

The disclosure further relates to polynucleotide markers identified in the sugar beet genome including variants and derivatives thereof, which polynucleotide markers are developed based on a nucleic acid sequence which is obtainable from a genomic DNA region that shows perfect co-segregation with the bolting gene (*B* gene) associated phenotype in sugar beet and wherein the marker allows to discriminate between annual and biennial genotype or between different haplotypes within a plant grouping of sugar beet plants exhibiting a biennial or annual phenotype. In a preferred embodiment the polynucleotide markers of the present invention have a nucleic acid sequence obtainable from one or more of the nucleic acid sequences of the present invention and as described above. Preferably, the polynucleotide markers further comprise one or more polymorphisms, particularly a polymorphism that is based on an SNP, an SSR, a deletion or an insertion of at least one nucleotide, but especially a polymorphism based on an SNP, which polymorphism is diagnostic for the B allele at the B locus. Such polynucleotide markers are preferably capable of detecting at least one of the various SNPs present in the different alleles of the genomic sequence set forth as SEQ ID NO: 8 herein and shown in Tables 7-1 (further depicted in Figure 10) and 7-2 (further depicted in Figure 10), wherein said polynucleotide marker is capable of differentiating between different alleles, particularly between annual and biennial sugar beet lines. In a preferred disclosure the polynucleotide marker are capable of detecting at least one SNP selected from the group comprising SNPs at positions #224, #351, #615, #897, #1082, #1841, #1915, #2334, #11592, #12316, #12490, or #12544 of the sequence set forth as SEQ ID NO: 8 and as shown in Tables 7-1 (further depicted in Figure 10) and 7-2 (further depicted in Figure 11). A further aspect of the present invention relates to a set of polynucleotide markers comprising a plurality of the polynucleotide markers of the present invention and described above. In this context, the term "plurality" refers to a set of more than one polynucleotide marker, which preferably consists of two, three or more markers.

Based on the information provided in this application it will be possible, for a skilled person, to identify or develop markers not explicitly disclosed herein but genetically closely linked to, or, preferably, located within the bolting gene or *B* gene or linked to the markers disclosed herein. The skilled person knows that other markers may provide at least equal utility in screening assays and marker assisted selection.

Molecular markers, preferentially End point TaqMan®, can, for example, be developed based on SNPs characterized from sequenced PCR products that are amplified from annual and biennial plants. Here, several PCR amplifications will be performed in order to cover the whole sequence of the gene. New molecular markers will then be tested within different annual and biennial genetic backgrounds to evaluate the robustness of the molecular test.

Disclosed a molecular marker is a DNA fragment amplified by PCR, *e.g.,* a SSR marker or a RAPD marker. The presence or absence of an amplified DNA fragment is indicative of the presence or absence of the trait itself or of a particular allele of the trait. A difference in the length of an amplified DNA fragment is indicative of the presence of a particular allele of a trait, and thus enables to distinguish between different alleles of a trait.

In a specific embodiment of the invention simple sequence repeat (SSR) markers are used to identify invention-relevant alleles in the parent plants and/or the ancestors thereof, as well as in the progeny plants resulting from a cross of said parent plants. There are several further methods or approaches available, known to those skilled in the art, which can be used to identify and/or develop markers in linkage disequilibrium and/or linked to and/or located in the *B* gene region, as well as markers that represent the actual causal mutations responsible for the biennial genotype. Without being fully exhaustive some approaches, known by those skilled in the art, include:
- *use of disclosed sequences*/*markers in hybridization approaches to identify other sequence in the region of interest:* primer sequences as disclosed herein and/or marker/gene sequences (or part thereof) that can be determined using the primer sequences as disclosed herein may be used as (hybridization) probes in isolating nucleic acid sequences/genes flanking the markers and/or linked and/or associated and/or specific for the B gene region from a genomic nucleic acid sample and/or RNA or cDNA sample or pool of samples (for example screening of genomic resources like BAC libraries or gDNA or cDNA library screening);
- *use of disclosed sequences*/*markers in PCR approaches to identify other sequence in the region of interest:* primer sequences as disclosed herein and/or marker/(candidate)gene sequences (or part thereof) that can be determined using the primer sequences as disclosed may be used as (PCR) amplification primers to amplify a nucleic acid sequence/gene flanking and/or linked to and/or associated with and/or specific for the QTL region from a genomic nucleic acid sample and/or RNA or cDNA sample or pool of samples either or not isolated from a specific plant tissue and/or after specific treatment of the plant and from sugar beet or in principal any other organism with sufficient homology;
- *use of disclosed sequences*/*markers in PCR approaches to identify other sequence in the region of interest:* the nucleotide sequences/genes of one or more markers can be determined after internal primers for said marker sequences may be designed and used to further determine additional flanking sequence/genes within the *B* gene region and/or genetically linked and/or associated with the trait;
- *use of disclosed sequences*/*markers in mapping and*/*or comparative mapping approaches to identify markers in the same region(s) (positioning of the B gene on other maps):* based on positional information and/or marker information as disclosed herein, markers, of any type, may be identified by genetic mapping approaches, eventually (if already needed) by positioning of the disclosed markers (by genetic mapping or extrapolation based on common markers across maps) on a (high density) genetic map(s), and/or integrated genetic or consensus map(s). Markers already known and/or new markers genetically linked and/or positioned in the vicinity of the disclosed markers and/or *B* gene region may be identified and/or obtained and eventually used in *B* gene (fine-) mapping and/or *B* gene cloning and/or MAS breeding applications;
- *use of disclosed sequences*/*markers in 'in-silico' approaches to identify additional sequences*/*markers*/*(candidate)genes in B gene region(s):* primer sequences as disclosed herein and/or marker/(candidate)gene sequences (or part thereof) that can be determined using the primer sequences as disclosed herein or based on linked markers may be used in 'in-silico' methods to search sequence or protein databases (e.g., BLAST) for (additional) flanking and/or homolog sequences/genes and/or allelic diversity (both genomic and/or cDNA sequences or even proteins and both originating from capsicum and/or any other organism) genetically linked and/or associated with the traits as described herein and/or located in the B gene region;
- *use of disclosed sequences*/*markers in physical mapping approaches (positioning of B gene on physical map or genome sequence):* primer sequences as disclosed herein and/or marker/gene sequences (or part thereof) that can be determined using the primer sequences as disclosed herein or using other markers genetically linked to the markers disclosed herein and/or located in the B gene region may be positioned on a physical map and/or (whole) genome sequence in principal of any organism with sufficient homology to identify (candidate) sequences/markers/genes applicable in *B* gene(fine-mapping) and/or *B* gene cloning and/or MAS breeding applications;
- *use of disclosed sequences*/*markers to position B-gene on other (physical) maps or genomes (across species)* primer sequences as disclosed herein and/or marker/gene sequences (or part thereof) that can be determined using the primer sequences as disclosed herein may be used in comparative genome or syntheny mapping approaches to identify homolog region and homolog and/or ortholog sequences/(candidate) genes genetically linked and/or positioned in the *B* gene region and applicable in *B* gene(fine-mapping) and/or *B* gene cloning and/or MAS breeding applications;
- *use of disclosed sequences*/*markers to select the appropriate individuals allowing the identification of markers in region of interest by genetic approaches:* primer sequences and/or markers as disclosed herein may be used to select individuals with different/contrasting *B* gene alleles. Genetic association approaches and/or bulk segregant analysis (BSA, Michelmore *et al.,* 1991) can be used to identify markers/genes in the specific region (*B* gene region) of interest and/or associated or genetically linked to the described traits; or
- *use of disclosed information to search for (positional) candidate genes:* the disclosed information may be used to identify positional and/or functional candidate genes which may be associated with the described traits and/or genetically linked.

In another specific embodiment of the invention a marker based on a single nucleotide polymorphism is used to identify invention-relevant alleles in the parent plants and/or the ancestors thereof, as well as in the progeny plants resulting from a cross of said parent plants.

The majority of commercial seed productions for sugar beet are done in southern France and northern Italy. In both regions, the presence of annual weed beets can cause pollen contamination in the seed productions, resulting in annuals in the commercial seed. This is not acceptable to a customer, and therefore all commercial seed lots are grown in regions, such as Argentina where no wild beets are growing directly after harvesting the seed. The plants are not vernalized and the presence of bolters is used to identify seed lots contaminated with annuals.

The polynucleotide markers may therefore be used for quality control of commercial seed lots by screening of commercial biennial sugar beet seed for annual contaminants and for identifying annuals/biennials in breeding programs, which use the annual trait to speed up the breeding process, or when the annual trait is introduced together with new sources of genetic variation.

Different assays based on the gene sequence according to the invention and as described herein above can thus be developed and used to screen plant material for the presence or absence of the annuality allele.

In the past molecular marker techniques have been developed which can be used for genetic mapping, gene cloning, marker assisted plant breeding and for genome fingerprinting and investigating genetic relationships. Genetic markers are developed based on DNA polymorphisms in the nucleotide sequences of genomic regions and can either be detected by restriction enzymes, or by means of two priming sites.

There are several types of molecular markers that may be used in marker-based selection including restriction fragment length polymorphism (RFLP), random amplification of polymorphic DNA (RAPD), amplified restriction fragment length polymorphism (AFLP), single sequence repeats (SSR) and single nucleotide polymorphisms SNPs.

The information content of the different types of markers may be different depending on the method that was used to obtain the marker data and the population in which the markers were scored. For example, it is not always possible to distinguish genome fragments that are present in homozygous condition from heterozygous fragments. In a heterogeneous population like an F₂, co-dominant markers like restriction fragment length polymorphisms (RFLPs, Botstein *et al.,* 1980) and co-dominantly scored amplified fragment length polymorphisms (AFLPs, Vos *et al.,* 1995) yield more information than dominant markers like random amplified polymorphic DNAs (RAPDs, Welsh and McCleland, 1990) and dominantly scored AFLPs. RFLPs are co-dominant and are able to identify a unique locus. RFLP involves the use of restriction enzymes to cut chromosomal DNA at specific short restriction sites, polymorphisms result from duplications or deletions between the sites or mutations at the restriction sites.

AFLP requires digestion of cellular DNA with a restriction enzyme before using PCR and selective nucleotides in the primers to amplify specific fragments. With this method up to 100 polymorphic loci can be measured and only relatively small DNA sample are required for each test.

The most preferred method of achieving such amplification of nucleotide fragments that span a polymorphic region of the plant genome employs the polymerase chain reaction ("PCR") (Mullis *et al.,* 1986) ;), using primer pairs involving a backward primer and a forward primer that are capable of hybridizing to the proximal sequences that define a polymorphism in its double-stranded form.

In contrast to RFLPs, PCR-based techniques require only a small percentage (approximately 10%) of the DNA amount as template to produce large quantities of the target sequence by PCR amplification.

One such PCR based technique is RAPD, which utilizes low stringency polymerase chain reaction (PCR) amplification with single primers of arbitrary sequence to generate strain-specific arrays of anonymous DNA fragments. The method requires only tiny DNA samples and analyses a large number of polymorphic loci. However, the unpredictable behaviour of short primers which is affected by numerous reaction conditions, inheritance in a dominant manner, and population specificity are the main disadvantages of RAPDs.

Microsatellites, or simple sequence repeats (SSRs), simple sequence length polymorphisms (SSLPs), short tandem repeats (STRs), simple sequence motifs (SSMs), and sequence target microsatellites (STMs) represent a class of repetitive sequences which are widely dispersed throughout the genome of eukaryotes. The variation in number and length of the repeats is a source of polymorphism even between closely related individuals. SSR analysis is based on these (short-repeat) sequences which are selectively amplified to detect variations in simple sequence repeats. Such microsatellite sequences can be easily amplified by PCR using a pair of flanking locus-specific oligonucleotides as primers and detect DNA length polymorphisms (Litt and Luty, 1989; Weber and May, 1989).

Mutations at a single nucleotide position resulting in substitutions, deletions or insertions give rise to single nucleotide polymorphisms or SNPs, which occur approximately every 1.3 kb in human (Cooper *et al.,* 1985; Kwok *et al.,* 1996). Most polymorphisms of this type have only two alleles and are also called biallelic loci. Positional cloning based on SNPs may accelerate the identification of disease traits and a range of biologically informative mutations (Wang *et al.,* 1998).

PCR extension assays that efficiently pick up point mutations may be used to detect SNPs. The procedure requires little DNA per sample. Three widely used types of SNP detection assays using PCR method are cleaved amplified polymorphic sequences (CAPS) (Konieczny and Ausubel, 1993; Thiel *et al.,* 2004), derived CAPS (dCAPS) (Michaels and Amasino, 1998; Neff *et al*., 1998), and single strand conformation polymorphism (SSCP) (Orita *et al.,* 1989).

CAPS polymorphisms are differences in restriction fragment lengths caused by SNPs or INDELs that create or abolish restriction endonuclease recognition sites in PCR amplicons produced by locus-specific oligonucleotide primers. CAPS assays are performed by digesting locus-specific PCR amplicons with one or more restriction enzymes and then separating the digested DNA on agarose or polyacrylamide gels

dCAPS is a modification of the CAPS technique that allows detection of most single-nucleotide changes by utilizing mismatched PCR primers. Using the method, a restriction enzyme recognition site that includes the SNP is introduced into the PCR product by a primer containing one or more mismatches to template DNA. The PCR product modified in this manner is then subjected to restriction enzyme digestion, and the presence or absence of the SNP is determined by the resulting restriction pattern.

The SSCP technique separates denatured double stranded DNA on a non-denaturing gel, and thus allows the secondary structure, as well as the molecular weight, of single stranded DNA to determine gel mobility.

The ARMS (amplification refractory mutation system)-PCR procedure (Ye *et al.,* 2001) involves the use of a single PCR for SNP genotyping (Fan *et al.,* 2003; Chiapparino *et al.,* 2004). A tetra-primer, employing two primer pairs, is used to amplify two different alleles of a SNP in a single PCR reaction.

Alternative methods may be employed to amplify such fragments, such as the "Ligase Chain Reaction" ("LCR") (Barany, F., 1991)), which uses two pairs of oligonucleotide probes to exponentially amplify a specific target. The sequences of each pair of oligonucleotides are selected to permit the pair to hybridize to abutting sequences of the same strand of the target. Such hybridization forms a substrate for a template-dependent ligase. As with PCR, the resulting products thus serve as a template in subsequent cycles and an exponential amplification of the desired sequence is obtained.

LCR can be performed with oligonucleotides having the proximal and distal sequences of the same strand of a polymorphic site. In one embodiment, either oligonucleotide will be designed to include the actual polymorphic site of the polymorphism. In such an embodiment, the reaction conditions are selected such that the oligonucleotides can be ligated together only if the target molecule either contains or lacks the specific nucleotide that is complementary to the polymorphic site present on the oligonucleotide. Alternatively, the oligonucleotides may be selected such that they do not include the polymorphic site (see, Segev, PCT Application WO 90/01069).

A further method that may alternatively be employed is the "Oligonucleotide Ligation Assay" ("OLA") (Landegren *et al.,* 1988). The OLA protocol uses two oligonucleotides that are designed to be capable of hybridizing to abutting sequences of a single strand of a target. OLA, like LCR, is particularly suited for the detection of point mutations. Unlike LCR, however, OLA results in "linear" rather than exponential amplification of the target sequence.

Nickerson *et al.,* 1990 have described a nucleic acid detection assay that combines attributes of PCR and OLA (Nickerson *et al*., 1990). In this method, PCR is used to achieve the exponential amplification of target DNA, which is then detected using OLA. In addition to requiring multiple, and separate, processing steps, one problem associated with such combinations is that they inherit all of the problems associated with PCR and OLA.

Schemes based on ligation of two (or more) oligonucleotides in the presence of a nucleic acid having the sequence of the resulting "di-oligonucleotide," thereby amplifying the di-oligonucleotide, are also known (Wu and Wallace, 1989), and may be readily adapted to the purposes of the present invention.

In still another embodiment of the invention a marker based on a deletion or an insertion ("INDEL") of at least one nucleotide is used to identify invention-relevant alleles in the parent plants and/or the ancestors thereof, as well as in the progeny plants resulting from a cross of said parent plants. These markers can be developed based on the sequence of the polynucleotides according to the invention and as described herein before.

In particular, the markers can be used in an allelic discrimination assay, particularly in an assay for discriminating between different haplotypes within plant groupings of sugar beet plants exhibiting a biennial genotype. Said assay is based on a set of probe polynucleotides comprising two separate probe molecules that are complementary, for example, to a subregion of the *BvPRR7* gene obtainable by PCR amplification based on forward primer *PRR7(T1)*-F and reverse primer *PRR7(T1)*-R as given in SEQ ID NO: 13 and SEQ ID NO: 14, respectively, which probe molecules differ only by one base mismatch and are probes PRR7(T1)-VIC (SEQ ID NO: 15) and PRR7(T1)-FAM (SEQ ID NO: 16). Further preferred sets are forward primer *PRR7(T6)-F* as depicted in SEQ ID NO: 49 and reverse primer *PRR7(T6)-R* as depicted in SEQ ID NO: 50 together with probes PRR7(T6)-VIC (SEQ ID NO: 51) and PRR7(T6)-FAM (SEQ ID NO: 52), as well as forward primer 1r22(T1)-F as depicted in SEQ ID NO: 55 and reverse primer 1r22(T1)-R as depicted in SEQ ID NO: 56 together with probes rr22(T1)-VIC (SEQ ID NO: 57) and 1r22(T1)-FAM (SEQ ID NO: 58).

Such assays, wherein a set of probe polynucleotides is employed, preferably comprise at least two separate probe molecules which differ by at least one mismatch, particularly by two or more mismatches located at adjacent sites, but especially by one single mismatch, wherein a first probe molecule, particularly a labelled probe molecule, more particularly a probe molecule labelled with a first fluorescent dye and a quencher, represents one allele and a second probe molecule, particularly a labelled probe molecule, more particularly a probe molecule labelled with a second fluorescent dye and a quencher, which is not identical with the first dye, represents the other allele, and wherein said set of probe polynucleotides is used for discriminating between the two allelic variants. Further two different fluorescent labels can be employed, the fluorescence of which can be easily distinguished. For example, a first probe is labelled with a first fluorescent dye (like, for example FAM) and a second probe is labelled with a second fluorescent dye (like, for example VIC). In a preferred embodiment of such an assay of the present invention the amplified fragment obtained in step b) of the allelic discrimination assay of the present invention described above is additionally probed with a second fluorescence-labelled probe molecule comprising a sequence specific for the biennial allele. In this assay an increase of the dye fluorescence of the first probe only is indicative of the presence of the annual allele. The two dyes used in this assay are preferably VIC and FAM. Generally, the assays of the present invention preferably employ two 2 primers (*i.e*., a pair of primers according to the invention) and at least one probe for the annual allele. A second probe can further be employed, which is a probe for the biennial allele.

In another aspect of the invention, an assay is provided involving markers that can discriminate specifically between annual plants and biennial plants and can thus be used, for example, for quality control of seed lots.

In particular, the invention relates to an assay, which is based on a set of probe polynucleotides comprising two separate probe molecules that are complementary, for example, to a to a subregion of the *BvPRR7* gene obtainable by PCR amplification based on forward primer *PRR7(T1)*-F and reverse primer *PRR7(T1)*-R as given in SEQ ID NO: 13 and SEQ ID NO: 14, respectively, which probe molecules differ only by one base mismatch and are probes *PRR7(T1)*-VIC (SEQ ID NO: 15) and PRR7(T1)-FAM (SEQ ID NO: 16). Further preferred sets are forward primer PRR7(T6)-F as depicted in SEQ ID NO: 49 and reverse primer PRR7(T6)-R as depicted in SEQ ID NO: 50 together with probes PRR7(T6)-VIC (SEQ ID NO: 51) and PRR7(T6)-FAM (SEQ ID NO: 52), as well as forward primer 1r22(T1)-F as depicted in SEQ ID NO: 55 and reverse primer 1r22(T1)-R as depicted in SEQ ID NO: 56 together with probes rr22(T1)-VIC (SEQ ID NO: 57) and 1r22(T1)-FAM (SEQ ID NO: 58).

Disclosed is a method for identifying annual contaminations in commercial seeds. Preferably this method comprises the use of the marker-based allelic discrimination assay of the present invention and described herein.

The following examples are intended solely to illustrate one or more preferred embodiments of the invention and are not to be construed as limiting the scope of the invention.

### EXAMPLES

The following Examples provide illustrative embodiments. In light of the present disclosure and the general level of skill in the art, the skilled person will appreciate that the following Examples are intended to be exemplary only and that numerous changes, modifications, and alterations can be employed without departing from the scope of the presently claimed subject matter.

### EXAMPLE 1: CHARACTERIZATION OF THE SUGAR BEET PRR7 GENE

### EXAMPLE 1.1: Mapping of the putative PRR7 homologue from sugar beet

Based on a candidate gene approach for the identification and characterization of putative bolting control genes in sugar beet, the EST sequence with accession number CV301305 was identified as the putative beet homologue of *PRR7* by means of homology searches using BLAST. SEQ ID NO: 1 shows the nucleotide sequence of EST CV301305. The corresponding amino acid sequence shows the partial presence of a Pseudo Response Regulator receiver (PRR, pfam00072) or Signal Receiver (REC, cd00156) domain (Figure 1), a hallmark of the PRR gene family that all play key roles in the circadian clock (Nakamichi *et al.,* 2005). Figure 2 shows the alignment of the amino acid sequence of CV301305 with *PRR7,* its closest *Arabidopsis* homologue, which has been described as a component of the temperature-sensitive circadian system (Nakamichi *et al.,* 2007; Salomé and McClung 2005). The circadian clock is known to control several developmental processes in plants including flowering time control (Imaizumi and Kay, 2006; Zhou et *al.,* 2007).

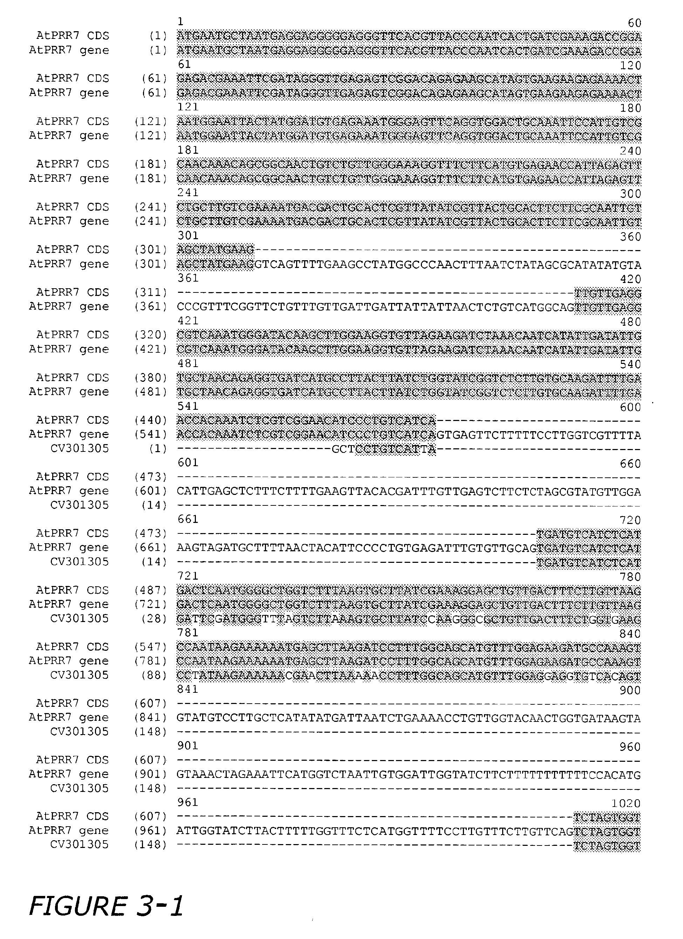

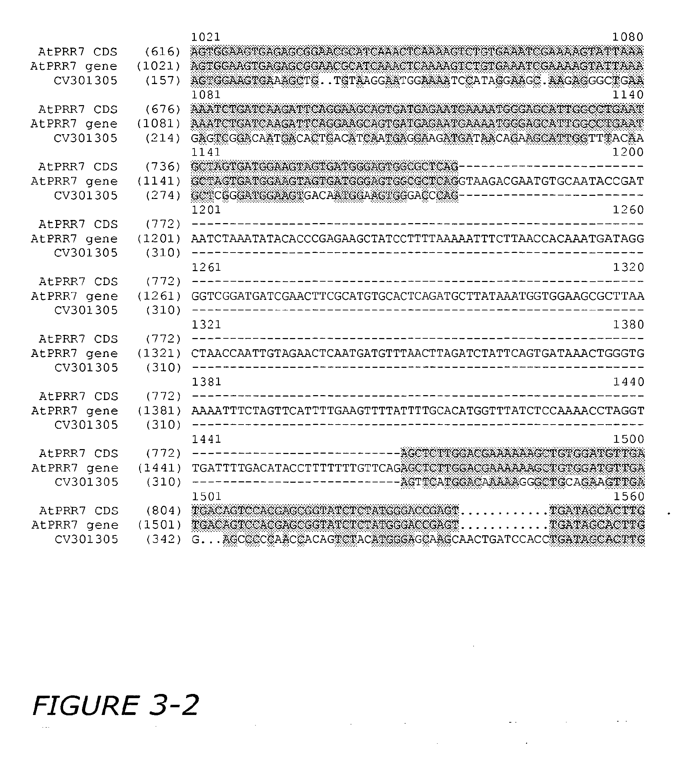

Based on the above observations, the putative gene structure of the partial beet *PRR7* fragment was deduced using the alignment between the genomic sequence and the mRNA of the *Arabidopsis PRR7* gene (AT5G02810 and NM_120359, respectively) to the *BvPRR7* sugar beet EST (CV301305), which revealed the presence of several putative intronic regions (Figure 3). Primers PRR7-F and PRR7-R (SEQ ID NOs: 2 and 3) encompassing the third putative intronic region delivered an amplification product of approximately 0.5 Kb when using genomic beet DNA as template. The PCR conditions for the amplification reaction were as follows: primary denaturation at 95°C for 5 min followed by 35 amplification cycles of 30 seconds at 95°C, 30 seconds at 60°C and 30 seconds at 72°C and followed by 5 min at 72°C. PCR experiments were run at a GeneAMP PCR System 9600 instrument from Applied Biosystems Inc. using Platinum *Taq* DNA polymerase and the corresponding reaction mix from Invitrogen Corporation as recommended by the supplier. Sequence analysis of the PCR product enabled the reconstruction of the genomic sequence around intron 3 of the *BvPRR7* gene fragment, and confirmed the presence of an intron of 296 base pairs in length (SEQ ID NO: 4).

The genomic fragment of the *BvPRR7* gene was amplified and sequenced across a panel of sugar beet parental lines consisting of 15 biennial and one annual line. All biennial lines revealed monomorphic for *BvPRR7* as only two different haplotypes were observed: one biennial allele and one annual allele (Table1). In order to map *BvPRR7* in a population segregating for the annual habit, an assay was developed targeting the SNP at position #160 (SEQ ID NO: 4) using the EndPoint TaqMan® technology. Table 2 summarizes the nucleotide sequences of the primers and probes designed for the PRR7(T1) TaqMan® assay targeting SNP at position #160; the reactions further consisted of the TaqMan® Universal PCR Master Mix, No AmpErase® UNG (2X) from Applied Biosystems Inc. according to the manufacturer's recommendations. The PCR amplification was performed as follows: 95°C for 10 min followed by 40 cycles of 95°C for 15 sec and 60°C for 1 min, using an ABI PRISM 7700 Sequence Detector instrument. EndPoint measurement was performed using the Sequence Detection System 2.0 software.

**Table 1: Polymorphisms observed between 1 annual and 15 biennial sugar beet lines for the BvPRR7 gene fragment spanning intron 3.**

| | | | | |
|---|---|---|---|---|
| SEQ ID NO 4: pos. | 87 | 160 | 406 | |
| haplotype#1 | T | T | G | annual |
| haplotype#2 | C | C | A | biennial |

| | | | | |
|---|---|---|---|---|
| The header row indicates the nucleotide position at the genomic sequence of the *BvPRR7* gene fragment (as depicted in SEQ ID NO: 4). The rows titled "haplotype#1" and "haplotype#2" represent the 2 haplotypes observed across the panel of 16 lines. | | | | |

**Table 2: Nucleotide sequences of primers and probes corresponding to the TaqMan assay PRR7(T1) for the genotyping of SNP #160**

| Precursor names | Nucleotide sequence (5' to 3' | SEQ ID NO: |
|---|---|---|
| PRR7(T1)-F | GAGGTGTCACAGTGTAAGTGTCT | 13 |
| PRR7(T1)-R | AAAGACTGCTACACGAACCACTAAG | 14 |
| PRR7(T1)-FAM | FAM-CTGATGAAAAGCTG-MGB-NFQ | 16 |
| PRR7(T1)-VIC | VIC-CTGATGGAAAGCTG-MGB-NFQ | 15 |

Using the above PRR7(T1) assay, the *BvPRR7* gene was mapped in a F2 population of 198 individuals derived from a cross between the annual line and a biennial line polymorphic for the SNP at position #160. *BvPRR7* maps at chromosome II at an approximate distance of 1 cM downstream of the GJ131 marker (Figure 4), a region known to contain the *B* gene for vernalization-independent flowering (Möhring *et al.,* 2004; Gaafar *et al.,* 2005). The results of the PRR7(T1) assay show a perfect match between the predicted genotype of the *B* gene and the genotype of the *BvPRR7* gene. The genotype of the *B* gene was predicted based on phenotypic evaluation of the F3 populations derived from the individual F2 plants for vernalization-independent flowering. Table 3 summarizes the graphical representation of the fine-map of the *B* gene region for 9 individual progeny plants comprising the closest recombination events. The combination of its map position and its biological function relating to the temperature-sensitive circadian rhythm (Salomé and McClung, 2005) obviously make *BvPRR7* a strong candidate for the *B* gene.

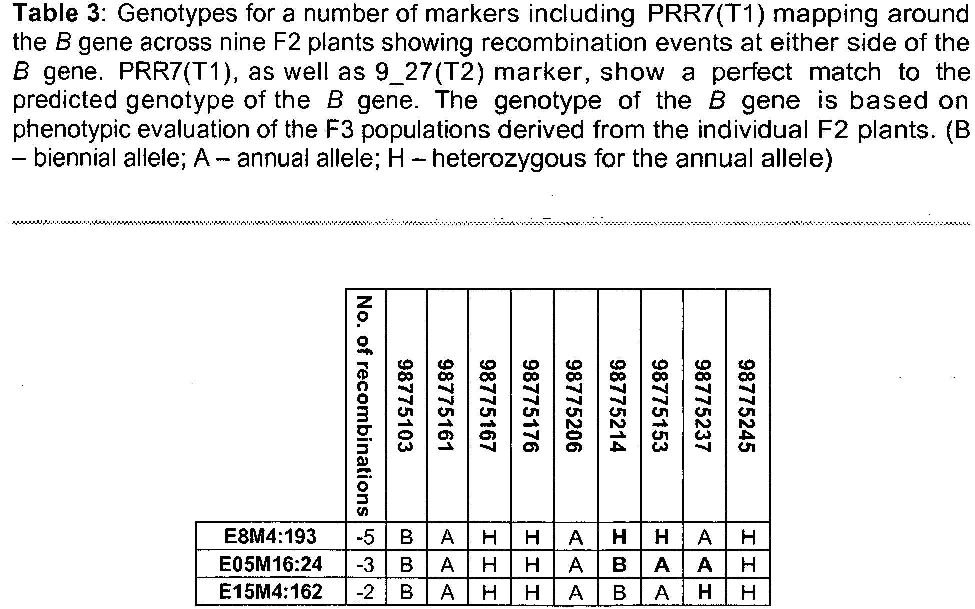

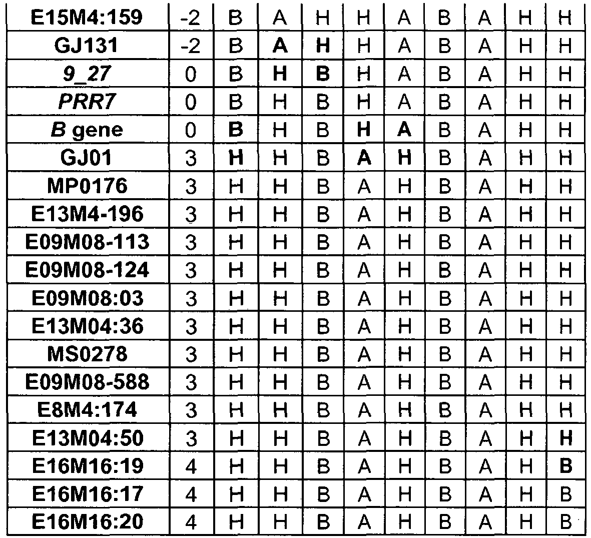

### EXAMPLE 1.2: Recovery of the full-length genomic sequence of BvPRR7

Using the primers PRR7-F and PRR7-R, a sugar beet BAC library was screened by means of PCR. The library was developed from the biennial commercial cultivar H20 and calculated to represent 6 genome equivalents with an average insert size of 120 Kb (McGrath *et al.,* 2004). DNA pools for this library are distributed by Amplicon Express, Pullman WA. The PCR conditions for the screening of the DNA pools were as follows: primary denaturation at 95°C for 5 min followed by 35 amplification cycles of 30 seconds at 95°C, 30 seconds at 60°C and 30 seconds at 72°C and followed by 5 min at 72°C. PCR experiments were run at a GeneAMP PCR System 9700 instrument from Applied Biosystems Inc. using Platinum *Taq* DNA polymerase and the corresponding reaction mix from Invitrogen Corporation as recommended by the supplier. Subsequent screenings of the DNA pools for the presence of the *BvPRR7* fragment according to the supplier's instructions resulted in the positive identification of BAC SBA079-L24.

In order to obtain the full-length sequence of the *BvPRR7* gene, BAC SBA079-L24 was sent to MWG Biotech AG, Germany for sequence analysis by means of the 454 sequencing technology. Where necessary, gaps between the obtained contigs were filled by regular Sanger sequencing to yield one single genomic sequence for the *BvPRR7* gene (SEQ ID NO: 8). Based on the alignment of the genomic sequence to EST CV301305 and on sequence homology to the *PRR7* gene from *Arabidopsis,* the putative gene structure of the beet *BvPRR7* gene comprising introns and exons was predicted as shown in Figure 5. The corresponding amino acid sequence of *BvPRR7* is shown under SEQ ID NO: 11. Alignment of the amino acid sequence of *BvPRR7* to all members of the PRR gene family from *Arabidopsis* including *TOC1* (*PRR1*), *PRR3, PRR5*, *PRR7* and *PRR9* illustrates the strong conservation of the Pseudo Response Regulator receiver domain (PRR) motif (pfam00072) near the NH2-terminus and the CCT motif (pfam06203) at the COOH-terminus (Figure 6). In addition to the PRR gene family from *Arabidopsis, BvPRR7* also shares strong homology to the *PRR7* homologue in cereals as illustrated by the phylogenetic tree shown in Figure 7. The tree shown in Figure7 was constructed by applying the Neighbor-Joining method (Saitou and Nei, 1987) on several members of PRR gene family from several plant species including *Beta vulgaris* BvPRR7, *Arabidopsis thaliana* (TOC1, NP_200946; PRR3, NP_568919; PRR5, NP_568446; PRR7, NP_568107; and PRR9, NP_566085), *Oryza sativa* (PRR37, Q0D3B6), *Hordeum vulgare* (PPD-H1, AAY17586) and *Triticum aestivum* (PPD-D1, ABL09477). The unrooted dendrograms were generated from the alignment of the amino acid sequences using ClustalW, and the phylogenetic tree was displayed by MEGA4 (Tamura *et al.,* 2007). Bootstraps values for 1000 re-samplings are shown on each branch. Surprisingly, the *PRR7* homologue in cereals, better known as *Ppd,* is known to represent a major determinant of the photoperiod response (Turner *et al.,* 2005; Beales *et al.,* 2007) rather than the vernalization response as suggested here for sugar beet.

### EXAMPLE 1.3: Fine-mapping of the B locus

Based on the results of the initial mapping described in Example 1.1 above, a large fine-resolution mapping was initiated to saturate the region around molecular markers GJ131 and GJ01 used for mapping and to confirm the correlation between the predicted genotype of the B gene and the genotype of the *BvPRR7* gene. A total of 5157 F2 individuals obtained from several populations segregating for the annual habit, were analyzed with the two flanking markers GJ01(T1) and GJ131(T1) (Gaafar *et al.,* 2005). In total 71 F2 plants were identified that have recombined between the two flanking markers. The mapping interval for the *B* gene was accordingly calculated at 0.69 cM. The recombinant plants were subsequently genotyped using the PRR7(T1) assay described above and the 9_27(T2) and ED031700(T1) assays available for the interval as disclosed in European patent application EP 1 995 320 A1. Table 4 summarizes the nucleotide sequences of the primers and probes designed for the GJ131 (T1), 9_27(T2), ED031700(T1), PRR7(T1) and GJ01 (T1) TaqMan® assays; the reactions further employed the TaqMan® Universal PCR Master Mix, No AmpErase® UNG (2X) from Applied Biosystems Inc. according to the manufacturer's recommendations. The PCR amplification was performed as follows: 95°C for 10 min followed by 40 cycles of 95°C for 15 sec and 60°C for 1 min, using an Applied Biosystems 7500 Real-Time PCR System instrument.

**Table 4: Nucleotide sequences of primers and probes used in the GJ131(T1), 9_27(T2), ED031700(T1), PRR7(T1) and GJ01(T1) TaqMan® assays, respectively.**

| **GJ131(T1) Assay** | | |
|---|---|---|
| | SEQ ID NO | sequence |
| Forward primer | 17 | GCCCGTACAAACAAAGACTTCTC |
| Reverse primer | 18 | ACGCAGAATGTTGATGATGATACA |
| TaqMan VIC probe | 19 | TCCATCTCTCCACAGCTT |
| TaqMan VAM probe | 20 | TCCATCTCCCCACAGCT |

| **9_27(T2) Assay** | | |
|---|---|---|
| Forward primer | 25 | TGCCAAAACACACATTGTACCTATACA |
| Reverse primer | 26 | TGCCTCTGGCTCCTTGAAG |
| TaqMan VIC probe | 27 | CATCTCTACAACACTACC |
| TaqMan VAM probe | 28 | ATCTCTACAAGACTACC |

| **ED031700(T1) Assay** | | |
|---|---|---|
| Forward primer | 21 | TAAAGGTGGTAATTTTAGAGAATTTTAGGA |
| Reverse primer | 22 | GCTCGTTTTGAAAAAATTTGGG |
| TaqMan VIC probe | 23 | TTTAATTCGCATCCTTCT |
| TaqMan VAM probe | 24 | TTAATTCGCAAACTTCT |

| **PRR7(T1) Assay** | | |
|---|---|---|
| Forward primer | 13 | GAGGTGTCACAGTGTAAGTGTCT |
| Reverse primer | 14 | AAAGACTGCTACACGAACCACTAAG |
| TaqMan VIC probe | 15 | CTGATGGAAAGCTG |
| TaqMan VAM probe | 16 | CTGATGAAAAGCTG |

| **GJ01(T1) Assay** | | |
|---|---|---|
| Forward primer | 29 | GAACCCAGGATTACTCGTGAGC |
| Reverse primer | 30 | AAAAGTAGAATAAAATGTAACCTCCTCCATCTC |
| TaqMan VIC probe | 31 | ACGCAAGATAACATCAC |
| TaqMan VAM probe | 32 | ACGCAAGATAACGTCAC |

The allelic status of the *B* gene was deduced from the phenotypic observations made on the individual F2 plants (*i.e.* bolting or non-bolting under long-day conditions of 18 hours day and 6 hours night), as well as the corresponding progeny populations obtained by selfing of the F2 plants. Table 5 provides a graphical representation of the fine-resolution map of the *B* gene region summarizing the genotypic and phenotypic data obtained for the various recombinant plants. The perfect correlation between the genotype of the *PRR7* gene and the phenotype across all recombinants allows to conclude that the beet *PRR7* homologue is indeed the *B* gene. When assuming a single recombination event at each side of the *B* gene, the mapping interval for the *B* gene is now reduced to 0,02 cM, with the *PRR7* gene co-localizing on top of the *B* gene for the annual habit.

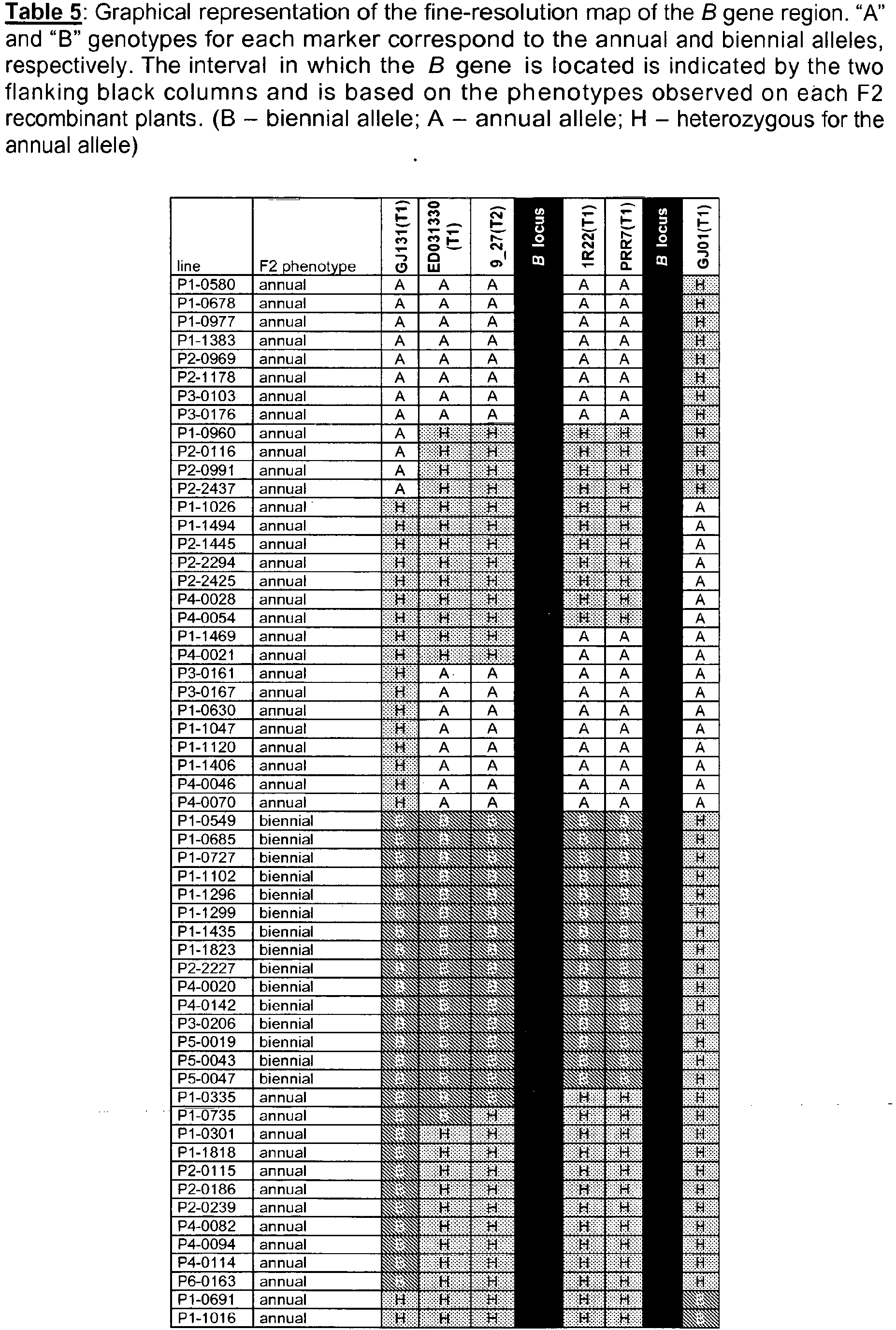

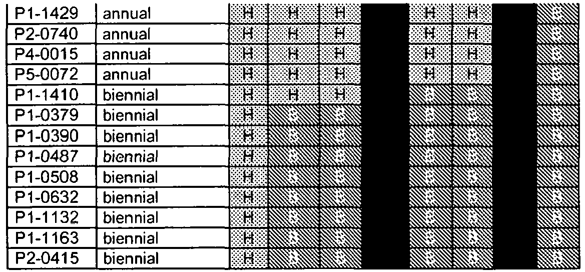

### EXAMPLE 1.4: Gene expression analysis of BvPRR7

For gene expression analysis, seedlings from annual, biennial and vernalized biennial plants were grown in controlled environment chambers at a constant temperature of 18 °C and a photoperiod of 16 hours light/8 hours dark (LDs) or 8 hours light/16 hours dark (SDs), respectively. Leaf samples were harvested every two hours over a period of 24 hours and total RNA was isolated using the RNAqueous®-4PCR Kit commercialized by Ambion, basically following the supplier's instructions. The RNA samples were converted to cDNA using the RETROscript® Kit (Ambion) starting from 1 µg of total RNA as template. The expression of the *BvPRR7* gene was measured by means of quantitative PCR (qPCR) using the *Power* SYBR® Green PCR Master Mix (Applied Biosystems Inc.) on a Real Time PCR 7500 System instrument. The PCR conditions were as follows: primary denaturation at 95°C for 10 min followed by 40 amplification cycles of 15 seconds at 95°C and 1 min at 60°C. The nucleotide sequences of the forward and reverse primer for *BvPRR7* are as follows: 5'-TTGGAGGAGGTGTCACAGTTCTAG-3' (SEQ ID NO: 45) and 5'-TGTCATTGTCCGACTCTTCAGC-3' (SEQ ID NO: 46), respectively. The isocitrate dehydrogenase (*BvICDH*) gene (AF173666) was used as reference gene for normalizing the expression of *BvPRR7.* The primer sequences designed for this reference gene consisted of 5'-CACACCAGATGAAGGCCGT-3' (SEQ ID NO: 47) and 5'-CCCTGAAGACCGTGCCAT-3' (SEQ ID NO: 48). Expression levels were calculated as the average of three biological replicates and each qPCR reaction was repeated three times.

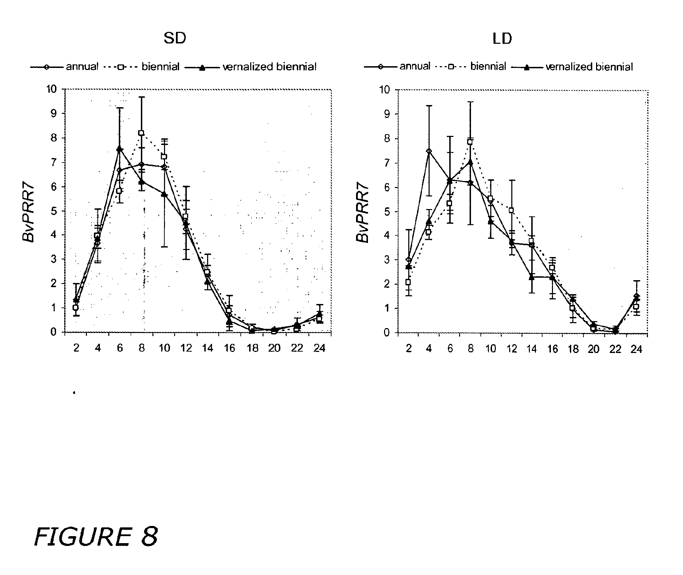

As illustrated in Figure 8, *BvPRR7* shows a diurnal oscillation of expression for all three classes of plants (*i.e.* annual, biennial and vernalized biennial plants) with a peak around 7 h after dawn both under LD (16 hours light/8 hours dark) and SD (8 hours light/16 hours dark) conditions. This experiment confirms the rhythmic and circadian expression of *BvPRR7* as described for most of the clock-associated genes identified thus far (McClung, 2006).

### EXAMPLE 1.5: Allelic variability and association to the vernalization requirement

Using the primer pairs provided in Table 6, the entire coding region of the *BvPRR7* gene as well as ± 1.0 Kb of its promoter region were amplified and sequenced across a panel of biennial and annual accessions. This panel comprised 3 biennial elite lines from the Syngenta germplasm pool as well as annual and biennial wild and weed beets collected across Europe. The PCR conditions for the amplifications were as follows: primary denaturation at 95°C for 5 min followed by 35 amplification cycles of 30 seconds at 95°C, 30 seconds at 60°C and 30 seconds at 72°C and followed by 5 min at 72°C. PCR experiments were run at a GeneAMP PCR System 9700 instrument from Applied Biosystems Inc. using Platinum *Taq* DNA polymerase and the corresponding reaction mix from Invitrogen Corporation as recommended by the supplier. The graphical representation of the observed genotypes shows several annual alleles and 2 biennial alleles (cf. Tables 7-1 and 7-2 also shown as (see also Figures 10 and 11, respectively). Several polymorphisms show a strong correlation between the allelic variation observed for *BvPRR7* and the annual or biennial plant habit. This observation further strengthens the causal relationship between *BvPRR7* and the B locus for vernalization independent flowering in sugar beet. Table 7-1 (Figure 10) shows the polymorphisms identified in the promoter region when comparing the annual and biennial alleles. Plant lines having heterozygous forms of the allele were removed from the analysis. SNP positions indicated in the table are numbered in accordance to SEQ ID NO: 8. Nucleotide positions indicated by an asterix (*) can be used for the discrimination of the annual and biennial alleles. As can be seen from Table 7-1 (Figure 10), the SNPs at positions #11592, #12316, #12490 and #12544, respectively, of the promoter region can be used to distinguish all annual alleles from the biennial alleles. The polymorphisms identified in the coding region when comparing the annual and biennial alleles are shown in Table 7-2 (Figure 11). Plant lines having heterozygous forms were again removed from the analysis. In Table 7-2 (Figure 11) the SNP and amino acid positions are numbered in accordance to SEQ ID NOs: 9 and 11, respectively. Nucleotide and amino acid positions indicated by an asterix (*) can be used for the discrimination of the annual and biennial alleles. Amongst the SNPs detected in the coding region, the SNPs at positions #224, #351, #615, #897, #1082, #1841, #1915, and #2334, respectively, can be used to distinguish all annual alleles from the biennial alleles. For the purpose of quality assurance, any one or a combination of more than one of the SNPs detected in the coding region as well as in the promoter region can be used for detecting the presence of the annual allele in commercial seed lots of biennial cultivars by means of molecular markers targeting this one or more SNP(s).

**Table 6: Nucleotide sequences of primers used for the amplification and sequencing of the coding region as well as of ±1 Kb of the 5' UTR region of the BvPRR7 gene.**

| **primer ID** | **SEQ ID NO** | **sequence 5'** | **orientation** | **use in combination with** | **location on *BvPRR7*** |
|---|---|---|---|---|---|
| SELA3977 | 33 | CGTGTCGAATATTGATTTACTGAGATC | Forward | SELA3988 | 5' UTR |
| SELA3988 | 34 | TAACCCATCATGTCTTTTCAACAATC | Reverse | SELA3977 | 5' UTR |
| SELA4442 | 35 | AAGAATACCGAGAGTTTTTTCCC | Forward | SELA3809 | 5' UTR |
| SELA3809 | 36 | TCACCAATTCTTTATATCATATCATGACA | Reverse | SELA4442 | 5' UTR |
| SELA3810 | 37 | GAGAAAAGGGTTTTAGATGGTAAGTTTT | Forward | SELA3807 | 5' UTR |
| SELA3807 | 38 | CATTTGTTGAAGTAGGTGATAAGGACAA | Reverse | SELA3810 | intron2 |
| SELA3766 | 39 | TTTGATGCTTTTTTCAGGCCA | Forward | SELA3769 | intron2 |
| SELA3769 | 40 | AATATGTGTGAGAAAATGGTGGCA | Reverse | SELA3766 | intron5 |
| SELA3857 | 41 | TCCATTTGAGGAGTAGGTATGATGAG | Forward | SELA3860 | intron5 |
| SELA3860 | 42 | TCTTGAGCTGCTGATCCACGT | Reverse | SELA3857 | exon8 |
| SELA3861 | 43 | CTGCATCTGGTAAGCCTGGTG | Forward | SELA3864 | exon8 |
| SELA3864 | 44 | AATGTGACCCGTAAACGCCT | Reverse | SELA3861 | 3'UTR |

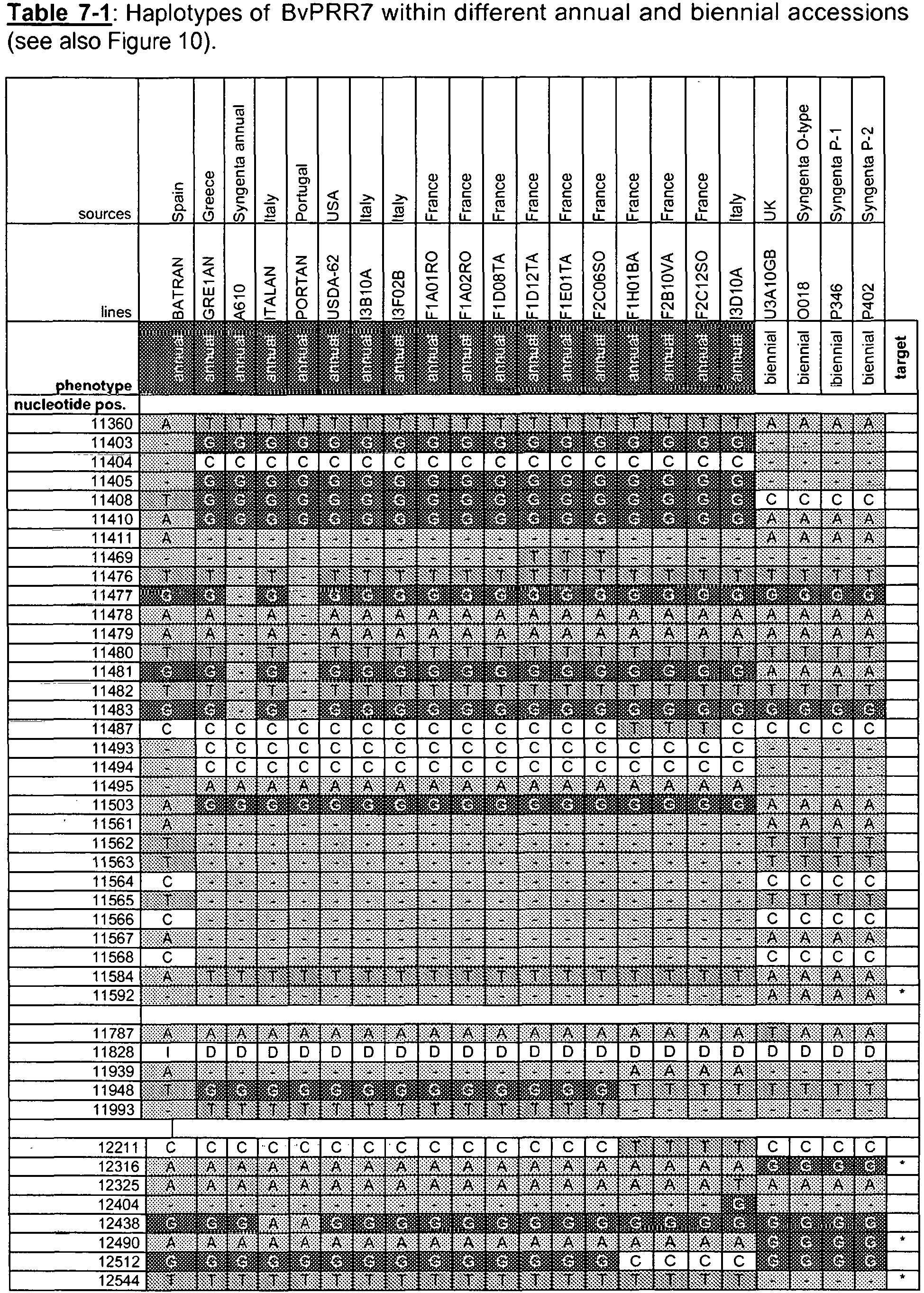

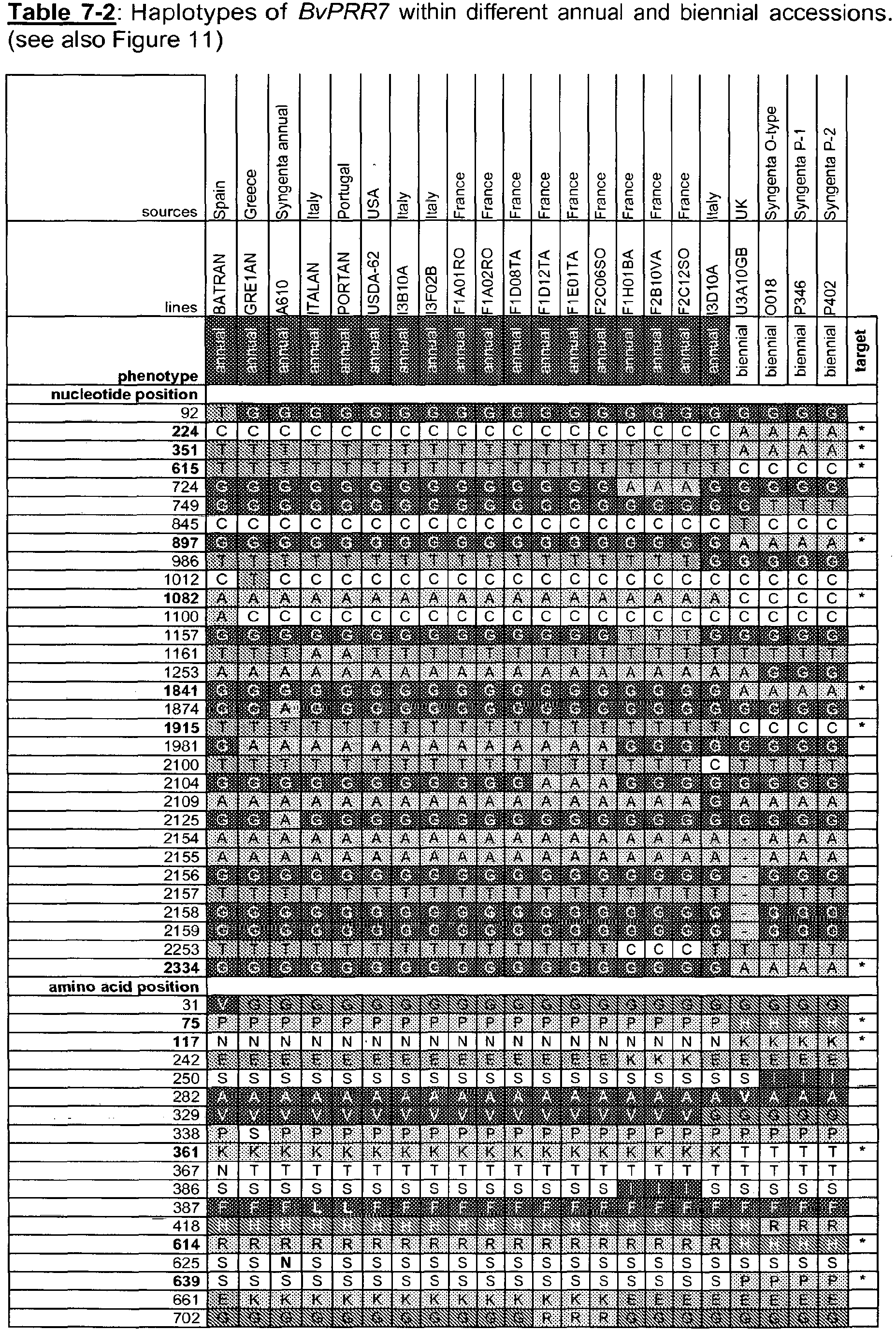

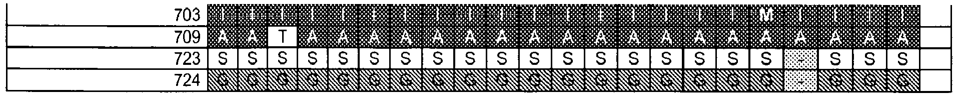

### EXAMPLE 1.6: Allelic discrimination between annual and biennial sugar beet plants

The presence of 'bolters', *i.e.* sugar beet plants carrying the annual *B* allele, in commercial seed lots due to the influx of annual pollen during the hybrid seed production, represents a major quality parameter in the production and marketing of sugar beet. For quality control in seed production it is thus important to have a means allowing to distinguish between annual and biennial plants.

For allelic discrimination DNA is isolated from plants or seeds to be tested by employing conventional DNA isolation methods. The DNA is then tested in a TaqMan® assay targeting SNP at position #2334 in the coding region. The nucleotide sequences used in this assay were as follow: PRR7(T6)-F: 5'-GCTATC-GGTATTCCTTCCTTTGTTT-3' (SEQ ID NO: 49), PRR7(T6)-R: 5'-CTCGTGTTCGT-GGGCAATT-3' (SEQ ID NO: 50), PRR7(T6)-VIC: 5'-VIC-CTCGTACCTGGCGCAC-MGB-NFQ-3' (SEQ ID NO: 51) and PRR7(T6)-FAM: 5'-FAM-CTCGCACCTGGCGC-AC-MGB-NFQ-3' (SEQ ID NO: 52). The PCR reaction further consisted of the TaqMan® Universal PCR Master Mix and No AmpErase® UNG (2X) from Applied Biosystems Inc. according to the manufacturer's recommendations. The PCR amplification was performed as follows: 95°C for 10 min followed by 40 cycles of 95°C for 15 sec and 60°C for 1 min, using a Real Time PCR 7500 System instrument. EndPoint measurement was performed using the Sequence Detection System 2.0 software. If the analysis shows a substantial increase in VIC dye fluorescence only, this indicates homozygosity for Allele X (i.e. homozygosity for the biennial allele). Substantial increase in FAM dye fluorescence only indicates homozygosity for Allele Y (i.e. homozygosity for the annual allele). If both fluorescent signals are substantially increased, the plant is heterozygous (i.e. an annual plant with heterozygosity for the *B* locus).

Figure 9 shows the result of an allelic discrimination assay of a set of annual and biennial individual plants.

Nucleotide sequences can be similarly used in this assay and provide similar results (i.e., which allow to discriminate between annual and biennial individual plants) are as follow: 1r22(T1)-F: 5'-GATAAATTCTGACCCGCATCACA-3' (SEQ ID NO: 55), 1r22(T1)-R : 5'-GGACTGAGTTGATAATAATCAACTTTCC-3' (SEQ ID NO: 56), 1r22(T1)-VIC : 5'-VIC-CTAGCGCAATTTC-MGB-NFQ-3' (SEQ ID NO: 57) and 1r22(T1)-FAM: 5'-FAM-AGCTAGCGCCCAATT-MGB-NFQ-3' (SEQ ID NO: 58).

### EXAMPLE 2: Transgenic validation of BvPRR7 by means of a complementation study

The annual plant habit conferred by the *B* gene behaves as a single dominant trait; the requirement for vernalization in biennial plants accordingly is recessive. The transformation of an annual allele of *BvPRR7* into a biennial genotype thus is predicted to bestow the annual flowering behavior onto the biennial acceptor genotype. There should thus be no need to vernalize the transgenic plants to induce bolting as the transformed annual allele of *BvPRR7* is supposed to overrule the need for vernalization conferring the annual habit. To verify this hypothesis, the coding sequence of an annual allele of *BvPRR7* under the control of an annual promoter together with a terminator fragment was transformed into biennial genotype G018. The plasmid map of the binary vector carrying the gene cassettes for both the PMI selectable marker gene and the annual *BvPRR7* allele is shown in Figure 10. The experimental procedure used for the transformation of sugar beet was essentially as disclosed by Chang *et al.,* 2002 using sugar beet meristems as explant material and the phosphomannose isomerase (PMI) gene as selectable marker. SEQ ID NO: 53 depicts the nucleotide sequence of the coding region of the annual *PRR7* allele (nucleotides 1306 to 3672 of SEQ ID NO: 49) downstream of 1,3 kb of its promoter region (nucleotides 1 to 1305 of SEQ ID NO: 49). Transgenic shoots were checked for PMI activity (Joersbo *et al.,* 1998) and subsequently rooted, potted in soil and transferred to the greenhouse. Negative controls consisted of shoots of both non-transgenic annual and biennial sugar beet plants that underwent the same *in vitro* regeneration procedure, but without *Agrobacterium* infection and mannose selection. Plants were grown in growth chambers at a constant temperature of 18 °C and a photoperiod of 17 hours light and 7 hours dark.

Under these conditions (without induction of bolting by applying cold temperatures) the non-transgenic biennial controls do not show any signs of bolting within an observation period of up to 12 weeks, whereas the annual control plants start to bolt normally within 6 to 8 weeks. Contrary to the non-transgenic biennial control plants, a substantial number of transgenic events starts bolting within four to ten weeks and basically behaves as annual plants despite their biennial genetic background. Transgenic plants that bolted and flowered are cross-pollinated with a biennial maintainer line to produce offspring. Progeny plants are tested for PMI activity and subsequently monitored for bolting and flowering without vernalization. These progeny plants show a one to one segregation ratio and a perfect correlation between PMI activity and the annual habit. These data confirm the causal relationship between *BvPRR7* and vernalization-independent flowering in sugar beet.

### EXAMPLE 3: Transgenic suppression of BvPRR7 confers bolting resistance

Since *BvPRR7* plays a key role in the vernalization response in sugar beet, *BvPRR7* represents an obvious candidate for engineering bolting resistance by suppressing the vernalization response. For this purpose a *BvPRR7* cDNA fragment of 0.6 Kb (SEQ ID NO: 1) was assembled into an RNAi cassette under the control of the constitutive Ubi3 promoter from *Arabidopsis* (Norris *et al.,* 1993). The inverted repeat of the *BvPRR7* fragment is separated by the second intron from the potato StLS1 gene (Eckes *et al.,* 1986; Vancanneyt *et al.,* 1990) to stabilize the RNAi cassette, but also to improve the efficiency of the RNAi phenomenon (Wang and Waterhouse, 2001; Smith *et al.,* 2000). The plasmid map of the binary vector carrying the RNAi gene cassette for *BvPRR7* and the PMI selectable marker gene is shown in Figure 11. The RNAi cassette was transformed into the biennial genotype G018 and selection for PMI-positive shoots was carried out as described in the previous example. PMI-positive shoots and non-transgenic controls were rooted and transferred to the greenhouse for an acclimatization period of two weeks minimum at 18 °C prior to the vernalization treatment. Once well-established, the transgenic plants were exposed to the vernalization treatment consisting of a period of 14 weeks at a constant temperature of 6 °C and 12 hours low artificial light. Prior to applying bolting-inductive conditions, vernalized plants were slowly acclimatized for two weeks in climate chambers by stepwise increasing the temperature from 10 to 18 °C. Plants were subsequently repotted into to larger pots (2 liter), and monitored for bolting while exposed to a constant temperature of 18 °C and a long-day photoperiod of 17 hours light / 7 hours dark. Non-transgenic control plants started bolting between four to six weeks post vernalization. Transgenic plants suppressed for *BvPRR7* frequently showed a delay in bolting ranging from only two weeks to more than two months. A few events did not show any bolting behavior at all under the conditions applied in the greenhouse. Apart from the delay in bolting and flowering, transgenic plants developed normally and showed no phenotypic aberrations. In general, plants delayed in bolting show a higher leaf number at the time of bolting as a result of the prolonged vegetative stage.

### REFERENCES:

Abe J., Guan G.-P. and Shimamoto Y., 1997. A gene complex for annual habit in sugar beet (Beta vulgaris L.). Euphytica 94: 129-135.
Barany F., 1991. Genetic Disease Detection and DNA Amplification Using Cloned Thermostable Ligase. Proc. Natl. Acad. Sci. USA, 88:189-93.
Batzer M.A., Carlton J.E. and Deininger P.L., 1991. Enhanced evolutionary PCR using oligonucleotides with inosine at the 3'-terminus. Nucleic Acids Res. 19:5081.
Beales J., Turner A., Griffiths S., Snape J.W. and Laurie D.A., 2007. A Pseudo-Response Regulator is misexpressed in the photoperiod insensitive Ppd-D1a mutant of wheat (Triticum aestivum L.). Theor. Appl. Genet. 115: 721-733.
Botstein D., White R.L., Skolnick M. and Davis R.W., 1980. Construction of genetic linkage map in man using restriction length polymorphisms. Am. J. Hum. Genet., 32: 314-331.
Brunke K.J. and Wilson S.L., 1993. Brassica hsp80 promoter. EP0559603
Chang Y.-F., Zhou H., Dunder E.M., Rouse S.N., Gu W. and Boutreau E., 2002. Methods for stable transformation of plants. WO 02/14523
Chiapparino E., Lee D. and Donini P., 2004. Genotyping single nucleotide polymorphisms in barley by tetra-primer ARMS-PCR. Genome 47:414-420.
Cooper D.N., Smith B.A., Cooke H.J. et al., 1985. An estimate of unique DNA sequence heterozygosity in the human genome. Hum. Genet. 69:201-205.
Eckes P., Rosahl S., Schell J. and Willmitzer L., 1986. Isolation and characterization of a light-inducible, organ-specific gene from potato and analysis of its expression after tagging and transfer into tobacco and potato shoots. Mol Gen Genet 205, 14-22.
English J.J., Mueller E. and Baulcombe D.C., 1996. Suppression of virus accumulation in transgenic plants exhibiting silencing of nuclear genes. Plant Cell 8:179-188.
Fan J.B., Oliphant A., Shen R., Kermani B.G., Garcia F., Gunderson K.L., Hansen M., et al., 2003. Highly parallel SNP genotyping. Cold Spring Harb Symp Quant Biol 68:69-78.
Felsenstein J., 1985. Confidence limits on phylogenies: An approach using the bootstrap. Evolution 39:783-791.
Gaafar R. M., Hohmann U. and Jung C., 2005. Bacterial artificial chromosome-derived molecular markers for early bolting in sugar beet. Theor. Appl. Genet. 110, Number 6: 1027-1037.
Imaizumi T. and Kay S.A., 2006. Photoperiodic control of flowering: not only by coincidence. Trends Plant Science 11: 550-557.
Joersbo M., Donaldson I., Kreiberg K., Petersen S.G., Brunstedt J. and Okkels F.T., 1998. Analysis of mannose selection used for transformation of sugar beet. Mol Breeding 4:111-117.
Konieczny A. and Ausubel F.M., 1993. A procedure for mapping Arabidopsis mutations using co-dominant ecotype-specific PCR-based markers, Plant J. 4, pp. 403-410.
Kwok P.Y., Deng Q., Zakeri H., Taylor S.L. and Nickerson D.A., 1996. Increasing the information content of STS-based genome maps: Identifying polymorphisms in mapped STSs. Genomics 31: 123-126.
Landegren U., Kaiser R., Sanders J. and Hood L., 1988. A ligase-mediated gene detection technique. Science 241 4869, pp. 1077-1080.
Litt M. and Luty J.A., 1989. Hypervariable microsatellite revealed by in-vitro amplification of a dinucleotide repeat in the cardiac muscle actin gene. Am J Hum Genet 44: 397-401.
McClung C.R., 2006. Plant circadian rhythms. Plant Cell 18: 792-803.
McGrath J.M., Shaw R.S., de los Reyes B.G. and Weiland J.J., 2004. Construction of a Sugar Beet BAC Library from a Hybrid with diverse Traits. Plant Mol Biol Rep 22: 23-28.
Michaels S.D. and Amasino R.M., 1998. A robust method for detecting single-nucleotide changes as polymorphic markers by PCR. The Plant Journal 14(3): 381-385.
Möhring S., Salamini F. and Schneider K., 2005. Multiplexed, linkage group-specific SNP marker sets for rapid genetic mapping and fingerprinting of sugar beet (Beta vulgaris L.). Molecular Breeding, Volume 14, Number 4: 475-488.
Mullis K.B., Faloona F.A., Scharf S., Saiki R., Horn G. and Erlich H., 1986. Specific enzymatic amplification of DNA in vitro: the polymerase chain reaction. Cold Spring Harbor Symp Quant Biol 51:263-273.
Nakamichi N., Kita M., Ito S., Sato E., Yamashino T. and Mizuno T., 2005. PSEUDO-RESPONSE REGULATORS, PRR9, PRR7 and PRR5, together play essential roles close to the circadian clock of Arabidopsis thaliana. Plant Cell Physiol., 46: 686-698.
Neff M.M., Neff J.D., Chory J. and Pepper A.E., 1998. dCAPS, a simple technique for the genetic analysis of single nucleotide polymorphisms: experimental applications in Arabidopsis thaliana genetics. Plant J 14: 387-392.
Nickerson D.A., Kaiser R., Lappin S., Stewart J., Hood L. and Landegren U., 1990. Automated DNA diagnostics using an ELISA-based oligonucleotide ligation assay. Proc. Natl. Acad. Sci. USA. 1990 Nov;87(22):8923-7.
Norris S. R., Meyer S. E. and Callis J., 1993. The intron of Arabidopsis thaliana polyubiquitin genes is conserved in location and is a quantitative determinant of chimeric gene expression. Plant Mol Biol 21: 895-906.
Ohtsuka E., Matsuki S., Ikehara M., Takahashi Y. and Matsubara K., 1985. An alternative approach to deoxyoligonucleotides as hybridization probes by insertion of deoxyinosine at ambiguous codon positions, J. Biol. Chem. 260(5):2605-2608.
Okamuro J.K. and Goldberg R.B., 1989. Regulation of Plant Gene Expression: General Principles. Biochemistry of Plants, vol. 15:1-82.
Orita M., Suzuki Y., Sekiya T. and Hayashi K., 1989. Rapid and sensitive detection of point mutations and DNA polymorphism using he polymerase chain reaction. Genomics 5:874-879.
Pearson W.R., 1990. Rapid and Sensitive Sequence Comparison with FASTP and FASTA. Methods in Enzymology 183, 63-98.
Rossolini G.M., Cresti S., Ingianni A., Cattani P., Riccio M.L. and Satta G., 1994. Use of deoxyinosine-containing primers vs degenerate primers for polymerase chain reaction based on ambiguous sequence information, Mol. Cell Probes 8 (1994), pp. 91-98.
Ruiz M.T., Voinnet O. and Baulcombe D.C., 1998. Initiation and maintenance of virus-induced gene silencing. Plant Cell 10, 937-946.
Salomé P.A. and McClung C.R., 2005. PSEUDO-RESPONSE REGULATOR 7 and 9 are partially redundant genes essential for the temperature responsiveness of the Arabidopsis circadian clock. Plant Cell 17: 791-803.
Saitou N. and Nei M., 1987. The neighbor-joining method: A new method for reconstructing phylogenetic trees. Molecular Biology and Evolution 4:406-425.
Segev D., 1990. PCT Pub. No. WO 90/01069.
Smith N.A., Singh S.P., Wang M.B., Stoutjesdijk P.A. Green A.G. and Waterhouse P.M., 2000. Total silencing by intron-spliced hairpin RNAs. Nature 407: 319-320.
Smith T.F. and Waterman M.S., 1981. Advances in Applied Mathematics. 2, 482-489.
Tamura K., Dudley J., Nei M. and Kumar S., 2007. MEGA4: Molecular Evolutionary Genetics Analysis (MEGA) software version 4.0. Molecular Biology and Evolution 24:1596-1599.
Thiel T., Kota R., Grosse I., Stein N. and Graner A., 2004. SNP2CAPS: a SNP and INDEL analysis tool for CAPS marker development. Nucleic Acids Res 32(1): e5.
Tijssen P., 1993. Laboratory Techniques in Biochemistry and Molecular Biology-Hybridization with Nucleic Acid Probes part I chapter 2 "Overview of principles of hybridization and the strategy of nucleic acid probe assays" Elsevier, New York.
Turner A., Beales J., Faure S., Dunford R.P. and Laurie D.A., 2005. The Pseudo-Response Regulator Ppd-H1 provides adaptation to photoperiod in barley. Science 310: 1031-1034.
Vancanneyt G., Schmidt R., O'Connor-Sanchez A., Willmitzer L. and Rocha-Sosa M., 1990. Construction of an intron-containing marker gene: Splicing of the intron in transgenic plants and its use in monitoring early events in Agrobacterium-mediated plant transformation. Mol. Gen. Genet. 220: 245-250.
Vandesompele J., De Preter K., Pattyn F., Poppe B, Van Roy N., De Paepe A. and Speleman F., 2002. Accurate normalization of real-time quantitative RT-PCR data by geometric averaging of multiple internal control genes. Genome Biology 3: 1-11.
Vos P., Hogers R., Bleeker M., et al. 1995. AFLP: a new technique for DNA fingerprinting. Nucleic Acids Research 23(21):4407-4414.
Wang D.G., Fan J.B., Siao C.J. et al., 1998. Large-scale identification, mapping, and genotyping of single-nucleotide polymorphisms in the human genome. Science 15, 1077-82.
Wang M.B. and Waterhouse P.M., 2001. Application of gene silencing in plants. Curr. Opin. Plant Biol 5:124-150.
Weber J.L. and May P.E., 1989. Abundant class of human DNA polymorhisms which can be typed using the polymerare chain reaction. Am. J. Hum. Genet. 44,388-396.
Welsh J. and McCleland M., 1990. Fingerprinting genomes using PCR with arbitrary primers. Nucleic Acids Research 18 (24):7213-7218.
Wu D.Y. and Wallace R.B., 1989. Genomics 4:560-569.
Ye S., Dhillon S., Ke X., Collins A.R. and Day I.N., 2001. An efficient procedure for genotyping single nucleotide polymorphisms. Nucleic Acids Res., 29, E88-E88.
Zhou Y., Sun X.-D. and Ni M., 2007. Timing of photoperiodic flowering: light perception and circadian clock. J. Integrative Plant Biol. 49: 28-34.
Zuckerkandl E. and Pauling L., 1965. Evolutionary divergence and convergence in proteins, pp. 97-166 in Evolving Genes and Proteins, edited by V. Bryson and H.J. Vogel. Academic Press, New York.

## Claims

1. A nucleic acid sequence, which encodes the *BvPRR7* gene of sugar beet, comprising a nucleic acid sequence selected from the group of nucleic acid sequences consisting of SEQ ID NOs: 4, 5, 6, 7, 8, 9, 10, 53 or 54.

2. Nucleic acid sequence comprising SEQ ID NO: 8, wherein said sequence comprises one or more nucleic acid substitutions, deletions, or additions as shown in Tables 7-1 and 7-2, wherein the polymorphisms shown in Tables 7-1 and 7-2 are representing 18 annual and 2 biennial alleles of SEQ ID NO: 8, respectively, wherein Tables 7-1 and 7-2 are depicted as follows:

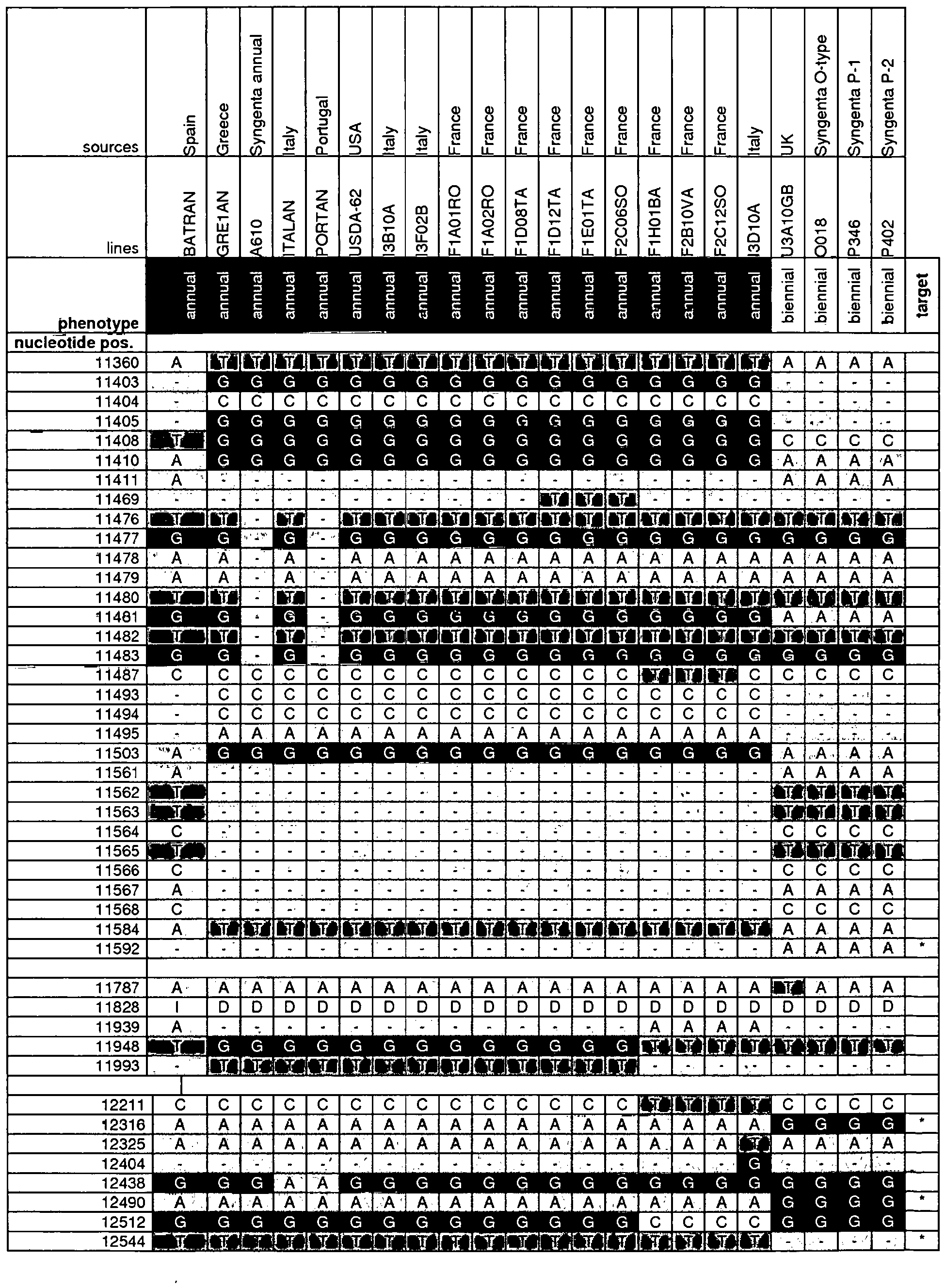

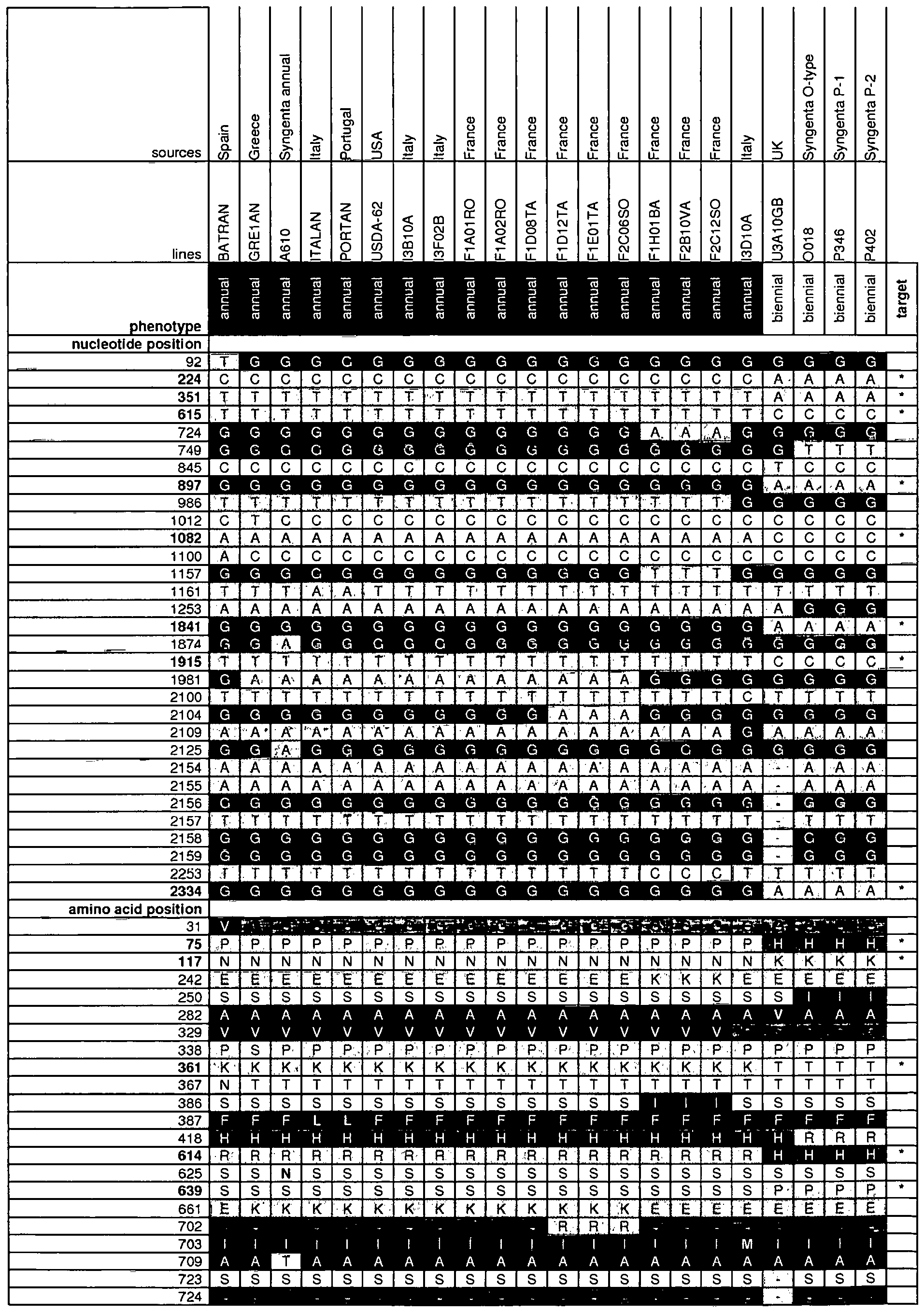

3. Chimeric construct for transgenic downregulation of *BvPRR7* gene expression in sugar beet, wherein said chimeric contruct comprises a nucleic acid sequence under the control of regulatory elements that are functional in plants, wherein said nucleic acid sequences is encoding a dsRNA which is capable of targeting mRNAs for degradation which are produced by transcription of the endogenous sugar beet DNA sequence encoding the *BvPRR7* protein, and wherein said nucleic acid molecule encoding said dsRNA has a length of at least 21 nucleotides and is 90% identical with at least a part of the nucleic acid sequence selected from the group of nucleic acid sequences consisting of SEQ ID NOs: 1, 7, 8, 9, 10, 53 or 54.

4. Chimeric construct according to claim 3 further comprising a selection marker which allows discrimination between transformed and non-transformed plant material in a selection process.

5. Chimeric construct according to claim 3 or 4 wherein the nucleic acid molecule encoding said dsRNA molecule has the nucleotide sequence consisting of SEQ ID NO: 1 under the control of a constitutive promoter.

6. Plant expression vector comprising the chimeric construct according to any one of claims 3 to 5.

7. Plant expression vector according to claim 6 which is an RNAi expression vector.

8. Sugar beet plant cell comprising a chimeric construct according to any one of claims 3 to 5 or a vector according to any one of claims 6 and 7.

9. Transgenic sugar beet plant having a phenotype of delayed bolting comprising a plant cell according to claim 8 or a chimeric construct according to any one of claims 3 to 5.

10. Sugar beet plant parts selected from the group consisting of seeds, embryos, microspores, zygotes, protoplasts, ovules, pollen, taproots, cotyledons, extracts or biological samples, which are derived from the transgenic sugar beet plant according to claim 9, wheren said plant parts comprise a plant cell according to claim 8 or a chimeric construct according to any one of claims 3 to 5.

11. Method of transforming sugar beet plants, wherein said method comprises the use of a nucleic acid sequence according to claim 1 or 2 or of a chimeric construct according to any one of claims 3 to 5 or of a vector according to any one of claims 6 and 7.

12. Pair of primers consisting of a forward primer and a reverse primer which primers are capable of annealing to a nucleotide sequence within a genomic region of the sugar beet genome DNA that shows perfect co-segregation with *BvPRR7,* wherein said primers anneal to a nucleic acid sequence according to claim 1 or 2.

13. Pair of primers according to claim 12
which anneals to a nucleotide sequence as set forth in SEQ ID NO: 8 and amplifies an informative fragment from said sequence comprising a polymorphism selected from the polymorphisms based on a SNP present in the different alleles of said sequence as shown in Tables 7-1 and 7-2 and wherein Tables 7-1 and 7-2 are depicted as shown in claim 2.

14. A pair of primers according to claim 12 or 13 comprising
a. forward primer PRR7(T6)-F as depicted in SEQ ID NO: 49 and reverse primer PRR7(T6)-R as set forth in SEQ ID NO: 50 for amplifying a fragment comprising SNP #2334; or
b. forward primer *PRR7*(T1)-F as set forth in SEQ ID NO: 13 and reverse primer *PRR7*(T1)-R as depicted in SEQ ID NO: 14 for amplifying a fragment comprising SNP #160.

15. Method of identifying the absence or presence of an allele associated with annuality in a sugar beet plant, which allows to discriminate between annual and biennial sugar beet plants, wherein a pair of primers according to any one of claims 12 to 14 is used.

## Patentansprüche

1. Nukleinsäuresequenz, die für das *BvPRR7-Gen* der Zuckerrübe codiert, umfassend eine Nukleinsäuresequenz, ausgewählt aus der Gruppe der Nukleinsäuresequenzen, die aus SEQ ID NO: 4, 5, 6, 7, 8, 9, 10, 53 oder 54 besteht.

2. Nukleinsäuresequenz, umfassend SEQ ID NO: 8, wobei die Sequenz eine oder mehrere Nukleinsäuresubstitutionen, -deletionen oder -additionen wie in den Tabellen 7-1 und 7-2 dargestellt umfasst, wobei die in den Tabellen 7-1 und 7-2 dargestellten Polymorphismen jeweils 18 einjährige bzw. 2 zweijährige Allele von SEQ ID NO: 8 darstellen, wobei die Tabellen 7-1 und 7-2 wie folgt abgebildet sind:

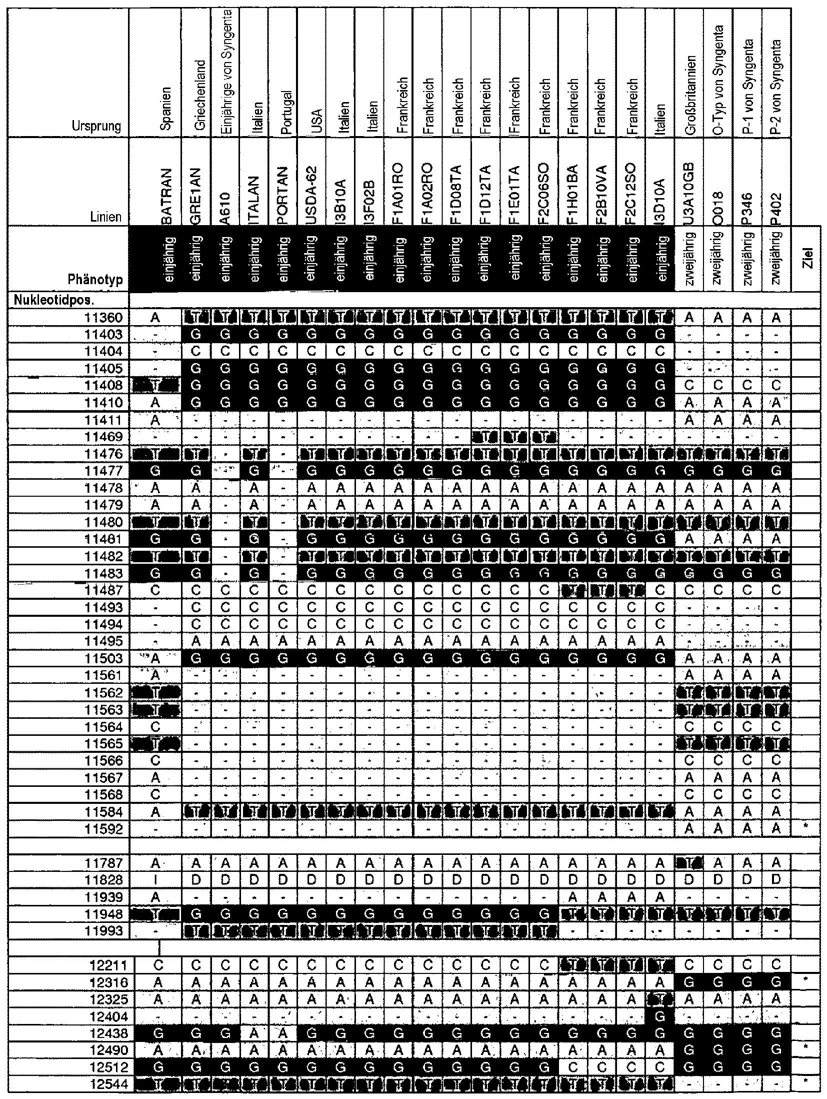

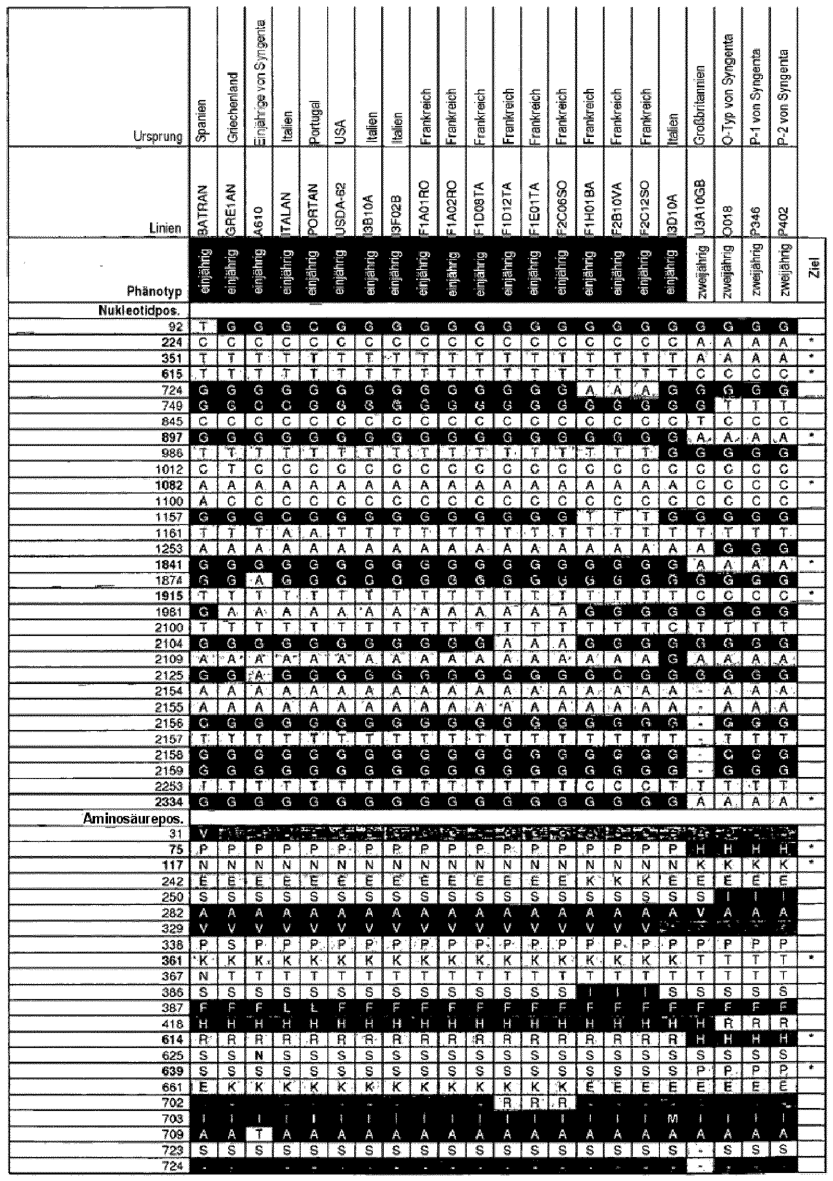

3. Chimäres Konstrukt für das transgene Herunterregulieren der *BvPRR7*-Genexpression in der Zuckerrübe, wobei das chimäre Konstrukt eine Nukleinsäuresequenz unter der Kontrolle von Regulationselementen, die in Pflanzen funktionell sind, umfasst, wobei die Nukleinsäuresequenzen für eine dsRNA codiert, die fähig ist, auf mRNAs, die durch Transkription der endogenen Zuckerrüben-DNA-Sequenz, die für das *BvPRR7*-Protein codiert, zwecks Abbau zielzusteuern, und wobei das Nukleinsäuremolekül, das für die dsRNA codiert, eine Länge von mindestens 21 Nukleotiden aufweist und zu 90% mit mindestens einem Teil der Nukleinsäuresequenz, ausgewählt aus der Gruppe von Nukleinsäuresequenzen bestehend aus SEQ ID NO: 1, 7, 8, 9, 10, 53 oder 54, identisch ist.

4. Chimäres Konstrukt nach Anspruch 3, das weiterhin einen Selektionsmarker umfasst, der es ermöglicht, in einem Selektionsverfahren zwischen transformiertem und nichttransformiertem Pflanzenmaterial zu unterscheiden.

5. Chimäres Konstrukt nach Anspruch 3 oder 4, wobei das Nukleinsäuremolekül, das für das dsRNA-Molekül codiert, die Nukleotidsequenz bestehend aus SEQ ID NO: 1 unter der Kontrolle eines konstitutiven Promoters aufweist.

6. Pflanzenexpressionsvektor, umfassend das chimäre Konstrukt nach einem der Ansprüche 3 bis 5.

7. Pflanzenexpressionsvektor nach Anspruch 6, bei dem es sich um einen RNAi-Expressionsvektor handelt.

8. Zuckerrübenpflanzenzelle, umfassend ein chimäres Konstrukt nach einem der Ansprüche 3 bis 5 oder einen Vektor nach einem der Ansprüche 6 und 7.

9. Transgene Zuckerrübenpflanze mit einem verzögertem Schossphänotyp, umfassend eine Pflanzenzelle nach Anspruch 8 oder ein chimäres Konstrukt nach einem der Ansprüche 3 bis 5.

10. Zuckerrübenpflanzenzelle, ausgewählt aus der Gruppe bestehend aus Samen, Embryonen, Mikrosporen, Zygoten, Protoplasten, Ovula, Pollen, Pfahlwurzeln, Kotyledonen, Extrakten oder biologischen Proben, die von der transgenen Zuckerrübenpflanze nach Anspruch 9 stammen, wobei die Pflanzenteile eine Pflanzenzelle nach Anspruch 8 oder ein chimäres Konstrukt nach einem der Ansprüche 3 bis 5 umfassen.

11. Verfahren zum Transformieren von Zuckerrübenpflanzen, wobei das Verfahren die Verwendung einer Nukleinsäuresequenz nach Anspruch 1 oder 2 oder eines chimären Konstrukts nach einem der Ansprüche 3 bis 5 oder eines Vektors nach einem der Ansprüche 6 und 7 umfasst.

12. Primerpaar, bestehend aus einem Vorwärtsprimer und einem Rückwärtsprimer, wobei die Primer fähig sind, sich an eine Nukleotidsequenz innerhalb einer Genomregion der Zuckerrübengenom-DNA, die perfekt mit *BvPRR7* kosegregiert, anzulagern, wobei sich die Primer an einer Nukleinsäuresequenz nach Anspruch 1 oder 2 anlagern.

13. Primerpaar nach Anspruch 12, das sich an eine Nukleotidsequenz gemäß SEQ ID NO: 8 anlagert und ein informationstragendes Fragment von der Sequenz, das einen Polymorphismus umfasst, der aus den Polymorphismen ausgewählt ist, die auf einem in den unterschiedlichen Allelen der Sequenz vorliegenden SNP basieren, wie in den Tabellen 7-1 und 7-2 dargestellt, amplifiziert, wobei die Tabellen 7-1 und 7-2 wie in Anspruch 2 gezeigt dargestellt sind.

14. Primerpaar nach Anspruch 12 oder 13, das Folgendes umfasst:
a. Vorwärtsprimer PRR7(T6)-F wie in SEQ ID NO: 49 dargestellt und Rückwärtsprimer PRR7(T6)-R wie in SEQ ID NO: 50 dargestellt zum Amplifizieren eines Fragments, das SNP #2334 umfasst; oder
b. Vorwärtsprimer *PRR7*(T1)-F wie in SEQ ID NO: 13 dargestellt und Rückwärtsprimer *PRR7*(T1)-R wie in SEQ ID NO: 14 dargestellt, zum Amplifizieren eines Fragments, das SNP #160 umfasst.

15. Verfahren zum Identifizieren der Ab- oder Anwesenheit eines mit Einjährigkeit in einer Zuckerrübenpflanze assoziierten Allels, das es ermöglicht, zwischen einjährigen und zweijährigen Zuckerrübenpflanzen zu unterscheiden, wobei ein Primerpaar nach einem der Ansprüche 12 bis 14 eingesetzt wird.

## Revendications

1. Séquence d'acide nucléique, qui code pour le gène *BvPRR7* de la betterave à sucre, comprenant une séquence d'acide nucléique choisie dans le groupe des séquences d' acides nucléiques consistant en SEQ ID NO : 4, 5, 6, 7, 8, 9, 10, 53 ou 54.

2. Séquence d'acide nucléique comprenant SEQ ID NO : 8, ladite séquence comprenant une ou plusieurs substitutions, délétions ou additions d'acides nucléiques, telles que présentées dans les Tableaux 7-1 et 7-2, les polymorphismes présentés dans les Tableaux 7-1 et 7-2 représentant respectivement 18 allèles annuels et 2 allèles bisannuels de SEQ ID NO : 8, les Tableaux 7-1 et 7-2 se présentant comme suit :

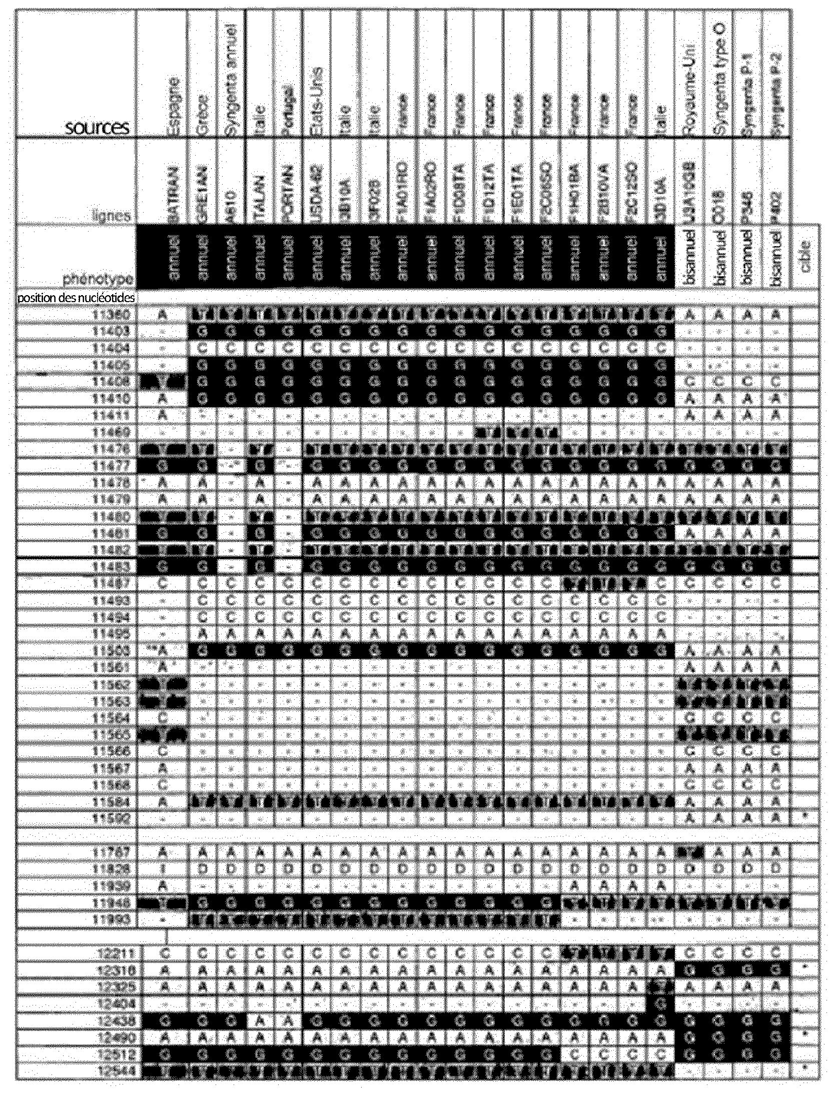

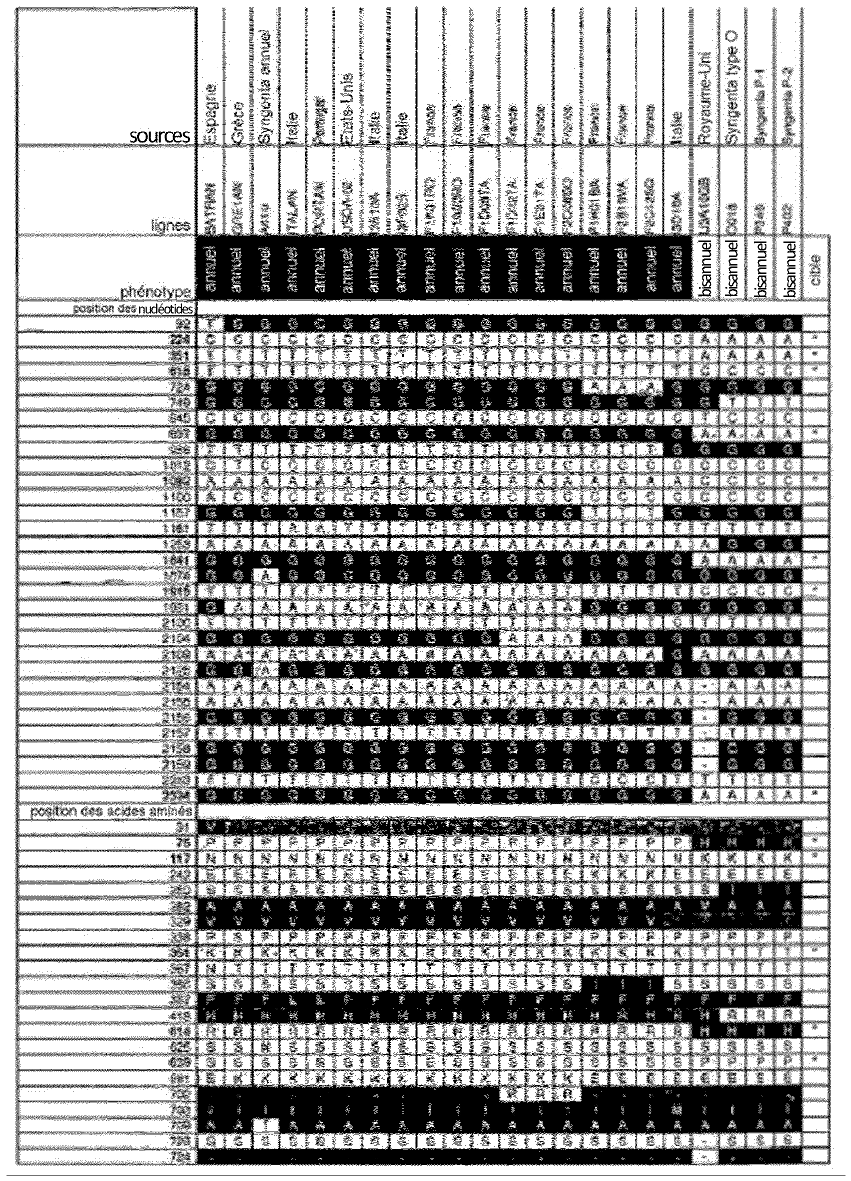

3. Construction chimère pour régulation négative transgénique de l'expression du gène *BvPRR7* dans la betterave à sucre, ladite construction chimère comprenant une séquence d'acide nucléique sous le contrôle d'éléments régulateurs qui sont fonctionnels dans les plantes, lesdites séquences d'acides nucléiques codant pour un ARNdb qui est capable de cibler des ARNm pour une dégradation, qui sont produits par transcription de la séquence d'ADN de la betterave à sucre endogène codant pour la protéine *BvPRR7,* et ladite molécule d'acide nucléique codant pour ledit ARNdb ayant une longueur d'au moins 21 nucléotides et ayant une identité de 90 % avec au moins une partie de la séquence d'acide nucléique choisie dans le groupe des séquences d'acides nucléiques consistant en SEQ ID NO : 1, 7, 8, 9, 10, 53 ou 54.

4. Construction chimère selon la revendication 3, comprenant en outre un marqueur de sélection, qui permet une distinction entre un matériel végétal transformé et un matériel végétal non transformé dans un processus de sélection.

5. Construction chimère selon la revendication 3 ou 4, dans laquelle la molécule d'acide nucléique codant pour ladite molécule d'ARNdb a la séquence nucléotidique consistant en SEQ ID NO : 1 sous le contrôle d'un promoteur constitutif.

6. Vecteur d'expression de plante comprenant la construction chimère selon l'une quelconque des revendications 3 à 5.

7. Vecteur d'expression de plante selon la revendication 6, qui est un vecteur d'expression d'ARNi.

8. Cellule végétale de betterave à sucre comprenant une construction chimère selon l'une quelconque des revendications 3 à 5 ou un vecteur selon l'une quelconque des revendications 6 et 7.

9. Plante de betterave à sucre transgénique ayant un phénotype de montée à graines retardée, comprenant une cellule végétale selon la revendication 8 ou une construction chimère selon l'une quelconque des revendications 3 à 5.

10. Parties de plante de betterave à sucre choisies dans le groupe consistant en les graines, les embryons, les microspores, les zygotes, les protoplastes, les ovules, le pollen, les racines pivotantes, les cotylédons et les extraits ou les échantillons biologiques, qui dérivent de la plante de betterave à sucre transgénique selon la revendication 9, lesdites parties de plante comprenant une cellule végétale selon la revendication 8 ou une construction chimère selon l'une quelconque des revendications 3 à 5.

11. Procédé de transformation de plantes de betterave à sucre, ledit procédé comprenant l'utilisation d'une séquence d'acide nucléique selon la revendication 1 ou 2 ou d'une construction chimère selon l'une quelconque des revendications 3 à 5 ou d'un vecteur selon l'une quelconque des revendications 6 et 7.

12. Paire d'amorces consistant en une amorce directe et une amorce inverse, lesquelles amorces étant capables de s'anneler à une séquence nucléotidique à l'intérieur d'une région génomique de l'ADN du génome de la betterave à sucre qui présente une co-ségrégation parfaite avec *BvPRR7,* lesdites amorces s'annelant à une séquence d'acide nucléique selon la revendication 1 ou 2.

13. Paire d'amorces selon la revendication 12, qui s'annèle à une séquence nucléotidique telle que présentée dans SEQ ID NO : 8 et amplifie un fragment informatif provenant de ladite séquence comprenant un polymorphisme choisi parmi les polymorphismes se fondant sur un SNP présent dans les différents allèles de ladite séquence, tels que présentés dans les Tableaux 7-1 et 7-2, les Tableaux 7-1 et 7-2 étant tels que présentés dans la revendication 2.

14. Paire d'amorces selon la revendication 12 ou 13, comprenant
a. une amorce directe PRR7(T6)-F telle que présentée dans SEQ ID NO : 49 et une amorce inverse *PRR7*(T6)-R telle que présentée dans SEQ ID NO : 50, pour amplifier un fragment contenant le SNP #2334 ; ou
b. une amorce directe *PRR7*(T1)-F telle que présentée dans SEQ ID NO : 13 et une amorce inverse *PRR7*(T1)-R telle que présentée dans SEQ ID NO : 14 pour amplifier un fragment comprenant le SNP #160.

15. Procédé pour identifier la présence ou l'absence d'un allèle associé à l'annualité dans une plante de betterave à sucre, qui permet de faire une distinction entre les plantes de betterave à sucre annuelles et bisannuelles, dans lequel on utilise une paire d'amorces selon l'une quelconque des revendications 12 à 14.


### SEQUENCE LISTING

<110> Syngenta Participations AG
<120> TRANSGENIC SUGAR BEET PLANTS
<130> 71918 PCT2
<150> PCT/EP2008/056390
   <151> 2008-05-28
<160> 58
<170> PatentIn version 3.3
<210> 1
   <211> 840
   <212> DNA
   <213> Beta vulgaris
<400> 1
   gctcctgtca ttatgatgtc atctcatgat tcgatgggtt tagtcttaaa gtgcttatcc    60
   aagggcgctg ttgactttct ggtgaagcct ataagaaaaa acgaacttaa aaacctttgg   120
   cagcatgttt ggaggaggtg tcacagttct agtggtagtg gaagtgaaag ctgtgtaagg   180
   aatggaaaat ccataggaag caagagggct gaagagtcgg acaatgacac tgacatcaat   240
   gaggaagatg ataacagaag cattggttta caagctcggg atggaagtga caatggaagt   300
   gggacccaga gttcatggac aaaaagggct gcagaagttg agagccccca accacagtct   360
   acatgggagc aagcaactga tccacctgat agcacttgtg ctcaggtcat ttatccaatg   420
   tctgaggcat ttgccagcag ctggatgcct ggatccatgc aggaacttga tggacaggat   480
   catcaatatg acaatgtccc aatgggaaag gatttggaga ttggagtacc tagaatttca   540
   gattcacggc taaatggacc aaacaaaacg gttaagttag caactactgc tgaggaaaac   600
   caatattcac agttagacct caaccaggaa aatgatggtc gaagttttga tgaagagaac   660
   ctggagatga ataatgataa acctaaaagt gagtggatta aacaggctat gaactcacca   720
   ggaaaagttg aagaacatcg tagaggaaat aaagtatctg atgcaccacc cgaaatttca   780
   aaataaagga caaaggcatg caacatgtcg aggatatgcc ttctcttgtg ctcagtctga   840
<210> 2
   <211> 23
   <212> DNA
   <213> Artificial
<220>
   <223> oliginucleotide sequence - Primer PRR7-F
<400> 2
   atgtcatctc atgattcgat ggg 23
<210> 3
   <211> 21
   <212> DNA
   <213> Artificial
<220>
   <223> oligonucleotide sequence - Primer PRR7-R
<400> 3
   tcagccctct tgcttcctat g 21
<210> 4
   <211> 493
   <212> DNA
   <213> Beta vulgaris
<400> 4
   atgtcatctc atgattcgat gggtttagtc ttaaagtgct tatccaaggg cgctgttgac 60
   tttctggtga agcctataag aaaaaatgaa cttaaaaacc tttggcagca tgtttggagg 120
   aggtgtcaca gtgtaagtgt ctttacattt tccagctttt catcagctta gtggttcgtg 180
   tagcagtctt tcagattttc gaactttcta gcacatatga caaattaaac ctgcatgcta 240
   attcccgatt agataatgga ataagctctt tcagctggtc ttttacttct ttctcttctc 300
   ctcttatgaa aaactggtat gccactatgc atcttgttcc aggtgtttgt ttagtgtttc 360
   tttcctttat tcgttttttt gtttttattt ttaattttaa ttttaatttt tcctcattct 420
   ttttttagtc tagtggtagt ggaagtgaaa gctgtgtaag gaatggaaaa tccataggaa 480
   gcaagagggc tga 493
<210> 5
   <211> 493
   <212> DNA
   <213> Beta vulgaris
<400> 5
   atgtcatctc atgattcgat gggtttagtc ttaaagtgct tatccaaggg cgctgttgac 60
   tttctggtga agcctataag aaaaaatgaa cttaaaaacc tttggcagca tgtttggagg 120
   aggtgtcaca gtgtaagtgt ctttacattt tccagctttt catcagctta gtggttcgtg 180
   tagcagtctt tcaaattttc gaactttcta gcacatatga caaattaaac ctgcatgcta 240
   attcccgatt agataatgga ataagctctt tcagctggtc ttttacttct ttctcttctc 300
   ctcttatgaa aaactggtat gccactatgc atcttgttcc aggtgtttgt ttagtgtttc 360
   tttcctttat tcgttttttt gtttttattt ttaattttaa ttttagtttt tcctcattct 420
   ttttttagtc tagtggtagt ggaagtgaaa gctgtgtaag gaatggaaaa tccataggaa 480
   gcaagagggc tga 493
<210> 6
   <211> 493
   <212> DNA
   <213> Beta vulgaris
<400> 6
   atgtcatctc atgattcgat gggtttagtc ttaaagtgct tatccaaggg cgctgttgac 60
   tttctggtga agcctataag aaaaaaygaa cttaaaaacc tttggcagca tgtttggagg 120
   aggtgtcaca gtgtaagtgt ctttacattt tccagcttty catcagctta gtggttcgtg 180
   tagcagtctt tcarattttc gaactttcta gcacatatga caaattaaac ctgcatgcta 240
   attcccgatt agataatgga ataagctctt tcagctggtc ttttacttct ttctcttctc 300
   ctcttatgaa aaactggtat gccactatgc atcttgttcc aggtgtttgt ttagtgtttc 360
   tttcctttat tcgttttttt gtttttattt ttaattttaa ttttartttt tcctcattct 420
   ttttttagtc tagtggtagt ggaagtgaaa gctgtgtaag gaatggaaaa tccataggaa 480
   gcaagagggc tga 493
<210> 7
   <211> 15037
   <212> DNA
   <213> Beta vulgaris
<400> 7
   attattgtac atayawgacy atttacgtaa ctaaattaaa aaaagtttta aaaatgcaaa     60
   acagaaaata aaatcaaata tcgacatttg gaaatttata atagaaatga ataaaaataa    120
   gggagaaata aatgaagaac aaaataaatg agaaagagaa ttaaaatggt tcttgaaaaa    180
   taaatgagag agaaaaggag ggaatgagtg agtgatgaga gagaaagagc tggcccactt    240
   tcaaaaattc tgccaaaagc ctgccaaatt ttggccctcc taaaagcatc aaaactacgt    300
   agttttggcc aaggtgtagg atgctcatcc tacacctccg tgcaggatct aaattgcgct    360
   tagaaatagg gtctcctaat atttctctac tagcattttt tgcacgcgat gcgtgcttga    420
   atttttttca agatagaaac tcgatttttt tcgacgtatg taaaagtcaa aatttaaaca    480
   ttagacatac aaagtataat tgtttttagt tacaaaattt aattggttta gtctctgtaa    540
   cttgagtttc tcaccagtct tttttttttt tttttttttt tttactttca aagttaaatt    600
   ctatgaacaa aatagaaatt ttattgaatt tatctatgat ttctaatatt actccctccg    660
   acccaaaata tagttcccat ttcccttttt tcacggtaat ttatgcaaat agaatataag    720
   agggatagta aagatttttt gtttatttaa ataaatgttg tatgggaaaa gatgatttta    780
   ggagagaaag tagagaataa ttggtgaaag agtattaatt gtaacatttt ggttgaataa    840
   acaaaggaaa aaacaaaatt caagaagcaa ataaatgaga attgtttcct tgaataatgc    900
   aaaagtgggt tttaattccc aaaatatgcc caaaaataaa aaaattccct gtgtaccgtc    960
   cacgtaagac ggcacgcgag attttttttt cctacttcaa tacaaccgct acttaaagta   1020
   gcggtttact gatttttttt tttatctact taggtaaaac cttggcgctg agtgatataa   1080
   ctcgctactt caagtagcga tttactgaaa tccccaactc catagtttga tatgtgcttg   1140
   caacattttg cccaggtaaa ccgctactca gggtagcggt ttatgtgtat aaaccgctac   1200
   ttaaagtagc ggtttatttt aatataaacc actattgtga gtagcggttt acgtgggcaa   1260
   aaacaaaaaa aaaaatagtt tctcgcgtgt cgtcctacgt ggacggtacg cagggaattt   1320
   tttaattttt gggcatattt tgggaactaa aacccacttt tgcattattc aaggaaaaaa   1380
   ttcaaataaa tgatgggaca cggtttttct agacaaatta cgaaaaaatg tggaactaaa   1440
   tatgaaaatg gaaactatat tttgggacac ccaaaatgga aatgggaatt atattttggg   1500
   acggagggag tataattttt tagttgattt ttgaattaag tatactactt catatattgt   1560
   taagaaactg gacacttgga tttcaagtca aatttttgtg agtatgtatt gacgttgtag 1620
   tgtattggtt gtagtttgta agttaatttt tgtttttgta aagtttactc atttgagtga 1680
   tttgtataat gtaaattatg caattctatg attttagttg acttgtgagt gattgttata 1740
   attttatttc cattattttt atttgaatct ccctttggtt tgtatgtgaa tttgtaattt 1800
   agaaaggcaa aggggtaaaa tagtctcttc attcgggaac accatagttc ccctccttcc 1860
   cttatataat aaagatgatg atgatttttg ataataatga tttgtaagtg aattatgtga 1920
   atgtttttgt atgtattgac gtcctagtat attagtttta gtttgtaagt taattttttt 1980
   gtttttgtaa agtttcccga tcatttgagt gattttcgtg attttttgtg attttctcaa 2040
   ttctatgagt gatttgtaaa gtttcttgat ataagtgatt tctgagtggt gttgaattaa 2100
   tttccggtgg ctttgttaga accccatttt agtattgaca tttcttttgt aatttagaaa 2160
   gggaaagggg ggtaaaatag gcatttcaaa aaaggacacc attgctcccc ccttccctta 2220
   tgtaattgag atatcttaaa agaataccga gagttttttc ccataaagga gtattttttt 2280
   taaaattttt tccataaagg agtatttatt agtaccaagt tgatttccca aatcattatc 2340
   cttgcgcaaa ttgcataatg gagatatttg gtgttgacgt gtgaatatgg ggccataata 2400
   ataggaggtc aaaaacaaaa ctacaagggt taaaatcgtc acaatattaa acaagcatct 2460
   cacattctca ctggtcactt ttttttaacc tattaaaaga acaaaccttt aactctcctc 2520
   acaatctgac acgtgtcgaa tattgattta ctgagatcaa tttagatcct ctcccttaga 2580
   ctcttctgtc ttctcagtac agctttagat ctcaacctcc atgtcagcaa agttacctta 2640
   cgtgtcatcc tacgtggcct ctccttctac ccctcactcc tccacgtcaa cattttcctc 2700
   caaaattaaa aaatcatttt tttattatat ttacttgaat gtatataata atgtctactg 2760
   atcttcttct ttagaactat ctccttctct cattggaacc tcaaaatcat tcttatttta 2820
   tttcgagaaa aggaaaaaaa agcacatctt ttttgaagat taatttgtgg attattattg 2880
   agcttcatcg tattaaaaaa catagtaaaa gttctttcct catttgtctt tttattcatc 2940
   taattttttt tagtgaagaa ccctaatttt gtttgtgaat tctcaagttc aagttttgat 3000
   ttgggtattt tttttgatga aatttgtgca gctgtaggat gttatcgtgc tgagaaaagg 3060
   gttttagatg gtaagttttt ttttctttga tttctctctc ctactttttt ttttgttttg 3120
   ctttagataa tactgtcatg atatgatata aagaattggt gatttgggta gtttatttaa 3180
   cctatgatta tgtgttattt gttttgatct ttcaatttat ctggtgctgt gtgtatatat 3240
   gttttgtttt tcttcaagta tttggttatt attgaagtgg gtaattagga atttgctact 3300
   aatctatgga tttgggttct gttgtgatta atttactata gatttgaggt ttaatttatg 3360
   ttttataggt tagaaaagga aatcaatgat ttgtttgtgg atttgagtag attgtttgtt 3420
   agtgtgtgta tgatgatatt aacttccatt attcttcccc aaattagggg taattgatgg 3480
   ttttttgcat accgaaggcg tattctcttt gatgatggag tgattgttga aaagacatga 3540
   tgggttaaag ttgcaggatt atttcatttc aataaacata attgatcaat ttggatctgt 3600
   tgaatgaggt tgattcacaa aaatgaagat gggcccggtg ttgccaagtc ggtggcagag 3660
   cttaatcaac atatagttgc tgtgaaaaaa gaaggtaggg gtagggttgc aggtgaaggg 3720
   caggggcttt ccgaggagga cgaactgaga attattgagg atggtgaaga tgcaaacagc 3780
   aggcgttctt tgagttctgt tcagcttcca gttcatactc acaggcatca gccacaagta 3840
   caaccccagg ggagagtctg ttgggagagg tttctccctg ttggatctcc taaggttttg 3900
   ctcgtagaaa gtgatgactc aactcgtcat attgttagtg ctttgctacg gaaatgtagc 3960
   tatgaaggtg atttgatctg ttttaatccc atatatgcaa tgtcttgtcc ttatcaccta 4020
   cttcaacaaa tgattaagag aattgtactc cctcgttcca aaataatagc aacacttagc 4080
   cttcccgtag actttaggga gcgtttggtt catattatgg tatgggtttg gaattaggaa 4140
   tgaaaccaag gtggtatggg gttggaactt gatacttaat accttgtatt tggtttcatt 4200
   taggaatgaa aaaatttctt ttatttgata cctagaggta aggtatgagc catacccacc 4260
   tccccccatg ggtttctaaa ccccatacct tatgggtttg aggtatgggt ttaaaattta 4320
   aaaataagtt aaacaaacac taggtatgtg ttttgttcat tccaaaccca tacctcatac   4380
   ctaaaactag tgaaccaaac acccccttaa ggatcttggg acaaagggaa tccattacta   4440
   gatctggtga cattaatacc taagtttaca tcagtttcac ttaaatcctt cgttttaaaa 4500
   aaagtaaaaa aacctgttag tctgagtaag tttactaatt tttgttctaa aattcaacac 4560
   attatctaca tgcaagcact tactagtaca atacaactca aacaatatat gcatcctatc 4620
   tgttcacaat gaaccgaaaa ctaatctttt catacccttg tttgatgctt ttttcaggcc 4680
   atacaaattt ctttaaccta aattgcctcc tcagtcactg ttcaaaattg cagttttaac 4740
   atcctcaaga ccatgtgatg tactgttaga ttatattaag accctattgt aaataaagca 4800
   tgtatagtgg aataaaatgc atgtcttcct actttttttt gggggtcatg aactcattgt 4860
   ttgatatttt gcagttgtag gggtgccaaa tggcatagaa gcatggaaaa tcttagaaga 4920
   tttgagcaat cagattgacc tagttttaac tgaggtagtc acatcaggac tctctggtat 4980
   aggtcttctg tccaagataa tgagtcacaa aagctgccag aatactcctg tcattagtga 5040
   gctttcgttc cttgttgtat tagtgtatgt tctgtatttg attttctttc tttgtgcata 5100
   tcttgccttg ttttttacaa ttatttagat tttagatgaa aatgtatact cattttatgg 5160
   tctttagctg caacatttga ttattttgtg tgcagtgatg tcatctcatg attcgatggg 5220
   tttagtctta aagtgcttat ccaagggcgc tgttgacttt ctggtgaagc ctataagaaa 5280
   aaacgaactt aaaaaccttt ggcagcatgt ttggaggagg tgtcacagtg taagtgtctt 5340
   tacattttcc agctttccat cagcttagtg gttcgtgtag cagtctttca aattttcgaa 5400
   ctttctagca catatgacaa attaaacctg catgctaatt cccgattaga taatggaata 5460
   agctctttca gctggtcttt tacttctttc tcttctcctc ttatgaaaaa ctggtatgcc 5520
   actatgcatc ttgttccagg tgtttgttta gtgtttcttt cctttattcg tttttttgtt 5580
   tttattttta attttaattt taatttttcc tcattctttt tttagtctag tggtagtgga 5640
   agtgaaagct gtgtaaggaa tggaaaatcc ataggaagca agagggctga agagtcggac 5700
   aatgacactg acatcaatga ggaagatgat aacagaagca ttggtttaca agctcgggat 5760
   ggaagtgaca atggaagtgg gacccaggta gtgctaaccc ctgtaatatt aaacttccta 5820
   tagtaggtgt ggttaatgtg acgctgttaa ggccttttgg gtggttgctt ctagttcact 5880
   aaggataata agaaatagct cgctattgat agttagggca cctcaatatc acctcctctt 5940
   gtatgtttgt tgaactacat ttttagccag acttgagtat tttatcctga aggatagaac 6000
   aggtgcattt ttggttgcgg ttgttagttg ttactgttat gcaaagacta ttgccaccat 6060
   tttctcacac atatttaaca tggaagtgtc ctaaccaccc cccaacccaa aaaatgggag 6120
   ggagaaatta ctggagatgg gaaagaagtt acataaaaag ttagtcgttt gggtcatgat 6180
   tgtttgttgt atttgcaaag ttagcgcgtt ctcttcctgg atgcttcaaa ataagctgat 6240
   gcaccataaa gtaccactct tggcttcacc tgttggtgtg gacccaacca atgtaccctt 6300
   gttgatctcg agatagacaa agaggaagtt taatttctct ttatatgtta tctctcttca 6360
   atttgttagc agctatgtct ctttcgtgga catttagaac ccatgttagg ttcatattta 6420
   tagttaggtg attgtatcaa aattgccatc acaataaaca gaacattaat ttctattggg 6480
   aaggattcaa ggatcaaata tacaggaaag agcagtgtag gagatatcat cttgttgaac 6540
   aacaaaagaa acattaacat caactggtga taatctttgc aagattggat gacaaaatga 6600
   ggagtcgatc taatataaaa caaattggga actgtcagct atatcctgca tatcaagaat 6660
   ggagaccttt aagaaaagta agaccatttt ttgttgggaa gtcaagccat tgtcccagtt 6720
   tccttgtgaa atttagttca tcttagcttt cttctaccaa catgaattct ctttcctttc 6780
   agcccttgca aacttggttt tatgctaatt atcagtgttt ccttcattta gtacgctgag 6840
   agggtttatt tggttgatca aagaatactt gatgaccttg aggtagatgc tctacatgga 6900
   gaagttcctc taagtgtaca aagaatctag ttcgaccaac tttgatttag gaagagataa 6960
   cacgatcacc tcgtggtcta gactctggag aggtcaaagt gtgcaaaagg gtatttttga 7020
   aagacaatgg cttgttgatt catgactgaa attggatggt cgtgactgag catatactat 7080
   tagtggttct cttctaaggt gatataagta tgtgataacc caatcctgta tatttcttcg 7140
   aggacatcaa ttgtgctact attctagggt gctggagacc catacatata gagccattga 7200
   caattaacac aaacttcaac cacttatttt tatttcattt aagctatcaa tccctaagaa 7260
   agagcccatc caagctcctg ctttaggtgc atcccctccc ttttcagcta gtgcacaaaa 7320
   aatgaacttt cgagatagac tgctaaattt gctttgtcaa gaagacaaaa ttttgataca 7380
   caactgtaat tgcattttat gacacttacg ctgatatatc tgcaagtgaa gttgatatgc 7440
   aaaaactatg tagcctcctt cgtctacggt aatagatctc cgtcaatgtg atgcttgtgt 7500
   gccatcataa aatgatattg ggtctttaga ctctgttact ctacagctga aggatcttag 7560
   ccttggcatt tatatccttt ttatccaaaa gttaaaaaaa gcggaccgtt tgacccatgt 7620
   aaggaaaaag gaaaggaatc gagaaagaca aaggagggga aagaagttaa atctcctaaa 7680
   aagcttgttt tgtgcggtga gagagggagc gacttgaaat tgccattgat gatgattggt 7740
   tcacaattgt aatcgaaatc aaactcactc tctctctctc tctctctctt atcacccccc 7800
   tcaaactata acatcacagt cctttaaacg tgactgtttc gggggatagt gactggtagg   7860
   gatgggcaag ggtcgggtct ggctggaccc tagacccgga ccctaatttt tttttgtaga   7920
   cccaaacccg gaccctaagg gtctgaaaaa attggacctt gacccagacc cttagggtct   7980
   gaagggtcta gagggtcagg agggtccagg cttaaatttt ttattttgcc aaatttttag   8040
   cattattaat atcaataatc atttgaaatt cgcatgaaac aaacacaaaa aaaaatcgca   8100
   tgaatcaaac acaaaaattc gcatgaaaca aacactaaca tataaattga aaaaaacgaa   8160
   acaaacacaa acttataaac gaaaaaaatt gaaacaaaca caattccaaa catataaact   8220
   gaaaaaaaaa acgaaacaaa cacaaatata caaactgaaa aaaagaagaa acaaacacaa   8280
   cttacataag agttcagaat gggtgttata gtttatgttt tagtcattta gaaaatcaat   8340
   ttgttttttt tttaaagtta aaatgtatat attaaataag tttagggtct aaggtgttgg   8400
   aacatttata gggtaatggg tttgaaactc atatgggtat gtactagaag aggaggaggt   8460
   ctagtatgca aaaggttaga gtgcatcaag tggtaacaac gcgcattgtt ataccaatgt   8520
   cgcgagtcgc gacaggcgtc gcgggtcgcg accagcgcct cgcgagcttc ttcgcatgtc   8580
   gcgacgcgtc ttctgccttg gaatgcgaaa aaatgcctcg gcggttttat atccgttgtg   8640
   atgctttgtt gatcatttta atgactttta aggtctttta atcagtagat taaaggcctt   8700
   tgatgagtga ttaagatggg ggttatgtga ttaacctctc tagtcaatga aatgttgatt   8760
   atgcttatat aacctttgga ttcctatgag tgaggagtta gaagaaaatc agaattttct   8820
   atactctctc aaaagtcttc ttgcttagct taagagaaac cttgcaatct tctcttgagt   8880
   gttcttcaca aacacaaaac acaagttctt gttgattcac ttagaagatc atctaagtgg   8940
   attgtttctc tccattgtat ctcattagtt atttcgtgtt aacccggtga tcctagaggg   9000
   gcgaaattaa actaattgga aagcgtagtt tccgtgcctt ggagtgggat atccggttct   9060
   ctcattgatc acaagcctaa cataagggtc gggtctgggt ccaaatttta agacccggac   9120
   ccggacccta aaaaattcac ttggacccag acccggaccc ggactcttag ggtctgaaaa   9180
   agttggaccc aaacccttaa attagggtcg ggtccaacag ggtccgggta gggtcttgga   9240
   cccatgccca tccctagtga ttgggtagcc cattgcagaa tattgagaac gcaatataaa   9300
   ggggtgttga gaaagagggt tttgagtgta ttgtttaaga aagttgggaa aggaatgaga   9360
   gatgaagtac agaagaaaac gtctagaaag tgaagcatgg gagtctgttt cttttctttt   9420
   tcctaaagtt tcccaccaaa tgtcccttaa gtggttcagc cacgcctttg gacaagctta   9480
   ccaccaagct ccccatccca gatcatattt gaatcaaaca tctttctttt tttagaatat   9540
   tctttttttg tgcatgaaag ccaattccat gagatatgta ccttatattt ctctaaaata   9600
   tataaataat tgatgaagca attttcagat cattagataa gcgttctaca aaagaaccat   9660
   ctttttttgc ttccttgtgt acttggaaaa tgtagttccc atatataatt ttaccatggc   9720
   agtacttcta tagaccacta agttcttcgc ttgtgcaacc tatagtgcat ttaagagggt   9780
   ttaggtatag acagccttca ctttcaattg gttagagtct acctccagta tcactgacag   9840
   aattttcaat aggaacttct gtcataactt aattcgcaga aagcactaac taaacaaccc 9900
   cttagttctt tagttaagcg cttgattggt cacatccagc ttttagtttt tagtatggag 9960
   atttataaag tagtatgact tgagttgaat agtgaacgta agattagaca tatttatata 10020
   gtcgtgttaa ttttggaaac tgacaggagt gactagaaac cacttttttt gtgtccaaaa 10080
   tttccatata ttgtttttta aaaaaactgc taaatcacga tgataacaaa caaaccttac 10140
   acaggtaccg gaatgatatt gaaacaaatt gaggttagtg ataagccata atcccttacc 10200
   ttgaaattca gaggctgtct gctgcagtct ctatcatctt cttatttcac taaatcaatt 10260
   attacctgct tcaacctcaa cggtccgagg cttagacatt gtgtctttga tagtatcatc 10320
   acagctgaaa attaatgtgt actttcttct atttaaatac catttgagag tgcctttggt 10380
   agtcattatg aatgtcgtga gatcacaatc cgtgaaatat agttttcatc acattcttac 10440
   ctgcatgtgt aaggaaaagt atagcgttag tgttcaatct tttgctactt ctggtgactg 10500
   gtcaatggtc aaagtatgca gcatgatttt gtgtttgtca gtttcttctt taaataagtg 10560
   tgaactgctc tagtctaagt tgctcgaact cttaaaaagt gttggacttg ttagttgtta 10620
   catgtataca atgttgattg ggtgggcttt tccatatatt attatatttg ttgaatcaca 10680
   atgaagtacc tatttccatt tgaggagtag gtatgatgag gttagtaggg agtttgagtg 10740
   ttaaaggtta tgtgaagatg taaaaattca ctgacaatga gaccttagta tccgacggtc 10800
   ggaattttac caattttatt gccttgttac ctttctattt ttacttagta tttccttttc 10860
   ataaattttt gtgatctaga gttcatggac aaaaagggct gcagaagttg agagccccca 10920
   accacagtct acatgggagc aagcaactga tccacctgat agcacttgtg ctcaggtcat 10980
   ttatccaatg tctgaggcat ttgccagcag ctggatgcct ggatccatgc aggaacttga 11040
   tggacaggat catcaatatg gtatgtggta ctgtatttga tagaagttac aataatgtgt 11100
   aaactgaaac cacttaatga cctagtatcc atctgtatca gacaatgtcc caatgggaaa 11160
   ggatttggag attggagtac ctagaatttc agattcacgg ctaaatggac caaacaaaac 11220
   ggttaagtta gcaactactg ctgaggaaaa ccaatattca cagttagacc tcaaccagga 11280
   aaatgatggt cgaagttttg atgaagagaa cctggagatg aataatgata aacctaaaag 11340
   tgagtggatt aaacaggcta tgaactcacc aggaaaagtt gaagaacatc gtagaggaaa 11400
   taaagtatct gatgcaccac ccgaaatttc caaaataaag gacaaaggca tgcaacatgt 11460
   cgaggatatg ccttctcttg tgctcagtct gaagaggttg ggtgatattg cagacacgag 11520
   cactaatgtc tcagaccaga atattgttgg gcgttcagag ctttcagcct tcaccaggta 11580
   tgctagagaa ggtgaaactt gaatttatat aatggacaag tggacaatat ctcattttta 11640
   aattgttgca ggtacaattc aggcacaact ggtaaccagg gtcaaacagg taatgttggc 11700
   agttgctctc caccaaataa tagttcagaa gcagcaaagc agtcccattt tgatgctcca 11760
   catcaaattt cgaatagcag tagtaacaat aacaatatgg gctctactac taataagttc 11820
   ttcaaaaagc ctgctatgga cattgataag acacctgcaa aatcaacagt caactgttct 11880
   catcattcac atgtgtttga gccagtgcaa agttcccata tgtctaataa taaccttact 11940
   gcatctggta agcctggtgt tggctccgta aatggtatgc tgcaagaaaa cgtaccagta 12000
   aatgctgttc tgccgcaaga aaataacgtg gatcagcagc tcaagattca gcaccaccat 12060
   cactaccatc attacgatgt ccatagtgta cagcagctac caaaggtttc tgttcaacat 12120
   aatatgccca aaagcaagga tgtgacagca cccccacagt gtgggtcttc aaacacttgt 12180
   agatcgccaa ttgaagcaaa tgttgccaat tgcagtttga atggaagtgg tagtggaagc 12240
   aatcatggga gcaatttcct taatggaagt agtgctgctg tgaatgttga aggaacaaac 12300
   atggtcaatg atagtgggat agctgcaaaa gatggtgctg aaaatggaag tggtagtgga 12360
   agtggaagtg gtagtggtag tggtgttggt gtggatcaaa gtcgatcagc tcaacgagaa 12420
   gctgccttga ataaattccg tctcaagcgt aaagaaagat gctttgacaa aaaggtaata 12480
   ctccaaattc tctccagaat gtttatactt ggacatctag tatgtacatc cttgaatcta 12540
   aactgtaaaa gctgaatttc agaataaaaa acacaaatta tatcaagtat gaaggcagag 12600
   tattgtagta attatagttt ttctggtatg gaattagtac ttacatttac cagaagcctg 12660
   ctgtcacaag ccataatttg atcatcaagc aacaataatt tggccatttc ttgcttgtat 12720
   tgaaagtgag atgacttcaa acttatttgt gtatcatcac atcaggtgcg atatcaaagc 12780
   agaaagaagt tagcagatca aagacctcgt gttcgtgggc aattcgtgcg ccaggtacga 12840
   gaaaacaaag gaaggaatac cgatagctaa caccaattct ttccacaagt tgctgccaag 12900
   atcatttatg ccactctgat gtcagctgtc ttcatatgta caaatttcga attttatgtg 12960
   tgcatgaggt gctaaatact gtcaaacctc agtgattctg tttggtttag gctgtagaaa 13020
   gacatctttt cctttgtgtt ttcatggttc ttattttgag ctgtgttcac tactttttat 13080
   aacatggtag cccctggttg cctttggaaa taagcttttc cttaaaggtg tgatgcatat 13140
   aatcttgttt ggtgttagat tatatgatca tttcttcagg cgtttacggg tcacattttc 13200
   cggaatcctt tcaaacgcga ttccggaaac aatggctcat attttctttt ggtttcaagg 13260
   agaaggctat ttaaaacaga aaagatttag gttacagaaa tcagtgatga agcaatgagt 13320
   ttcattatag aataggtaga agtagggggt gttttttccg tactcttgag atagaaagtg 13380
   gggatagatt ctttggactc gtcagaaagg aataatatag ttgtctacct ttttcatttt 13440
   tagttcttgt aggagtttta ttccacttcc atttttgtaa aatttaggag ttgtaaggac 13500
   gtgtaaagag aatctgccat ccagatttta accgacggta aatttgttct tttcatgttt 13560
   tctcaagtaa ctataatgtt ttcatcgaat ctatagggat tttctaatgt gtacctgata 13620
   gaggcacaca gtaacaataa tataagtaca tatattcttt aagaataatg acatagtaat 13680
   tatattttta atacaaataa aagatgtcct tatgtaatga aacaaataac ttttccttga 13740
   aggtatgcca taattaatta ctttattttg aagatatttt atatttagtt tgggtagtgg 13800
   aactactaaa taaaaatatg gttatagtaa catgtactca tgtgcgaacc gaaaaaaacc 13860
   ctatgctttc tctaaaagtt cccaaaccct tgagcttata gccccgacgg cccagcgcag 13920
   gcttgctgga gcgccgcgtc gctcaccctg tcgccgacga gcctgcatgt cgtatcgttc 13980
   ggtcttctga aggtttagtt ttccctgttc ctctttgtgt tattcatcgt tcccatcccc 14040
   catgtctccc cttcccctgt cagtggttgt cggcctcccc ttcccctatt aatggttgtc 14100
   ggcctcccct tccctttccc ctaatagtgg ttgttggtct ccccttcccc tttcatgttg 14160
   tcaagttgtt cctttccccg ttctcccttt tcctagtcct cttttggtgt tcttgttgtt 14220
   gttagtttag tggctttggt tggttagttc ggctgagtgc ttcgtcgtcg tatgcccttc 14280
   cttgttcccc tatttggttt tggttatgtt ggggtttcgg ttaaccccgt tcccatgctt 14340
   aaacgtggga gggcctcagg atttagatat aaaggtcatc attctcgcgc ttagacgtga 14400
   gagggattaa gtgttcaggg ataagggctc cgttcctgcg cttaaacgtg ggagaactta 14460
   aaggttctag gttttacagg agttttggga ttggaaagta tatgaactct gtttggcaga 14520
   agatgacagt gcaatgtggg gattaatcat ttcgttttct tcctttttaa taagttagtc 14580
   tcttattatg agagttttct attagttcta atccccttaa tttcttgtag gggttgtaag 14640
   tctagtttgt cgttgtttag tatatctagt tcgagaagct cgaaagtttg aggttgtgga 14700
   aaaatgtact tactggttgc agatcaagaa tattaagacg aatgtttgac ttcaatttac 14760
   tattgcatca ggtaggaaat atggtgagtc atcgaatatc cattatggtt ggaatagtac 14820
   catatcatgg aagcggtttc gaagcgtgta tattagtaaa atagatgaag atattcaaat 14880
   cgatgtttta gattatcttt tatgtacgta agggtcatta ttgttgtaga tgttgtatgg 14940
   tttttaattt taatgataat ttttccttat tcccacttaa aagtaaacaa tgcattcatg 15000
   tgcacatatt agtacatata tttgtatata catctcg 15037
<210> 8
   <211> 24128
   <212> DNA
   <213> Beta vulgaris
<400> 8
   maaacgttgt gatcatctaa tattattgaa tatattatct ccataactta tcctaatatt    60
   atttagttta ttacacttga tcgaggacaa aatccttcaa tctcccactt gtctaagaac   120
   aagtgtgtaa ccttcaaact ccttaagtcg cttaatgtct aacttgatga catgataaca   180
   tcatatgttc atcataacaa tattcaagtc gttccttgaa atctgagttt gaactgtcga   240
   aacaaatgat taacttctta atccatttga gcacggccat gcattttcag ttctcactct   300
   tcaagaggcc aagacaccaa tcctaactct taggaggact tatccaatct tgtatgacca   360
   aagctcccac tcaattcata gcagttccaa tcgctgcttt tataacctcc ttttacggca   420
   cggcgttttg cagcgtcaag aacatactaa tccttaagta agaacagttt catactcatg   480
   tcaaaggaat ccactaaata tattaataag agtctcataa accttttaga gaactcccac   540
   taggtctgcc cagcgtgtat caactataca agcctatgca aatgactaga catctccatg   600
   tccctatagc ccatgaaact gcgctatcaa tcaacttgca atctagtcca tgaaattgaa   660
   tcatttacgt tcaacttaat gattcgaact agggactaag gtatattata actcctgttc   720
   actggataga gttccattcg tcaaatcacg tatttgacaa ttctatcaaa cgttataaaa   780
   tactttgaac gttttattta atactaaacc aagattaaat aagaacaaaa cttttattga 840
   taaacataaa cataacatat caaagcgagt aattataact gtgaactaat taaaagtaaa 900
   tagtacacaa ttaaacccac tctcctatat gcttaagccc tatagcccta gtatgactct 960
   catgcttggg ctgtggcaaa ggtttagtca aaggatcagc gacattacta tccgtatgaa 1020
   ccttgcaaac tattacatcc tttctctcaa cgatttctcg aatgagatga aactttctaa 1080
   gtacatgttt acatctttga tgtgatcttg gttccttaga ctgagctatg gcaccattgt 1140
   tatcacaatg taaaacaata ccatctccaa cactaggcac tactcctagc tccagaatga 1200
   acttcttcat ccaaacggct tcctttgctg catctgctgc agcaatatac tcagcttctg 1260
   tcgtagaatc agcgacagtg ctttgctttg aacttttcca gctcactgcc cctccattta 1320
   gacaaaagat gaaaccagat tgggatcgga aatcatcttt gtcagtttgg aaacttgcat 1380
   ctgtgtaacc ctcaacaatt aacttacttt tacctccata cactaagaaa ttatccttag 1440
   tccttctcaa gtactttagg atattcttag ctgcactcca gtgtgcgtca cctggatttg 1500
   attggaatct gctacacatg ctcaaggcat atgaaacatc tgggcgagta caaatcatgg 1560
   agtacataat ggagcctata gctgacgcat aaggaacatt actcattcgc ttaatctcat 1620
   caggcccaga aggacactga gtcttgctaa gcgacactcc atgttgcatg ggtaggaagc 1680
   ctctcttaga gttttccatg ttgaacttag tgacgatctt atctatataa gttcgttggc 1740
   taagtccgat catcctctta gacctatccc tatagatctt gatccccaaa atatactcgg 1800
   cgttttcgag gtctttcata gaaaaacaac tttttaacca ttccttgact gactcaagca 1860
   tgggaatgtt gtttcctatg agaagtatgt catctacata caagaccaag aagactatgt 1920
   tactcccact ttccttcttg taaacacaag actcttctcc atttttaaga aaaccaaact 1980
   ctttgattgc ctcatcaaaa cgaagattcc aactccgtga tgcttgcttc aatccataaa 2040
   tggatttttg aagcttacat accctcctag gattttctgg atccacaaaa ccctccggct 2100
   gtgtcatata cacatcctct ttcaagaacc cattcaagaa agcggttttg acatccattt 2160
   gccaaatctc gtaatcatag aaggcggcga tcgctaggag tatccgaacg gatttaagca 2220
   tggctaccgg tgaaaaggtt tcgtcatagt ctataccatg aacttgcttg aacccttttg 2280
   caaccaacct tgctttgtaa acctgaatat taccatcctt gtctgttttc actttgaaaa 2340
   cccatttgca accaataggt gtgatcccat cgggcaaatc taccaagtcc catacttgat 2400
   tttcagacat ggatgccatt tcggacctca tggcttcgag ccatttttcg gagtcttcac 2460
   tcatcaaagc ttgcttgtaa gtagtaggtt cctcaaattc taaaatcatt atctcagaat 2520
   tttcagttaa caagaaatca acaaacctct tggttggtat tcttgttcta ctagacttac 2580
   gaggggctgc aacaggagaa attttcttct caacaatatg agaattttcg cacgaattag 2640
   attcgagtgg gacaatagga ggttcgtgta cgggatgcac gtcctccaac acttggtcag 2700
   ttatgggaga agattcctcc aaaggaggaa ctacttcaag cccaacatct ggctcttgtg 2760
   tcgttgtctc taacatagga tgaactatgt ccatttgttg atcttctcga acttcttcga 2820
   gaaatacatt actcccactt gcctttttgg aaataaaatc tttttccaaa aagacaccac 2880
   gacgagcaac aaacactttg ccctcagtgc gattgtagaa gtaatagccc ttggtttcct 2940
   ttggataacc cacaaagaaa cacttatctg atttaggggc gagtttatct gaaagtaaac 3000
   gctttacata aacctcacat ccccaaatac gtagaaaaga caagtttgga acttttccac 3060
   tccatatctc atatggtgtc ttatctactg cctttgatgg agttctatta agtgtgaaag 3120
   tagcagtttc gagagcatat ccccagaagg atattggaag atcagcaaaa ctcatcatag 3180
   accgaaccat atcaagtaga gttcgattcc tcctttccga aacaccattc aactgaggtg 3240
   ttccaggcgg agtgagttgt gaaagaattc cacaactctt caagtgatca ttaaactctt 3300
   ggctcaaata ttcaccacca cgatcggatc gcagtgcttt gattgttttg ccaagctggt 3360
   tttggacttc attttggaat tctttgaatt tttcaaatga ttccgactta tgcttcatta 3420
   aatagacata tccatatcta cttaaatcgt ccgtaaaagt aatgaagtat ccaaaccctc 3480
   ccctagcttt tgtgctcatt ggtccacata catcagtatg tattaggccc aatagatcac 3540
   tgaccttttc accctttcca gtaaaaggtg actttgtcat tttacccatt aaacatgatt 3600
   cacacacatc aaatggctca aagtcaaaag atgttagaag tccatcttta tgcaacttct 3660
   gaatgcgttt cgcgtttatg tgtcctaaac gacaatgcca taagtaagta ggattagtat 3720
   cgcttgttct atgttttttg ttgtctatgt taaggacatc tttgtctaag tctaggtaat 3780
   atagaccatt agacctctta gcagtggcat aaaacattga attcaaataa acagagaaac 3840
   aacctttctc aattgtaaat gaaaaccctt cattatccaa aacaggaata gaaataatgt 3900
   ttttggtaat agcaggaacg taataacaat tattaagctc taatattaat ccagaaggca 3960
   aaggtagact ataagtccca actgcaacgg cggcaactct tgcaccattt ccaactcgca 4020
   gttccacctc tcctttagcc aaggttctac tccttcttag tccctgcaca ttcgaagtaa 4080
   tgtgagaaac acaaccggta tcaaataccc aagaagtaga tgttgctaaa ttaatgtcaa 4140
   tgacatagat acctgaagaa gaagccccag acttcttatc cttcaagtac tttgggcagt 4200
   tacgcttcca atgacctatt tgatcacaat agaagcactt ggcatttgcg gccacctttg 4260
   gctttgcctt tgcctgtggc ttaatagcag tcttagtggc aacttgcttg cccttatctt 4320
   gctttttctt gccagcccat tccttcttaa aacccttttc cttttgaacc ataagaactt 4380
   ccttcttagg tgcaatagtg atgttttgct cggcagtcat gagcatccca tgcaactcag 4440
   caagggtttt cgacacccca ttcatattga aattcaatcg aaacgtattg aaccccttat 4500
   gaagtgagtg cagaatgatg tcagtagcca actcttggct ataaggaaag cccaacctct 4560
   ccatggcttc aaaataacca atcatttcga agacatgagt ggcgaccggc ttgccttcaa 4620
   ctaacgaaca ctcaagaata gccttgtgag tttcatacct ctctatccga gcttgttgtt 4680
   gaaacatggt tttcaactgt cggataatgc tataggcatc caagcttgca aacctctttt 4740
   gaaggcttgg ttccatagcc gctagcataa ggcaagtaac aattactgac ctttctgcaa 4800
   cggccttatg ggcctctttc tcagcatcag tagaggtagt agttaaaact gggatcggtg 4860
   tttcaagaac atcctctcga ccttcggacc taagaacaat tcttagattc ctttcccaat 4920
   caaggaagtt gttcccgttc aatttatcct tctcaaggat tgaacgtaag ttaaaaggtg 4980
   aattattgtt gttaccagac atgatatcta catagaagat gcaaaaagta taagtatgtt 5040
   tatcataata gcttttaaca aattttaaac actttaaaat aaaagctatg cacttgacca 5100
   attttaatgt gtcccttttg aatcaagtgg ttctaagatc ctatcaaaca tgatttataa 5160
   gtggactttg gcctcaactt aaaaccaagt ttaaaaggta agtaaactcc tttactaatt 5220
   acaacaattg taactcttag ttaatgggtg attgctaagg tgattacgct cccaggtaag 5280
   gaagttaccc acaacgttgg ggagagcctt cctaatccta gacagagcat gtcacccaaa 5340
   cacaaaaacc cataaacttt gctacaaaat ccaaaaccgt tttgatgatt ttgttgggcc 5400
   aaaccaaact aaacttgcaa atttcggaaa tttactctac ttagcccaag attgaaagta 5460
   atactctgct ttggcagaac ctattactaa cgatcaagtt ttagtaggtg tttatttgga 5520
   atagcaaaaa cccaatattt tatttaaggg acctaagtaa attattatgt tgatttaatt 5580
   gctagtgaac atttaaataa ttaaatcaca agcataataa acttagaaag catttaaaag 5640
   caatatttaa atgcataaaa ttaaatatga tcctagtatg gcccctaaac ctaaagacta 5700
   ctctttaaga ctcccttgtt gaatcaccat ggatctccat ccttgtgctt cataggataa 5760
   gattgaatca ccattcttct tattaatact tgaataaata ttttttgaaa ttataaacta 5820
   aaaaattaca aaaaatacca acgatgcgta gatcgtattt agattacaaa aatacattaa 5880
   cgatacgcat atcgtatttt ctatccaagt tttgggccat actagtcacc gcatgcattc 5940
   ataatatcat atatacaaaa acatgcattt taatcaacta ttaaaataaa ttatcatgtt 6000
   ttaacaactt taaaacataa taacaccatg aagatttaat cacacattaa atcctatggt 6060
   tggtacctta agacaaaatt taatcatatt agaatttcgt ctcacaaagg ctttaaaata 6120
   ttaatctaac aaatttaatc atattaaact taaagaaaaa ttaaagcaat tgtaggcacc 6180
   acatataatt taatcatatt aaaaacaaaa acttaacatg atgactaacc acataaaaag 6240
   ggcatgaaag aattaatcaa ctattaatac taacaaccta acatgtaatt aacatcataa 6300
   aaaaataata atagttacta actccttagt aacccccttt aaaattaact agtcaattat 6360
   cacatataat taactaataa aattaaagct cattatttaa ttcaattatg acttaaatat 6420
   aaaattaatc accattaatt aatttatttg caaattggaa tatactcaaa aacaagaaaa 6480
   agaaagaaaa aaaaaaaaaa agcaggctgc caaggcagca gtgtacactg ccacctcagt 6540
   gccggccacc tgcgcgacca ccagaaacga ccagaacctg ccaccgcgtc gctggccacg 6600
   gcgaccagca ccggcagcac tgcagcgcag gcagcaggcc gcgccaacag cagcgcccag 6660
   cgcaggaagc tcgcgccgcg cgagccacca cgacgccggc cacagtccgg cggcacgcca 6720
   gccaccatgt cggtcgaatt ccggtggacc ttcccccttt ccctttcaat tatcatcaac 6780
   ccttgtgcat aattgaatga aagttacaac aaattgattt ggggaaaaaa ttagggttca 6840
   tatcaatttt gttttaaaaa aaamcatgaa ctaacacaaa aaatctgata ttttgtgatg 6900
   tgagatttca attttgagta taatatatat ttatatatat acatwaaaat ccaattttta 6960
   tgtttccaat caattaatat cataatatca attatgcaaa taaattcata tataaagccc 7020
   tcccttaatt gaattaaaaa atgaaataaa acatgcatca acatgatcat attaatctat   7080
   gcaataggct aactgatacc actgtaggaa cttagatgca taatgcggaa aatcaagtat   7140
   caaatacttg tacatctatc ccaagatcat tgcataaatt agtatgaatc aaacaatagt   7200
   atagaattat acctttgatg cgtatgttcc tcttgtcacc aaacttctag tggagatcac   7260
   cttagaacgt caagcgccgt tcctctaatg ttggtccacg aacaacactt ggatcaccac   7320
   gtatgctagt acggaagaga gaaaaacact ctcttacttt tgtggtgagg gccgaaaatg   7380
   agtgtgaaaa gactaaggga aaaatcagat ttttcactct agaagttgta aaagtgtata   7440
   tccacctttg taaccccata tcaatatata aggtggttac aaaagaggtg tttcatgagg   7500
   ctttatttts cctcataatg tcatacatta tgagtctaat aaactcatga gttacaactc   7560
   ttcccatcca tcatcaaacc gcgcaaccca tttcacaaat ggatttggat aaatatccaa   7620
   gtgtcattac ttgtgtgacc tcataggact caatgatatt agtagttggc cctaatcata   7680
   ttagtccaac aaaccacaat tagcttctag caaaacgttg tgatcatcta atattattga   7740
   atatattatc tccataactt atcctaatat tatttagttt attacacttg atcgaggaca   7800
   aaatccttca caaatgcata tggtttgatg tacaataata tacgagtgta catttgggta   7860
   ttttcaatga tcaaagtaat gaccatcagt gtacattgtg atttatcctt atttacgttg   7920
   gttgcggtac ctttttatta ttattattag ctccacctac agttgcatgt acatgcacgt   7980
   acctagcatg tacactttgt tgacattcat gtacattaac cgggttaacg ttacaattat   8040
   gttgttatgt gttgaccttt tgttttaata ctcgtattga gttttttttg ttttgtttgt   8100
   gtctatatca caaggattgt actttggatg tctattattg ttcattgtgt gttattgacg   8160
   attttatggg gggatgtcat tgtgcatttt gattttgtta atgaacaacc acgaagccaa   8220
   gaatgtacaa agaaacataa tagaataaaa gtaacccaat tcctaaagct gatgtcaagt   8280
   gagtaatttg caatctttgt acactggtgt gttgatgttt gttcgcttat gaaattcaat   8340
   atgtacaatt atagtcatat acctcatagt gctccaggtg ccacaaaaaa aactcaatat   8400
   gggtattaaa acaaaaggtc aatacataac aacatgattg caacccagtt aatgtacatg   8460
   aatgtcaaca aagtgtacat gcaaggtacg tgcatgtaca tgcaactgta tgtggagcta   8520
   ataataaaaa aaggtaccgc aaccaacgta aataaggata aatcacaatg tacactgatg   8580
   gtcattactt tgatcactga aaataaggat acattattgt ttgattgaat gttcactgga   8640
   tatgactcaa tgtacaaata ttctagcaag attgttcaat tattaagcct gaatgtacaa   8700
   tgttgttatg actgaatgtt caagttattt tatagagctg actttgttct gtgtacatta   8760
   aagttgcgtt aatgatcatt gtgtatgact aaatatacat tagcttcact aacatgcgtg   8820
   cataacatat tctatagaca caaacaaaac aaaaaaaaac tcaatatggg tactagatta   8880
   aaaggtcaac acataacaac ctgattgtaa cccagttaat gtacatgaat gtcaacaaag   8940
   tgtacatgca aggtacgtgc atgtacatgc aactgtagat ggagctaata ataaaaaaaa   9000
   ggttccacaa ccaacgtaaa taaggataaa tcacaatgta cactgatggt cattactttg   9060
   atcactgaaa atacccaaat gtacactcgt atattattgt acatcaaacc atatgcattt 9120
   gttacattaa aaaaagtttt aaaaatgcaa aacagaaaat aaaatcaaat atcgacattt 9180
   ggaaatttat aatagaaatg aataaaaata agggagaaat aaatgaagaa caaaataaat 9240
   gagaaagaga attaaaatgg ttcttgaaaa ataaatgaga gagaaaagga gggaatgagt 9300
   gagtgatgag agagaaagag ctggcccact ttcaaaaatt ctgccaaaag cctgccaaat 9360
   tttggccctc ctaaaagcat caaaactacg tagttttggc caaggtgtag gatgctcatc 9420
   ctacacctcc gtgcaggatc taaattgcgc ttagaaatag ggtctcctaa tatttctcta 9480
   ctagcatttt ttgcacgcga tgcgtgcttg aatttttttc aagatagaaa ctcgattttt 9540
   ttcgacgtat gtaaaagtca aaatttaaac attagacata caaagtataa ttgtttttag 9600
   ttacaaaatt taattggttt agtctctgta acttgagttt ctcaccagtc tttttttttt 9660
   tttttttttt ttttactttc aaagttaaat tctatgaaca aaatagaaat tttattgaat 9720
   ttatctatga tttctaatat tactccctcc gacccaaaat atagttccca tttccctttt 9780
   ttcacggtaa tttatgcaaa tagaatataa gagggatagt aaagattttt tgtttattta 9840
   aataaatgtt gtatgggaaa agatgatttt aggagagaaa gtagagaata attggtgaaa 9900
   gagtattaat tgtaacattt tggttgaata aacaaaggaa aaaacaaaat tcaagaagca 9960
   aataaatgag aattgtttcc ttgaataatg caaaagtggg ttttaattcc caaaatatgc 10020
   ccaaaaataa aaaaattccc tgtgtaccgt ccacgtaaga cggcacgcga gatttttttt 10080
   tcctacttca atacaaccgc tacttaaagt agcggtttac tgattttttt ttttatctac 10140
   ttaggtaaaa ccttggcgct gagtgatata actcgctact tcaagtagcg atttactgaa 10200
   atccccaact ccatagtttg atatgtgctt gcaacatttt gcccaggtaa accgctactc 10260
   agggtagcgg tttatgtgta taaaccgcta cttaaagtag cggtttattt taatataaac 10320
   cactattgtg agtagcggtt tacgtgggca aaaacaaaaa aaaaaatagt ttctcgcgtg 10380
   tcgtcctacg tggacggtac gcagggaatt ttttaatttt tgggcatatt ttgggaacta 10440
   aaacccactt ttgcattatt caaggaaaaa attcaaataa atgatgggac acggtttttc 10500
   tagacaaatt acgaaaaaat gtggaactaa atatgaaaat ggaaactata ttttgggaca 10560
   cccaaaatgg aaatgggaat tatattttgg gacggaggga gtataatttt ttagttgatt 10620
   tttgaattaa gtatactact tcatatattg ttaagaaact ggacacttgg atttcaagtc 10680
   aaatttttgt gagtatgtat tgacgttgta gtgtattggt tgtagtttgt aagttaattt 10740
   ttgtttttgt aaagtttact catttgagtg atttgtataa tgtaaattat gcaattctat 10800
   gattttagtt gacttgtgag tgattgttat aattttattt ccattatttt tatttgaatc 10860
   tccctttggt ttgtatgtga atttgtaatt tagaaaggca aaggggtaaa atagtctctt 10920
   cattcgggaa caccatagtt cccctccttc ccttatataa taaagatgat gatgattttt 10980
   gataataatg atttgtaagt gaattatgtg aatgtttttg tatgtattga cgtcctagta 11040
   tattagtttt agtttgtaag ttaatttttt tgtttttgta aagtttcccg atcatttgag 11100
   tgattttcgt gattttttgt gattttctca attctatgag tgatttgtaa agtttcttga 11160
   tataagtgat ttctgagtgg tgttgaatta atttccggtg gctttgttag aaccccattt 11220
   tagtattgac atttcttttg taatttagaa agggaaaggg gggtaaaata ggcatttcaa 11280
   aaaaggacac cattgctccc cccttccctt atgtaattga gatatcttaa aagaataccg 11340
   agagtttttt cccataaagg agtatttttt ttaaaatttt ttccataaag gagtatttat 11400
   tagtaccaag ttgatttccc aaatcattat ccttgcgcaa attgcataat ggagatattt 11460
   ggtgttgacg tgtgaatatg gggccataat aataggaggt caaaaacaaa actacaaggg 11520
   ttaaaatcgt cacaatatta aacaagcatc tcacattctc actggtcact tttttttaac 11580
   ctattaaaag aacaaacctt taactctcct cacaatctga cacgtgtcga atattgattt 11640
   actgagatca atttagatcc tctcccttag actcttctgt cttctcagta cagctttaga 11700
   tctcaacctc catgtcagca aagttacctt acgtgtcatc ctacgtggcc tctccttcta 11760
   cccctcactc ctccacgtca acattttcct ccaaaattaa aaaatcattt ttttattata 11820
   tttacttgaa tgtatataat aatgtctact gatcttcttc tttagaacta tctccttctc 11880
   tcattggaac ctcaaaatca ttcttatttt atttcgagaa aaggaaaaaa aagcacatct 11940
   tttttgaaga ttaatttgtg gattattatt gagcttcatc gtattaaaaa acatagtaaa 12000
   agttctttcc tcatttgtct ttttattcat ctaatttttt ttagtgaaga accctaattt 12060
   tgtttgtgaa ttctcaagtt caagttttga tttgggtatt ttttttgatg aaatttgtgc 12120
   agctgtagga tgttatcgtg ctgagaaaag ggttttagat ggtaagtttt tttttctttg 12180
   atttctctct cctacttttt tttttgtttt gctttagata atactgtcat gatatgatat 12240
   aaagaattgg tgatttgggt agtttattta acctatgatt atgtgttatt tgttttgatc 12300
   tttcaattta tctggtgctg tgtgtatata tgttttgttt ttcttcaagt atttggttat 12360
   tattgaagtg ggtaattagg aatttgctac taatctatgg atttgggttc tgttgtgatt 12420
   aatttactat agatttgagg tttaatttat gttttatagg ttagaaaagg aaatcaatga 12480
   tttgtttgtg gatttgagta gattgtttgt tagtgtgtgt atgatgatat taacttccat 12540
   tattcttccc caaattaggg gtaattgatg gttttttgca taccgaaggc gtattctctt 12600
   tgatgatgga gtgattgttg aaaagacatg atgggttaaa gttgcaggat tatttcattt 12660
   caataaacat aattgatcaa tttggatctg ttgaatgagg ttgattcaca aaaatgaaga 12720
   tgggcccggt gttgccaagt cggtggcaga gcttaatcaa catatagttg ctgtgaaaaa 12780
   agaaggtagg ggtagggttg caggtgaagg gcaggggctt tccgaggagg acgaactgag 12840
   aattattgag gatggtgaag atgcaaacag caggcgttct ttgagttctg ttcagcttcc 12900
   agttcatact cacaggcatc agccacaagt acaaccccag gggagagtct gttgggagag 12960
   gtttctccct gttggatctc ctaaggtttt gctcgtagaa agtgatgact caactcgtca 13020
   tattgttagt gctttgctac ggaaatgtag ctatgaaggt gatttgatct gttttaatcc 13080
   catatatgca atgtcttgtc cttatcacct acttcaacaa atgattaaga gaattgtact 13140
   ccctcgttcc aaaataatag caacacttag ccttcccgta gactttaggg agcgtttggt 13200
   tcatattatg gtatgggttt ggaattagga atgaaaccaa ggtggtatgg ggttggaact 13260
   tgatacttaa taccttgtat ttggtttcat ttaggaatga aaaaatttct tttatttgat 13320
   acctagaggt aaggtatgag ccatacccac ctccccccat gggtttctaa accccatacc 13380
   ttatgggttt gaggtatggg tttaaaattt aaaaataagt taaacaaaca ctaggtatgt 13440
   gttttgttca ttccaaaccc atacctcata cctaaaacta gtgaaccaaa caccccctta 13500
   aggatcttgg gacaaaggga atccattact agatctggtg acattaatac ctaagtttac 13560
   atcagtttca cttaaatcct tcgttttaaa aaaagtaaaa aaacctgtta gtctgagtaa 13620
   gtttactaat ttttgttcta aaattcaaca cattatctac atgcaagcac ttactagtac 13680
   aatacaactc aaacaatata tgcatcctat ctgttcacaa tgaaccgaaa actaatcttt 13740
   tcataccctt gtttgatgct tttttcaggc catacaaatt tctttaacct aaattgcctc 13800
   ctcagtcact gttcaaaatt gcagttttaa catcctcaag accatgtgat gtactgttag 13860
   attatattaa gaccctattg taaataaagc atgtatagtg gaataaaatg catgtcttcc 13920
   tacttttttt tgggggtcat gaactcattg tttgatattt tgcagttgta ggggtgccaa 13980
   atggcataga agcatggaaa atcttagaag atttgagcaa tcagattgac ctagttttaa 14040
   ctgaggtagt cacatcagga ctctctggta taggtcttct gtccaagata atgagtcaca 14100
   aaagctgcca gaatactcct gtcattagtg agctttcgtt ccttgttgta ttagtgtatg 14160
   ttctgtattt gattttcttt ctttgtgcat atcttgcctt gttttttaca attatttaga 14220
   ttttagatga aaatgtatac tcattttatg gtctttagct gcaacatttg attattttgt 14280
   gtgcagtgat gtcatctcat gattcgatgg gtttagtctt aaagtgctta tccaagggcg 14340
   ctgttgactt tctggtgaag cctataagaa aaaacgaact taaaaacctt tggcagcatg 14400
   tttggaggag gtgtcacagt gtaagtgtct ttacattttc cagctttcca tcagcttagt 14460
   ggttcgtgta gcagtctttc aaattttcga actttctagc acatatgaca aattaaacct 14520
   gcatgctaat tcccgattag ataatggaat aagctctttc agctggtctt ttacttcttt 14580
   ctcttctcct cttatgaaaa actggtatgc cactatgcat cttgttccag gtgtttgttt 14640
   agtgtttctt tcctttattc gtttttttgt ttttattttt aattttaatt ttaatttttc 14700
   ctcattcttt ttttagtcta gtggtagtgg aagtgaaagc tgtgtaagga atggaaaatc 14760
   cataggaagc aagagggctg aagagtcgga caatgacact gacatcaatg aggaagatga 14820
   taacagaagc attggtttac aagctcggga tggaagtgac aatggaagtg ggacccaggt 14880
   agtgctaacc cctgtaatat taaacttcct atagtaggtg tggttaatgt gacgctgtta 14940
   aggccttttg ggtggttgct tctagttcac taaggataat aagaaatagc tcgctattga 15000
   tagttagggc acctcaatat cacctcctct tgtatgtttg ttgaactaca tttttagcca 15060
   gacttgagta ttttatcctg aaggatagaa caggtgcatt tttggttgcg gttgttagtt 15120
   gttactgtta tgcaaagact attgccacca ttttctcaca catatttaac atggaagtgt 15180
   cctaaccacc ccccaaccca aaaaatggga gggagaaatt actggagatg ggaaagaagt 15240
   tacataaaaa gttagtcgtt tgggtcatga ttgtttgttg tatttgcaaa gttagcgcgt 15300
   tctcttcctg gatgcttcaa aataagctga tgcaccataa agtaccactc ttggcttcac 15360
   ctgttggtgt ggacccaacc aatgtaccct tgttgatctc gagatagaca aagaggaagt 15420
   ttaatttctc tttatatgtt atctctcttc aatttgttag cagctatgtc tctttcgtgg 15480
   acatttagaa cccatgttag gttcatattt atagttaggt gattgtatca aaattgccat 15540
   cacaataaac agaacattaa tttctattgg gaaggattca aggatcaaat atacaggaaa 15600
   gagcagtgta ggagatatca tcttgttgaa caacaaaaga aacattaaca tcaactggtg 15660
   ataatctttg caagattgga tgacaaaatg aggagtcgat ctaatataaa acaaattggg 15720
   aactgtcagc tatatcctgc atatcaagaa tggagacctt taagaaaagt aagaccattt 15780
   tttgttggga agtcaagcca ttgtcccagt ttccttgtga aatttagttc atcttagctt 15840
   tcttctacca acatgaattc tctttccttt cagcccttgc aaacttggtt ttatgctaat 15900
   tatcagtgtt tccttcattt agtacgctga gagggtttat ttggttgatc aaagaatact 15960
   tgatgacctt gaggtagatg ctctacatgg agaagttcct ctaagtgtac aaagaatcta 16020
   gttcgaccaa ctttgattta ggaagagata acacgatcac ctcgtggtct agactctgga 16080
   gaggtcaaag tgtgcaaaag ggtatttttg aaagacaatg gcttgttgat tcatgactga 16140
   aattggatgg tcgtgactga gcatatacta ttagtggttc tcttctaagg tgatataagt 16200
   atgtgataac ccaatcctgt atatttcttc gaggacatca attgtgctac tattctaggg 16260
   tgctggagac ccatacatat agagccattg acaattaaca caaacttcaa ccacttattt 16320
   ttatttcatt taagctatca atccctaaga aagagcccat ccaagctcct gctttaggtg 16380
   catcccctcc cttttcagct agtgcacaaa aaatgaactt tcgagataga ctgctaaatt 16440
   tgctttgtca agaagacaaa attttgatac acaactgtaa ttgcatttta tgacacttac 16500
   gctgatatat ctgcaagtga agttgatatg caaaaactat gtagcctcct tcgtctacgg 16560
   taatagatct ccgtcaatgt gatgcttgtg tgccatcata aaatgatatt gggtctttag 16620
   actctgttac tctacagctg aaggatctta gccttggcat ttatatcctt tttatccaaa 16680
   agttaaaaaa agcggaccgt ttgacccatg taaggaaaaa ggaaaggaat cgagaaagac 16740
   aaaggagggg aaagaagtta aatctcctaa aaagcttgtt ttgtgcggtg agagagggag 16800
   cgacttgaaa ttgccattga tgatgattgg ttcacaattg taatcgaaat caaactcact 16860
   ctctctctct ctctctctct tatcaccccc ctcaaactat aacatcacag tcctttaaac 16920
   gtgactgttt cgggggatag tgactggtag ggatgggcaa gggtcgggtc tggctggacc 16980
   ctagacccgg accctaattt ttttttgtag acccaaaccc ggaccctaag ggtctgaaaa 17040
   aattggacct tgacccagac ccttagggtc tgaagggtct agagggtcag gagggtccag 17100
   gcttaaattt tttattttgc caaattttta gcattattaa tatcaataat catttgaaat 17160
   tcgcatgaaa caaacacaaa aaaaaatcgc atgaatcaaa cacaaaaatt cgcatgaaac 17220
   aaacactaac atataaattg aaaaaaacga aacaaacaca aacttataaa cgaaaaaaat 17280
   tgaaacaaac acaattccaa acatataaac tgaaaaaaaa aacgaaacaa acacaaatat 17340
   acaaactgaa aaaaagaaga aacaaacaca acttacataa gagttcagaa tgggtgttat 17400
   agtttatgtt ttagtcattt agaaaatcaa tttgtttttt ttttaaagtt aaaatgtata 17460
   tattaaataa gtttagggtc taaggtgttg gaacatttat agggtaatgg gtttgaaact 17520
   catatgggta tgtactagaa gaggaggagg tctagtatgc aaaaggttag agtgcatcaa 17580
   gtggtaacaa cgcgcattgt tataccaatg tcgcgagtcg cgacaggcgt cgcgggtcgc 17640
   gaccagcgcc tcgcgagctt cttcgcatgt cgcgacgcgt cttctgcctt ggaatgcgaa 17700
   aaaatgcctc ggcggtttta tatccgttgt gatgctttgt tgatcatttt aatgactttt 17760
   aaggtctttt aatcagtaga ttaaaggcct ttgatgagtg attaagatgg gggttatgtg 17820
   attaacctct ctagtcaatg aaatgttgat tatgcttata taacctttgg attcctatga 17880
   gtgaggagtt agaagaaaat cagaattttc tatactctct caaaagtctt cttgcttagc 17940
   ttaagagaaa ccttgcaatc ttctcttgag tgttcttcac aaacacaaaa cacaagttct 18000
   tgttgattca cttagaagat catctaagtg gattgtttct ctccattgta tctcattagt 18060
   tatttcgtgt taacccggtg atcctagagg ggcgaaatta aactaattgg aaagcgtagt 18120
   ttccgtgcct tggagtggga tatccggttc tctcattgat cacaagccta acataagggt 18180
   cgggtctggg tccaaatttt aagacccgga cccggaccct aaaaaattca cttggaccca 18240
   gacccggacc cggactctta gggtctgaaa aagttggacc caaaccctta aattagggtc 18300
   gggtccaaca gggtccgggt agggtcttgg acccatgccc atccctagtg attgggtagc 18360
   ccattgcaga atattgagaa cgcaatataa aggggtgttg agaaagaggg ttttgagtgt 18420
   attgtttaag aaagttggga aaggaatgag agatgaagta cagaagaaaa cgtctagaaa 18480
   gtgaagcatg ggagtctgtt tcttttcttt ttcctaaagt ttcccaccaa atgtccctta 18540
   agtggttcag ccacgccttt ggacaagctt accaccaagc tccccatccc agatcatatt 18600
   tgaatcaaac atctttcttt ttttagaata ttcttttttt gtgcatgaaa gccaattcca 18660
   tgagatatgt accttatatt tctctaaaat atataaataa ttgatgaagc aattttcaga 18720
   tcattagata agcgttctac aaaagaacca tctttttttg cttccttgtg tacttggaaa 18780
   atgtagttcc catatataat tttaccatgg cagtacttct atagaccact aagttcttcg 18840
   cttgtgcaac ctatagtgca tttaagaggg tttaggtata gacagccttc actttcaatt 18900
   ggttagagtc tacctccagt atcactgaca gaattttcaa taggaacttc tgtcataact 18960
   taattcgcag aaagcactaa ctaaacaacc ccttagttct ttagttaagc gcttgattgg 19020
   tcacatccag cttttagttt ttagtatgga gatttataaa gtagtatgac ttgagttgaa 19080
   tagtgaacgt aagattagac atatttatat agtcgtgtta attttggaaa ctgacaggag 19140
   tgactagaaa ccactttttt tgtgtccaaa atttccatat attgtttttt aaaaaaactg 19200
   ctaaatcacg atgataacaa acaaacctta cacaggtacc ggaatgatat tgaaacaaat 19260
   tgaggttagt gataagccat aatcccttac cttgaaattc agaggctgtc tgctgcagtc 19320
   tctatcatct tcttatttca ctaaatcaat tattacctgc ttcaacctca acggtccgag 19380
   gcttagacat tgtgtctttg atagtatcat cacagctgaa aattaatgtg tactttcttc 19440
   tatttaaata ccatttgaga gtgcctttgg tagtcattat gaatgtcgtg agatcacaat 19500
   ccgtgaaata tagttttcat cacattctta cctgcatgtg taaggaaaag tatagcgtta 19560
   gtgttcaatc ttttgctact tctggtgact ggtcaatggt caaagtatgc agcatgattt 19620
   tgtgtttgtc agtttcttct ttaaataagt gtgaactgct ctagtctaag ttgctcgaac 19680
   tcttaaaaag tgttggactt gttagttgtt acatgtatac aatgttgatt gggtgggctt 19740
   ttccatatat tattatattt gttgaatcac aatgaagtac ctatttccat ttgaggagta 19800
   ggtatgatga ggttagtagg gagtttgagt gttaaaggtt atgtgaagat gtaaaaattc 19860
   actgacaatg agaccttagt atccgacggt cggaatttta ccaattttat tgccttgtta 19920
   cctttctatt tttacttagt atttcctttt cataaatttt tgtgatctag agttcatgga 19980
   caaaaagggc tgcagaagtt gagagccccc aaccacagtc tacatgggag caagcaactg 20040
   atccacctga tagcacttgt gctcaggtca tttatccaat gtctgaggca tttgccagca 20100
   gctggatgcc tggatccatg caggaacttg atggacagga tcatcaatat ggtatgtggt 20160
   actgtatttg atagaagtta caataatgtg taaactgaaa ccacttaatg acctagtatc 20220
   catctgtatc agacaatgtc ccaatgggaa aggatttgga gattggagta cctagaattt 20280
   cagattcacg gctaaatgga ccaaacaaaa cggttaagtt agcaactact gctgaggaaa 20340
   accaatattc acagttagac ctcaaccagg aaaatgatgg tcgaagtttt gatgaagaga 20400
   acctggagat gaataatgat aaacctaaaa gtgagtggat taaacaggct atgaactcac 20460
   caggaaaagt tgaagaacat cgtagaggaa ataaagtatc tgatgcacca cccgaaattt 20520
   ccaaaataaa ggacaaaggc atgcaacatg tcgaggatat gccttctctt gtgctcagtc 20580
   tgaagaggtt gggtgatatt gcagacacga gcactaatgt ctcagaccag aatattgttg 20640
   ggcgttcaga gctttcagcc ttcaccaggt atgctagaga aggtgaaact tgaatttata 20700
   taatggacaa gtggacaata tctcattttt aaattgttgc aggtacaatt caggcacaac 20760
   tggtaaccag ggtcaaacag gtaatgttgg cagttgctct ccaccaaata atagttcaga 20820
   agcagcaaag cagtcccatt ttgatgctcc acatcaaatt tcgaatagca gtagtaacaa 20880
   taacaatatg ggctctacta ctaataagtt cttcaaaaag cctgctatgg acattgataa 20940
   gacacctgca aaatcaacag tcaactgttc tcatcattca catgtgtttg agccagtgca 21000
   aagttcccat atgtctaata ataaccttac tgcatctggt aagcctggtg ttggctccgt 21060
   aaatggtatg ctgcaagaaa acgtaccagt aaatgctgtt ctgccgcaag aaaataacgt 21120
   ggatcagcag ctcaagattc agcaccacca tcactaccat cattacgatg tccatagtgt 21180
   acagcagcta ccaaaggttt ctgttcaaca taatatgccc aaaagcaagg atgtgacagc 21240
   acccccacag tgtgggtctt caaacacttg tagatcgcca attgaagcaa atgttgccaa 21300
   ttgcagtttg aatggaagtg gtagtggaag caatcatggg agcaatttcc ttaatggaag 21360
   tagtgctgct gtgaatgttg aaggaacaaa catggtcaat gatagtggga tagctgcaaa 21420
   agatggtgct gaaaatggaa gtggtagtgg aagtggaagt ggtagtggta gtggtgttgg 21480
   tgtggatcaa agtcgatcag ctcaacgaga agctgccttg aataaattcc gtctcaagcg 21540
   taaagaaaga tgctttgaca aaaaggtaat actccaaatt ctctccagaa tgtttatact 21600
   tggacatcta gtatgtacat ccttgaatct aaactgtaaa agctgaattt cagaataaaa 21660
   aacacaaatt atatcaagta tgaaggcaga gtattgtagt aattatagtt tttctggtat 21720
   ggaattagta cttacattta ccagaagcct gctgtcacaa gccataattt gatcatcaag 21780
   caacaataat ttggccattt cttgcttgta ttgaaagtga gatgacttca aacttatttg 21840
   tgtatcatca catcaggtgc gatatcaaag cagaaagaag ttagcagatc aaagacctcg 21900
   tgttcgtggg caattcgtgc gccaggtacg agaaaacaaa ggaaggaata ccgatagcta 21960
   acaccaattc tttccacaag ttgctgccaa gatcatttat gccactctga tgtcagctgt 22020
   cttcatatgt acaaatttcg aattttatgt gtgcatgagg tgctaaatac tgtcaaacct 22080
   cagtgattct gtttggttta ggctgtagaa agacatcttt tcctttgtgt tttcatggtt 22140
   cttattttga gctgtgttca ctacttttta taacatggta gcccctggtt gcctttggaa 22200
   ataagctttt ccttaaaggt gtgatgcata taatcttgtt tggtgttaga ttatatgatc 22260
   atttcttcag gcgtttacgg gtcacatttt ccggaatcct ttcaaacgcg attccggaaa 22320
   caatggctca tattttcttt tggtttcaag gagaaggcta tttaaaacag aaaagattta 22380
   ggttacagaa atcagtgatg aagcaatgag tttcattata gaataggtag aagtaggggg 22440
   tgttttttcc gtactcttga gatagaaagt ggggatagat tctttggact cgtcagaaag 22500
   gaataatata gttgtctacc tttttcattt ttagttcttg taggagtttt attccacttc 22560
   catttttgta aaatttagga gttgtaagga cgtgtaaaga gaatctgcca tccagatttt 22620
   aaccgacggt aaatttgttc ttttcatgtt ttctcaagta actataatgt tttcatcgaa 22680
   tctataggga ttttctaatg tgtacctgat agaggcacac agtaacaata atataagtac 22740
   atatattctt taagaataat gacatagtaa ttatattttt aatacaaata aaagatgtcc 22800
   ttatgtaatg aaacaaataa cttttccttg aaggtatgcc ataattaatt actttatttt 22860
   gaagatattt tatatttagt ttgggtagtg gaactactaa ataaaaatat ggttatagta 22920
   acatgtactc atgtgcgaac cgaaaaaaac cctatgcttt ctctaaaagt tcccaaaccc 22980
   ttgagcttat agccccgacg gcccagcgca ggcttgctgg agcgccgcgt cgctcaccct 23040
   gtcgccgacg agcctgcatg tcgtatcgtt cggtcttctg aaggtttagt tttccctgtt 23100
   cctctttgtg ttattcatcg ttcccatccc ccatgtctcc ccttcccctg tcagtggttg 23160
   tcggcctccc cttcccctat taatggttgt cggcctcccc ttccctttcc cctaatagtg 23220
   gttgttggtc tccccttccc ctttcatgtt gtcaagttgt tcctttcccc gttctccctt 23280
   ttcctagtcc tcttttggtg ttcttgttgt tgttagttta gtggctttgg ttggttagtt 23340
   cggctgagtg cttcgtcgtc gtatgccctt ccttgttccc ctatttggtt ttggttatgt 23400
   tggggtttcg gttaaccccg ttcccatgct taaacgtggg agggcctcag gatttagata 23460
   taaaggtcat cattctcgcg cttagacgtg agagggatta agtgttcagg gataagggct 23520
   ccgttcctgc gcttaaacgt gggagaactt aaaggttcta ggttttacag gagttttggg 23580
   attggaaagt atatgaactc tgtttggcag aagatgacag tgcaatgtgg ggattaatca 23640
   tttcgttttc ttccttttta ataagttagt ctcttattat gagagttttc tattagttct 23700
   aatcccctta atttcttgta ggggttgtaa gtctagtttg tcgttgttta gtatatctag 23760
   ttcgagaagc tcgaaagttt gaggttgtgg aaaaatgtac ttactggttg cagatcaaga 23820
   atattaagac gaatgtttga cttcaattta ctattgcatc aggtaggaaa tatggtgagt 23880
   catcgaatat ccattatggt tggaatagta ccatatcatg gaagcggttt cgaagcgtgt 23940
   atattagtaa aatagatgaa gatattcaaa tcgatgtttt agattatctt ttatgtacgt 24000
   aagggtcatt attgttgtag atgttgtatg gttttttaat ttaatgataa tttttcctta 24060
   ttcccactta aaagtaaaca atgcattcat gtgcacatat tagtacatat atttgtatat 24120
   acatctcg 24128
<210> 9
   <211> 2367
   <212> DNA
   <213> Beta vulgaris
<400> 9
   atgaggttga ttcacaaaaa tgaagatggg cccggtgttg ccaagtcggt ggcagagctt    60
   aatcaacata tagttgctgt gaaaaaagaa ggtaggggta gggttgcagg tgaagggcag   120
   gggctttccg aggaggacga actgagaatt attgaggatg gtgaagatgc aaacagcagg   180
   cgttctttga gttctgttca gcttccagtt catactcaca ggcatcagcc acaagtacaa   240
   ccccagggga gagtctgttg ggagaggttt ctccctgttg gatctcctaa ggttttgctc   300
   gtagaaagtg atgactcaac tcgtcatatt gttagtgctt tgctacggaa atgtagctat   360
   gaagttgtag gggtgccaaa tggcatagaa gcatggaaaa tcttagaaga tttgagcaat   420
   cagattgacc tagttttaac tgaggtagtc acatcaggac tctctggtat aggtcttctg   480
   tccaagataa tgagtcacaa aagctgccag aatactcctg tcattatgat gtcatctcat   540
   gattcgatgg gtttagtctt aaagtgctta tccaagggcg ctgttgactt tctggtgaag   600
   cctataagaa aaaacgaact taaaaacctt tggcagcatg tttggaggag gtgtcacagt   660
   tctagtggta gtggaagtga aagctgtgta aggaatggaa aatccatagg aagcaagagg   720
   gctgaagagt cggacaatga cactgacatc aatgaggaag atgataacag aagcattggt   780
   ttacaagctc gggatggaag tgacaatgga agtgggaccc agagttcatg gacaaaaagg   840
   gctgcagaag ttgagagccc ccaaccacag tctacatggg agcaagcaac tgatccacct   900
   gatagcactt gtgctcaggt catttatcca atgtctgagg catttgccag cagctggatg   960
   cctggatcca tgcaggaact tgatggacag gatcatcaat atgacaatgt cccaatggga  1020
   aaggatttgg agattggagt acctagaatt tcagattcac ggctaaatgg accaaacaaa  1080
   acggttaagt tagcaactac tgctgaggaa aaccaatatt cacagttaga cctcaaccag  1140
   gaaaatgatg gtcgaagttt tgatgaagag aacctggaga tgaataatga taaacctaaa  1200
   agtgagtgga ttaaacaggc tatgaactca ccaggaaaag ttgaagaaca tcgtagagga  1260
   aataaagtat ctgatgcacc acccgaaatt tccaaaataa aggacaaagg catgcaacat   1320
   gtcgaggata tgccttctct tgtgctcagt ctgaagaggt tgggtgatat tgcagacacg   1380
   agcactaatg tctcagacca gaatattgtt gggcgttcag agctttcagc cttcaccagg   1440
   tacaattcag gcacaactgg taaccagggt caaacaggta atgttggcag ttgctctcca   1500
   ccaaataata gttcagaagc agcaaagcag tcccattttg atgctccaca tcaaatttcg   1560
   aatagcagta gtaacaataa caatatgggc tctactacta ataagttctt caaaaagcct   1620
   gctatggaca ttgataagac acctgcaaaa tcaacagtca actgttctca tcattcacat   1680
   gtgtttgagc cagtgcaaag ttcccatatg tctaataata accttactgc atctggtaag   1740
   cctggtgttg gctccgtaaa tggtatgctg caagaaaacg taccagtaaa tgctgttctg   1800
   ccgcaagaaa ataacgtgga tcagcagctc aagattcagc accaccatca ctaccatcat   1860
   tacgatgtcc atagtgtaca gcagctacca aaggtttctg ttcaacataa tatgcccaaa   1920
   agcaaggatg tgacagcacc cccacagtgt gggtcttcaa acacttgtag atcgccaatt   1980
   gaagcaaatg ttgccaattg cagtttgaat ggaagtggta gtggaagcaa tcatgggagc   2040
   aatttcctta atggaagtag tgctgctgtg aatgttgaag gaacaaacat ggtcaatgat   2100
   agtgggatag ctgcaaaaga tggtgctgaa aatggaagtg gtagtggaag tggaagtggt   2160
   agtggtagtg gtgttggtgt ggatcaaagt cgatcagctc aacgagaagc tgccttgaat   2220
   aaattccgtc tcaagcgtaa agaaagatgc tttgacaaaa aggtgcgata tcaaagcaga   2280
   aagaagttag cagatcaaag acctcgtgtt cgtgggcaat tcgtgcgcca ggtacgagaa   2340
   aacaaaggaa ggaataccga tagctaa                                       2367
<210> 10
   <211> 2367
   <212> DNA
   <213> Beta vulgaris
<400> 10
   atgaggttga ttcacaaaaa tgaagatggg cccggtgttg ccaagtcggt ggcagagctt     60
   aatcaacata tagttgctgt gaaaaaagaa ggtaggggta gggttgcagg tgaagggcag    120
   gggctttccg aggaggacga actgagaatt attgaggatg gtgaagatgc aaacagcagg    180
   cgttctttga gttctgttca gcttccagtt catactcaca ggcctcagcc acaagtacaa    240
   ccccagggga gagtctgttg ggagaggttt ctccctgttg gatctcctaa ggttttgctc    300
   gtagaaagtg atgactcaac tcgtcatatt gttagtgctt tgctacggaa ttgtagctat    360
   gaagttgtag gggtgccaaa tggcatagaa gcatggaaaa tcttagaaga tttgagcaat    420
   cagattgacc tagttttaac tgaggtagtc acatcaggac tctctggtat aggtcttctg    480
   tccaagataa tgagtcacaa aagctgccag aatactcctg tcattatgat gtcatctcat    540
   gattcgatgg gtttagtctt aaagtgctta tccaagggcg ctgttgactt tctggtgaag    600
   cctataagaa aaaatgaact taaaaacctt tggcagcatg tttggaggag gtgtcacagt    660
   tctagtggta gtggaagtga aagctgtgta aggaatggaa aatccatagg aagcaagagg    720
   gctgaagagt cggacaatga cactgacagc aatgaggaag atgataacag aagcattggt    780
   ttacaagctc gggatggaag tgacaatgga agtgggaccc agagttcatg gacaaaaagg    840
   gctgcagaag ttgagagccc ccaaccacag tctacatggg agcaagcaac tgatccgcct    900
   gatagcactt gtgctcaggt catttatcca atgtctgagg catttgccag cagctggatg    960
   cctggatcca tgcaggaact tgatgtacag gatcatcaat atgacaatgt cccaatggga   1020
   aaggatttgg agattggagt acctagaatt tcagattcac ggctaaatgg accaaacaaa   1080
   aaggttaagt tagcaactac tgctgaggaa aaccaatatt cacagttaga cctcaaccag   1140
   gaaaatgatg gtcgaagttt tgatgaagag aacctggaga tgaataatga taaacctaaa   1200
   agtgagtgga ttaaacaggc tatgaactca ccaggaaaag ttgaagaaca tcatagagga   1260
   aataaagtat ctgatgcacc acccgaaatt tccaaaataa aggacaaagg catgcaacat   1320
   gtcgaggata tgccttctct tgtgctcagt ctgaagaggt tgggtgatat tgcagacacg   1380
   agcactaatg tctcagacca gaatattgtt gggcgttcag agctttcagc cttcaccagg   1440
   tacaattcag gcacaactgg taaccagggt caaacaggta atgttggcag ttgctctcca   1500
   ccaaataata gttcagaagc agcaaagcag tcccattttg atgctccaca tcaaatttcg   1560
   aatagcagta gtaacaataa caatatgggc tctactacta ataagttctt caaaaagcct   1620
   gctatggaca ttgataagac acctgcaaaa tcaacagtca actgttctca tcattcacat   1680
   gtgtttgagc cagtgcaaag ttcccatatg tctaataata accttactgc atctggtaag   1740
   cctggtgttg gctccgtaaa tggtatgctg caagaaaacg taccagtaaa tgctgttctg   1800
   ccgcaagaaa ataacgtgga tcagcagctc aagattcagc gccaccatca ctaccatcat   1860
   tacgatgtcc ataatgtaca gcagctacca aaggtttctg ttcaacataa tatgtccaaa   1920
   agcaaggatg tgacagcacc cccacagtgt gggtcttcaa acacttgtag atcgccaatt   1980
   aaagcaaatg ttgccaattg cagtttgaat ggaagtggta gtggaagcaa tcatgggagc   2040
   aatttcctta atggaagtag tgctgctgtg aatgttgaag gaacaaacat ggtcaatgat   2100
   agtgggatag ctgcaaaaga tggtactgaa aatggaagtg gtagtggaag tggaagtggt   2160
   agtggtagtg gtgttggtgt ggatcaaagt cgatcagctc aacgagaagc tgccttgaat   2220
   aaattccgtc tcaagcgtaa agaaagatgc tttgacaaaa aggtgcgata tcaaagcaga   2280
   aagaagttag cagatcaaag acctcgtgtt cgtgggcaat tcgtgcgcca ggtgcgagaa   2340
   aacaaaggaa ggaataccga tagctaa                                        2367
<210> 11
   <211> 788
   <212> PRT
   <213> Beta vulgaris
<400> 11
   Met Arg Leu Ile His Lys Asn Glu Asp Gly Pro Gly Val Ala Lys Ser
   1               5                   10                  15
   Val Ala Glu Leu Asn Gln His Ile Val Ala Val Lys Lys Glu Gly Arg
   20 25 30
   Gly Arg Val Ala Gly Glu Gly Gln Gly Leu Ser Glu Glu Asp Glu Leu
   35 40 45
   Arg Ile Ile Glu Asp Gly Glu Asp Ala Asn Ser Arg Arg Ser Leu Ser
   50 55 60
   Ser Val Gln Leu Pro Val His Thr His Arg His Gln Pro Gln Val Gln
   65 70 75 80
   Pro Gln Gly Arg Val Cys Trp Glu Arg Phe Leu Pro Val Gly Ser Pro
   85 90 95
   Lys Val Leu Leu Val Glu Ser Asp Asp Ser Thr Arg His Ile Val Ser
   100 105 110
   Ala Leu Leu Arg Lys Cys Ser Tyr Glu Val Val Gly Val Pro Asn Gly
   115 120 125
   Ile Glu Ala Trp Lys Ile Leu Glu Asp Leu Ser Asn Gln Ile Asp Leu
   130 135 140
   Val Leu Thr Glu Val Val Thr Ser Gly Leu Ser Gly Ile Gly Leu Leu
   145 150 155 160
   Ser Lys Ile Met Ser His Lys Ser Cys Gln Asn Thr Pro Val Ile Met
   165 170 175
   Met Ser Ser His Asp Ser Met Gly Leu Val Leu Lys Cys Leu Ser Lys
   180 185 190
   Gly Ala Val Asp Phe Leu Val Lys Pro Ile Arg Lys Asn Glu Leu Lys
   195 200 205
   Asn Leu Trp Gln His Val Trp Arg Arg Cys His Ser Ser Ser Gly Ser
   210 215 220
   Gly Ser Glu Ser Cys Val Arg Asn Gly Lys Ser Ile Gly Ser Lys Arg
   225 230 235 240
   Ala Glu Glu Ser Asp Asn Asp Thr Asp Ile Asn Glu Glu Asp Asp Asn
   245 250 255
   Arg Ser Ile Gly Leu Gln Ala Arg Asp Gly Ser Asp Asn Gly Ser Gly
   260 265 270
   Thr Gln Ser Ser Trp Thr Lys Arg Ala Ala Glu Val Glu Ser Pro Gln
   275 280 285
   Pro Gln Ser Thr Trp Glu Gln Ala Thr Asp Pro Pro Asp Ser Thr Cys
   290 295 300
   Ala Gln Val Ile Tyr Pro Met Ser Glu Ala Phe Ala Ser Ser Trp Met
   305 310 315 320
   Pro Gly Ser Met Gln Glu Leu Asp Gly Gln Asp His Gln Tyr Asp Asn
   325 330 335
   Val Pro Met Gly Lys Asp Leu Glu Ile Gly Val Pro Arg Ile Ser Asp
   340 345 350
   Ser Arg Leu Asn Gly Pro Asn Lys Thr Val Lys Leu Ala Thr Thr Ala
   355 360 365
   Glu Glu Asn Gln Tyr Ser Gln Leu Asp Leu Asn Gln Glu Asn Asp Gly
   370 375 380
   Arg Ser Phe Asp Glu Glu Asn Leu Glu Met Asn Asn Asp Lys Pro Lys
   385 390 395 400
   Ser Glu Trp Ile Lys Gln Ala Met Asn Ser Pro Gly Lys Val Glu Glu
   405 410 415
   His Arg Arg Gly Asn Lys Val Ser Asp Ala Pro Pro Glu Ile Ser Lys
   420 425 430
   Ile Lys Asp Lys Gly Met Gln His Val Glu Asp Met Pro Ser Leu Val
   435 440 445
   Leu Ser Leu Lys Arg Leu Gly Asp Ile Ala Asp Thr Ser Thr Asn Val
   450 455 460
   Ser Asp Gln Asn Ile Val Gly Arg Ser Glu Leu Ser Ala Phe Thr Arg
   465 470 475 480
   Tyr Asn Ser Gly Thr Thr Gly Asn Gln Gly Gln Thr Gly Asn Val Gly
   485 490 495
   Ser Cys Ser Pro Pro Asn Asn Ser Ser Glu Ala Ala Lys Gln Ser His
   500 505 510
   Phe Asp Ala Pro His Gln Ile Ser Asn Ser Ser Ser Asn Asn Asn Asn
   515 520 525
   Met Gly Ser Thr Thr Asn Lys Phe Phe Lys Lys Pro Ala Met Asp Ile
   530 535 540
   Asp Lys Thr Pro Ala Lys Ser Thr Val Asn Cys Ser His His Ser His
   545 550 555 560
   Val Phe Glu Pro Val Gln Ser Ser His Met Ser Asn Asn Asn Leu Thr
   565 570 575
   Ala Ser Gly Lys Pro Gly Val Gly Ser Val Asn Gly Met Leu Gln Glu
   580 585 590
   Asn Val Pro Val Asn Ala Val Leu Pro Gln Glu Asn Asn Val Asp Gln
   595 600 605
   Gln Leu Lys Ile Gln His His His His Tyr His His Tyr Asp Val His
   610 615 620
   Ser Val Gln Gln Leu Pro Lys Val Ser Val Gln His Asn Met Pro Lys
   625 630 635 640
   Ser Lys Asp Val Thr Ala Pro Pro Gln Cys Gly Ser Ser Asn Thr Cys
   645 650 655
   Arg Ser Pro Ile Glu Ala Asn Val Ala Asn Cys Ser Leu Asn Gly Ser
   660 665 670
   Gly Ser Gly Ser Asn His Gly Ser Asn Phe Leu Asn Gly Ser Ser Ala
   675 680 685
   Ala Val Asn Val Glu Gly Thr Asn Met Val Asn Asp Ser Gly Ile Ala
   690 695 700
   Ala Lys Asp Gly Ala Glu Asn Gly Ser Gly Ser Gly Ser Gly Ser Gly
   705 710 715 720
   Ser Gly Ser Gly Val Gly Val Asp Gln Ser Arg Ser Ala Gln Arg Glu
   725 730 735
   Ala Ala Leu Asn Lys Phe Arg Leu Lys Arg Lys Glu Arg Cys Phe Asp
   740 745 750
   Lys Lys Val Arg Tyr Gln Ser Arg Lys Lys Leu Ala Asp Gln Arg Pro
   755 760 765
   Arg Val Arg Gly Gln Phe Val Arg Gln Val Arg Glu Asn Lys Gly Arg
   770 775 780
   Asn Thr Asp Ser
   785
<210> 12
   <211> 788
   <212> PRT
   <213> Beta vulgaris
<400> 12
   Met Arg Leu Ile His Lys Asn Glu Asp Gly Pro Gly Val Ala Lys Ser
   1 5 10 15
   Val Ala Glu Leu Asn Gln His Ile Val Ala Val Lys Lys Glu Gly Arg
   20 25 30
   Gly Arg Val Ala Gly Glu Gly Gln Gly Leu Ser Glu Glu Asp Glu Leu
   35 40 45
   Arg Ile Ile Glu Asp Gly Glu Asp Ala Asn Ser Arg Arg Ser Leu Ser
   50 55 60
   Ser Val Gln Leu Pro Val His Thr His Arg Pro Gln Pro Gln Val Gln
   65 70 75 80
   Pro Gln Gly Arg Val Cys Trp Glu Arg Phe Leu Pro Val Gly Ser Pro
   85 90 95
   Lys Val Leu Leu Val Glu Ser Asp Asp Ser Thr Arg His Ile Val Ser
   100 105 110
   Ala Leu Leu Arg Asn Cys Ser Tyr Glu Val Val Gly Val Pro Asn Gly
   115 120 125
   Ile Glu Ala Trp Lys Ile Leu Glu Asp Leu Ser Asn Gln Ile Asp Leu
   130 135 140
   Val Leu Thr Glu Val Val Thr Ser Gly Leu Ser Gly Ile Gly Leu Leu
   145 150 155 160
   Ser Lys Ile Met Ser His Lys Ser Cys Gln Asn Thr Pro Val Ile Met
   165 170 175
   Met Ser Ser His Asp Ser Met Gly Leu Val Leu Lys Cys Leu Ser Lys
   180 185 190
   Gly Ala Val Asp Phe Leu Val Lys Pro Ile Arg Lys Asn Glu Leu Lys
   195 200 205
   Asn Leu Trp Gln His Val Trp Arg Arg Cys His Ser Ser Ser Gly Ser
   210 215 220
   Gly Ser Glu Ser Cys Val Arg Asn Gly Lys Ser Ile Gly Ser Lys Arg
   225 230 235 240
   Ala Glu Glu Ser Asp Asn Asp Thr Asp Ser Asn Glu Glu Asp Asp Asn
   245 250 255
   Arg Ser Ile Gly Leu Gln Ala Arg Asp Gly Ser Asp Asn Gly Ser Gly
   260 265 270
   Thr Gln Ser Ser Trp Thr Lys Arg Ala Ala Glu Val Glu Ser Pro Gln
   275 280 285
   Pro Gln Ser Thr Trp Glu Gln Ala Thr Asp Pro Pro Asp Ser Thr Cys
   290 295 300
   Ala Gln Val Ile Tyr Pro Met Ser Glu Ala Phe Ala Ser Ser Trp Met
   305 310 315 320
   Pro Gly Ser Met Gln Glu Leu Asp Val Gln Asp His Gln Tyr Asp Asn
   325 330 335
   Val Pro Met Gly Lys Asp Leu Glu Ile Gly Val Pro Arg Ile Ser Asp
   340 345 350
   Ser Arg Leu Asn Gly Pro Asn Lys Lys Val Lys Leu Ala Thr Thr Ala
   355 360 365
   Glu Glu Asn Gln Tyr Ser Gln Leu Asp Leu Asn Gln Glu Asn Asp Gly
   370 375 380
   Arg Ser Phe Asp Glu Glu Asn Leu Glu Met Asn Asn Asp Lys Pro Lys
   385 390 395 400
   Ser Glu Trp Ile Lys Gln Ala Met Asn Ser Pro Gly Lys Val Glu Glu
   405 410 415
   His His Arg Gly Asn Lys Val Ser Asp Ala Pro Pro Glu Ile Ser Lys
   420 425 430
   Ile Lys Asp Lys Gly Met Gln His Val Glu Asp Met Pro Ser Leu Val
   435 440 445
   Leu Ser Leu Lys Arg Leu Gly Asp Ile Ala Asp Thr Ser Thr Asn Val
   450 455 460
   Ser Asp Gln Asn Ile Val Gly Arg Ser Glu Leu Ser Ala Phe Thr Arg
   465 470 475 480
   Tyr Asn Ser Gly Thr Thr Gly Asn Gln Gly Gln Thr Gly Asn Val Gly
   485 490 495
   Ser Cys Ser Pro Pro Asn Asn Ser Ser Glu Ala Ala Lys Gln Ser His
   500 505 510
   Phe Asp Ala Pro His Gln Ile Ser Asn Ser Ser Ser Asn Asn Asn Asn
   515 520 525
   Met Gly Ser Thr Thr Asn Lys Phe Phe Lys Lys Pro Ala Met Asp Ile
   530 535 540
   Asp Lys Thr Pro Ala Lys Ser Thr Val Asn Cys Ser His His Ser His
   545 550 555 560
   Val Phe Glu Pro Val Gln Ser Ser His Met Ser Asn Asn Asn Leu Thr
   565 570 575
   Ala Ser Gly Lys Pro Gly Val Gly Ser Val Asn Gly Met Leu Gln Glu
   580 585 590
   Asn Val Pro Val Asn Ala Val Leu Pro Gln Glu Asn Asn Val Asp Gln
   595 600 605
   Gln Leu Lys Ile Gln Arg His His His Tyr His His Tyr Asp Val His
   610 615 620
   Asn Val Gln Gln Leu Pro Lys Val Ser Val Gln His Asn Met Ser Lys
   625 630 635 640
   Ser Lys Asp Val Thr Ala Pro Pro Gln Cys Gly Ser Ser Asn Thr Cys
   645 650 655
   Arg Ser Pro Ile Lys Ala Asn Val Ala Asn Cys Ser Leu Asn Gly Ser
   660 665 670
   Gly Ser Gly Ser Asn His Gly Ser Asn Phe Leu Asn Gly Ser Ser Ala
   675 680 685
   Ala Val Asn Val Glu Gly Thr Asn Met Val Asn Asp Ser Gly Ile Ala
   690 695 700
   Ala Lys Asp Gly Thr Glu Asn Gly Ser Gly Ser Gly Ser Gly Ser Gly
   705 710 715 720
   Ser Gly Ser Gly Val Gly Val Asp Gln Ser Arg Ser Ala Gln Arg Glu
   725 730 735
   Ala Ala Leu Asn Lys Phe Arg Leu Lys Arg Lys Glu Arg Cys Phe Asp
   740 745 750
   Lys Lys Val Arg Tyr Gln Ser Arg Lys Lys Leu Ala Asp Gln Arg Pro
   755 760 765
   Arg Val Arg Gly Gln Phe Val Arg Gln Val Arg Glu Asn Lys Gly Arg
   770 775 780
   Asn Thr Asp Ser
   785
<210> 13
   <211> 23
   <212> DNA
   <213> Artificial
<220>
   <223> oligonucleotide sequence - Primer PRR7(T1)-F
<400> 13
   gaggtgtcac agtgtaagtg tct 23
<210> 14
   <211> 25
   <212> DNA
   <213> Artificial
<220>
   <223> oligonucleotide sequence - Primer PRR7(T1)-R
<400> 14
   aaagactgct acacgaacca ctaag 25
<210> 15
   <211> 14
   <212> DNA
   <213> Artificial
<220>
   <223> oligonucleotide sequence - Probe PRR7(T1)-VIC
<400> 15
   ctgatggaaa gctg 14
<210> 16
   <211> 14
   <212> DNA
   <213> Artificial
<220>
   <223> oligonucleotide sequence - Probe PRR7(T1)-FAM
<400> 16
   ctgatgaaaa gctg 14
<210> 17
   <211> 23
   <212> DNA
   <213> Artificial
<220>
   <223> oligonucleotide sequence - Primer GJ131(T1)-F
<400> 17
   gcccgtacaa acaaagactt ctc 23
<210> 18
   <211> 24
   <212> DNA
   <213> Artificial
<220>
   <223> oligonucleotide sequence - Primer GJ131(T1)-R
<400> 18
   acgcagaatg ttgatgatga taca 24
<210> 19
   <211> 18
   <212> DNA
   <213> Artificial
<220>
   <223> oligonucleotide sequence - Probe GJ131(T1)-VIC
<400> 19
   tccatctctc cacagctt 18
<210> 20
   <211> 17
   <212> DNA
   <213> Artificial
<220>
   <223> oligonucleotide sequence - Probe GJ131(T1)-FAM
<400> 20
   tccatctccc cacagct 17
<210> 21
   <211> 30
   <212> DNA
   <213> Artificial
<220>
   <223> oligonucleotide sequence - Primer ED031700(T1)-F
<400> 21
   taaaggtggt aattttagag aattttagga 30
<210> 22
   <211> 22
   <212> DNA
   <213> Artificial
<220>
   <223> oligonucleotide sequence - Primer 031700(T1)-R
<400> 22
   gctcgttttg aaaaaatttg gg 22
<210> 23
   <211> 18
   <212> DNA
   <213> Artificial
<220>
   <223> oligonucleotide sequence - Probe ED031700(T1)-VIC
<400> 23
   tttaattcgc atccttct 18
<210> 24
   <211> 17
   <212> DNA
   <213> Artificial
<220>
   <223> oligonucleotide sequence - Probe ED031700(T1)-FAM
<400> 24
   ttaattcgca aacttct 17
<210> 25
   <211> 27
   <212> DNA
   <213> Artificial
<220>
   <223> oligonucleotide sequence - Primer 9_27(T2)-F
<400> 25
   tgccaaaaca cacattgtac ctataca 27
<210> 26
   <211> 19
   <212> DNA
   <213> Artificial
<220>
   <223> oligonucleotide sequence - Primer 9_27(T2)-R
<400> 26
   tgcctctggc tccttgaag 19
<210> 27
   <211> 18
   <212> DNA
   <213> Artificial
<220>
   <223> oligonucleotide sequence - Probe 9_27(T2)-VIC
<400> 27
   catctctaca acactacc 18
<210> 28
   <211> 17
   <212> DNA
   <213> Artificial
<220>
   <223> oligonucleotide sequence - 9_27(T2)-FAM
<400> 28
   atctctacaa gactacc 17
<210> 29
   <211> 22
   <212> DNA
   <213> Artificial
<220>
   <223> oligonucleotide sequence - Primer GJ01(T1)-F
<400> 29
   gaacccagga ttactcgtga gc 22
<210> 30
   <211> 33
   <212> DNA
   <213> Artificial
<220>
   <223> oligonucleotide sequence - Primer GJ01(T1)-R
<400> 30
   aaaagtagaa taaaatgtaa cctcctccat ctc 33
<210> 31
   <211> 17
   <212> DNA
   <213> Artificial
<220>
   <223> oligonucleotide sequence - Probe GJ01(T1)-VIC
<400> 31
   acgcaagata acatcac 17
<210> 32
   <211> 17
   <212> DNA
   <213> Artificial
<220>
   <223> oligonucleotide sequence - Probe GJ01(T1)-FAM
<400> 32
   acgcaagata acgtcac 17
<210> 33
   <211> 27
   <212> DNA
   <213> Artificial
<220>
   <223> oligonucleotide sequence - Primer SELA3977
<400> 33
   cgtgtcgaat attgatttac tgagatc 27
<210> 34
   <211> 26
   <212> DNA
   <213> Artificial
<220>
   <223> oligonucleotide sequence - Primer SELA3988
<400> 34
   taacccatca tgtcttttca acaatc 26
<210> 35
   <211> 23
   <212> DNA
   <213> Artificial
<220>
   <223> oligonucleotide sequence - Primer SELA4442
<400> 35
   aagaataccg agagtttttt ccc 23
<210> 36
   <211> 29
   <212> DNA
   <213> Artificial
<220>
   <223> oligonucleotide sequence - Primer SELA3809
<400> 36
   tcaccaattc tttatatcat atcatgaca 29
<210> 37
   <211> 28
   <212> DNA
   <213> Artificial
<220>
   <223> oligonucleotide sequence - Primer SELA3810
<400> 37
   gagaaaaggg ttttagatgg taagtttt 28
<210> 38
   <211> 28
   <212> DNA
   <213> Artificial
<220>
   <223> oligonucleotide sequence - Primer 3807
<400> 38
   catttgttga agtaggtgat aaggacaa 28
<210> 39
   <211> 21
   <212> DNA
   <213> Artificial
<220>
   <223> oligonucleotide sequence - Primer SELA3766
<400> 39
   tttgatgctt ttttcaggcc a 21
<210> 40
   <211> 24
   <212> DNA
   <213> Artificial
<220>
   <223> oligonucleotide sequence - Primer SELA3769
<400> 40
   aatatgtgtg agaaaatggt ggca 24
<210> 41
   <211> 26
   <212> DNA
   <213> Artificial
<220>
   <223> oligonucleotide sequence - Primer SELA3857
<400> 41
   tccatttgag gagtaggtat gatgag 26
<210> 42
   <211> 21
   <212> DNA
   <213> Artificial
<220>
   <223> oligonucleotide sequence - Primer SELA3860
<400> 42
   tcttgagctg ctgatccacg t 21
<210> 43
   <211> 21
   <212> DNA
   <213> Artificial
<220>
   <223> oligonucleotide sequence - Primer SELA3861
<400> 43
   ctgcatctgg taagcctggt g 21
<210> 44
   <211> 20
   <212> DNA
   <213> Artificial
<220>
   <223> oligonucleotide sequence - Primer SELA3864
<400> 44
   aatgtgaccc gtaaacgcct 20
<210> 45
   <211> 24
   <212> DNA
   <213> Artificial
<220>
   <223> oligonucleotide sequence- forward Primer for BvPRR7 for gene expression analysis
<400> 45
   ttggaggagg tgtcacagtt ctag 24
<210> 46
   <211> 22
   <212> DNA
   <213> Artificial
<220>
   <223> oligonucleotide sequence- reverse Primer for BvPRR7 for gene expression analysis
<400> 46
   tgtcattgtc cgactcttca gc 22
<210> 47
   <211> 19
   <212> DNA
   <213> Artificial
<220>
   <223> oligonucleotide sequence- forward Primer for BvlCDH for gene expression analysis
<400> 47
   cacaccagat gaaggccgt 19
<210> 48
   <211> 18
   <212> DNA
   <213> Artificial
<220>
   <223> oligonucleotide sequence- Reverse Primer for BvlCDH for gene expression analysis
<400> 48
   ccctgaagac cgtgccat 18
<210> 49
   <211> 25
   <212> DNA
   <213> Artificial
<220>
   <223> oligonucleotide sequence - Primer PRR7(T6)-F
<400> 49
   gctatcggta ttccttcctt tgttt 25
<210> 50
   <211> 19
   <212> DNA
   <213> Artificial
<220>
   <223> oligonucleotide sequence - Primer PRR7(T6)-R
<400> 50
   ctcgtgttcg tgggcaatt 19
<210> 51
   <211> 16
   <212> DNA
   <213> Artificial
<220>
   <223> oligonucleotide sequence - Probe PRR7(T6)-VIC
<400> 51
   ctcgtacctg gcgcac 16
<210> 52
   <211> 16
   <212> DNA
   <213> Artificial
<220>
   <223> oligonucleotide sequence - Probe PRR7(T6)-FAM
<400> 52
   ctcgcacctg gcgcac 16
<210> 53
   <211> 3672
   <212> DNA
   <213> Beta vulgaris
<400> 53
   aaaaaatttc cataaaggtg tatttattag gcgtagcggt tgatttccca aatcattatc 60
   cttgcgcaaa ttgcataatg gagatatttg gtgttgacgt ggggccataa ccataatagg 120
   gggtcaaaaa caaaactaca agggttaaaa tcgtcacaat attaaacaag catctcactg 180
   gtcacttttt ttttacctat taaagaacaa acctttaact ctcctcacaa tctgccacgt 240
   gtcgaataat tgatttactg agatcaattt agatcctctc ccttagactc ttctcagtac 300
   agctttagat ctcaacctcc atgtcagcaa agttacctta cgtgtcatcc tacgtggcct 360
   ctccttctac ccctcactcc tccacgtcaa cattttcctc caaaattaaa aaatcatttt 420
   tttattatat ttacttgaat gtatataata atgtctactg atcttcttct ttagaactat 480
   ctccttctct cattggaacc tcaaaatcat tcttatttta tttcgagaaa aggaaaaaaa 540
   agcacatctg ttttgaagat taatttgtgg attattattg agcttcatcg tatttaaaaa 600
   acatagtaaa agttctttcc tcatttgtct ttttattcat ctaatttttt tagtgaagaa 660
   ccctaatttt gtttgtgaat tctcaagttc aagttttgat ttgggtattt tttttgatga 720
   aatttgtgca gctgtaggat gttatcgtgc tgagaaaagg gttttagatg gtaagttttt 780
   tttttctttg atttctctct cctacttttt tttttgtttt gctttagata atactgtcat 840
   gatatgatat aaagaattgg tgatttgggt agtttattta acctatgatt atgtgttatt 900
   tgttttgatc tttcaattta tctggtactg tgtgtatata tgttttgttt ttcttcaagt 960
   atttggttat tattgaagtg ggtaattagg aatttgctac taatctatgg atttgggttc 1020
   tgttgtgatt aatttactat agatttgagg tttaatttat gttttatagg ttagaaaagg 1080
   aaatcaatga tttgtttgtg aatttgagta gattgtttgt tagtgtgtgt atgatgatat 1140
   taacttccat tatttcttcc ccaaattagg ggtaattgat ggttttttgc ataccgaagg 1200
   cgtattctct ttgatgatgg agtgattgtt gaaaagacat gatgggttaa agttgcagga 1260
   ttatttcatt tcaataaaca taattgatca atttggatct gttgaatgag gttgattcac 1320
   aaaaatgaag atgggcccgg tgttgccaag tcggtggcag agcttaatca acatatagtt 1380
   gctgtgaaaa aagaaggtag gggtagggtt gcaggtgaag ggcaggggct ttccgaggag 1440
   gacgaactga gaattattga ggatggtgaa gatgcaaaca gcaggcgttc tttgagttct 1500
   gttcagcttc cagttcatac tcacaggcct cagccacaag tacaacccca ggggagagtc 1560
   tgttgggaga ggtttctccc tgttggatct cctaaggttt tgctcgtaga aagtgatgac 1620
   tcaactcgtc atattgttag tgctttgcta cggaattgta gctatgaagt tgtaggggtg 1680
   ccaaatggca tagaagcatg gaaaatctta gaagatttga gcaatcagat tgacctagtt 1740
   ttaactgagg tagtcacatc aggactctct ggtataggtc ttctgtccaa gataatgagt 1800
   cacaaaagct gccagaatac tcctgtcatt atgatgtcat ctcatgattc gatgggttta 1860
   gtcttaaagt gcttatccaa gggcgctgtt gactttctgg tgaagcctat aagaaaaaat 1920
   gaacttaaaa acctttggca gcatgtttgg aggaggtgtc acagttctag tggtagtgga 1980
   agtgaaagct gtgtaaggaa tggaaaatcc ataggaagca agagggctga agagtcggac 2040
   aatgacactg acagcaatga ggaagatgat aacagaagca ttggtttaca agctcgggat 2100
   ggaagtgaca atggaagtgg gacccagagt tcatggacaa aaagggctgc agaagttgag 2160
   agcccccaac cacagtctac atgggagcaa gcaactgatc cgcctgatag cacttgtgct 2220
   caggtcattt atccaatgtc tgaggcattt gccagcagct ggatgcctgg atccatgcag 2280
   gaacttgatg tacaggatca tcaatatgac aatgtcccaa tgggaaagga tttggagatt 2340
   ggagtaccta gaatttcaga ttcacggcta aatggaccaa acaaaaaggt taagttagca 2400
   actactgctg aggaaaacca atattcacag ttagacctca accaggaaaa tgatggtcga 2460
   agttttgatg aagagaacct ggagatgaat aatgataaac ctaaaagtga gtggattaaa 2520
   caggctatga actcaccagg aaaagttgaa gaacatcata gaggaaataa agtatctgat 2580
   gcaccacccg aaatttccaa aataaaggac aaaggcatgc aacatgtcga ggatatgcct 2640
   tctcttgtgc tcagtctgaa gaggttgggt gatattgcag acacgagcac taatgtctca 2700
   gaccagaata ttgttgggcg ttcagagctt tcagccttca ccaggtacaa ttcaggcaca 2760
   actggtaacc agggtcaaac aggtaatgtt ggcagttgct ctccaccaaa taatagttca 2820
   gaagcagcaa agcagtccca ttttgatgct ccacatcaaa tttcgaatag cagtagtaac 2880
   aataacaata tgggctctac tactaataag ttcttcaaaa agcctgctat ggacattgat 2940
   aagacacctg caaaatcaac agtcaactgt tctcatcatt cacatgtgtt tgagccagtg 3000
   caaagttccc atatgtctaa taataacctt actgcatctg gtaagcctgg tgttggctcc 3060
   gtaaatggta tgctgcaaga aaacgtacca gtaaatgctg ttctgccgca agaaaataac 3120
   gtggatcagc agctcaagat tcagcgccac catcactacc atcattacga tgtccataat 3180
   gtacagcagc taccaaaggt ttctgttcaa cataatatgt ccaaaagcaa ggatgtgaca 3240
   gcacccccac agtgtgggtc ttcaaacact tgtagatcgc caattaaagc aaatgttgcc 3300
   aattgcagtt tgaatggaag tggtagtgga agcaatcatg ggagcaattt ccttaatgga 3360
   agtagtgctg ctgtgaatgt tgaaggaaca aacatggtca atgatagtgg gatagctgca 3420
   aaagatggta ctgaaaatgg aagtggtagt ggaagtggaa gtggtagtgg tagtggtgtt 3480
   ggtgtggatc aaagtcgatc agctcaacga gaagctgcct tgaataaatt ccgtctcaag 3540
   cgtaaagaaa gatgctttga caaaaaggtg cgatatcaaa gcagaaagaa gttagcagat 3600
   caaagacctc gtgttcgtgg gcaattcgtg cgccaggtgc gagaaaacaa aggaaggaat 3660
   accgatagct aa 3672
<210> 54
   <211> 4543
   <212> DNA
   <213> Beta vulgaris
<400> 54
   aaaaaatttc cataaaggtg tatttattag gcgtagcggt tgatttccca aatcattatc      60
   cttgcgcaaa ttgcataatg gagatatttg gtgttgacgt ggggccataa ccataatagg     120
   gggtcaaaaa caaaactaca agggttaaaa tcgtcacaat attaaacaag catctcactg     180
   gtcacttttt ttttacctat taaagaacaa acctttaact ctcctcacaa tctgccacgt     240
   gtcgaataat tgatttactg agatcaattt agatcctctc ccttagactc ttctcagtac     300
   agctttagat ctcaacctcc atgtcagcaa agttacctta cgtgtcatcc tacgtggcct     360
   ctccttctac ccctcactcc tccacgtcaa cattttcctc caaaattaaa aaatcatttt 420
   tttattatat ttacttgaat gtatataata atgtctactg atcttcttct ttagaactat 480
   ctccttctct cattggaacc tcaaaatcat tcttatttta tttcgagaaa aggaaaaaaa 540
   agcacatctg ttttgaagat taatttgtgg attattattg agcttcatcg tatttaaaaa 600
   acatagtaaa agttctttcc tcatttgtct ttttattcat ctaatttttt tagtgaagaa 660
   ccctaatttt gtttgtgaat tctcaagttc aagttttgat ttgggtattt tttttgatga 720
   aatttgtgca gctgtaggat gttatcgtgc tgagaaaagg gttttagatg gtaagttttt 780
   tttttctttg atttctctct cctacttttt tttttgtttt gctttagata atactgtcat 840
   gatatgatat aaagaattgg tgatttgggt agtttattta acctatgatt atgtgttatt 900
   tgttttgatc tttcaattta tctggtactg tgtgtatata tgttttgttt ttcttcaagt 960
   atttggttat tattgaagtg ggtaattagg aatttgctac taatctatgg atttgggttc 1020
   tgttgtgatt aatttactat agatttgagg tttaatttat gttttatagg ttagaaaagg 1080
   aaatcaatga tttgtttgtg aatttgagta gattgtttgt tagtgtgtgt atgatgatat 1140
   taacttccat tatttcttcc ccaaattagg ggtaattgat ggttttttgc ataccgaagg 1200
   cgtattctct ttgatgatgg agtgattgtt gaaaagacat gatgggttaa agttgcagga 1260
   ttatttcatt tcaataaaca taattgatca atttggatct gttgaatgag gttgattcac 1320
   aaaaatgaag atgggcccgg tgttgccaag tcggtggcag agcttaatca acatatagtt 1380
   gctgtgaaaa aagaaggtag gggtagggtt gcaggtgaag ggcaggggct ttccgaggag 1440
   gacgaactga gaattattga ggatggtgaa gatgcaaaca gcaggcgttc tttgagttct 1500
   gttcagcttc cagttcatac tcacaggcct cagccacaag tacaacccca ggggagagtc 1560
   tgttgggaga ggtttctccc tgttggatct cctaaggttt tgctcgtaga aagtgatgac 1620
   tcaactcgtc atattgttag tgctttgcta cggaattgta gctatgaagt tgtaggggtg 1680
   ccaaatggca tagaagcatg gaaaatctta gaagatttga gcaatcagat tgacctagtt 1740
   ttaactgagg tagtcacatc aggactctct ggtataggtc ttctgtccaa gataatgagt 1800
   cacaaaagct gccagaatac tcctgtcatt atgatgtcat ctcatgattc gatgggttta 1860
   gtcttaaagt gcttatccaa gggcgctgtt gactttctgg tgaagcctat aagaaaaaat 1920
   gaacttaaaa acctttggca gcatgtttgg aggaggtgtc acagttctag tggtagtgga 1980
   agtgaaagct gtgtaaggaa tggaaaatcc ataggaagca agagggctga agagtcggac 2040
   aatgacactg acagcaatga ggaagatgat aacagaagca ttggtttaca agctcgggat 2100
   ggaagtgaca atggaagtgg gacccagagt tcatggacaa aaagggctgc agaagttgag 2160
   agcccccaac cacagtctac atgggagcaa gcaactgatc cgcctgatag cacttgtgct 2220
   caggtcattt atccaatgtc tgaggcattt gccagcagct ggatgcctgg atccatgcag 2280
   gaacttgatg tacaggatca tcaatatgac aatgtcccaa tgggaaagga tttggagatt 2340
   ggagtaccta gaatttcaga ttcacggcta aatggaccaa acaaaaaggt taagttagca 2400
   actactgctg aggaaaacca atattcacag ttagacctca accaggaaaa tgatggtcga   2460
   agttttgatg aagagaacct ggagatgaat aatgataaac ctaaaagtga gtggattaaa   2520
   caggctatga actcaccagg aaaagttgaa gaacatcata gaggaaataa agtatctgat   2580
   gcaccacccg aaatttccaa aataaaggac aaaggcatgc aacatgtcga ggatatgcct   2640
   tctcttgtgc tcagtctgaa gaggttgggt gatattgcag acacgagcac taatgtctca   2700
   gaccagaata ttgttgggcg ttcagagctt tcagccttca ccaggtacaa ttcaggcaca   2760
   actggtaacc agggtcaaac aggtaatgtt ggcagttgct ctccaccaaa taatagttca   2820
   gaagcagcaa agcagtccca ttttgatgct ccacatcaaa tttcgaatag cagtagtaac   2880
   aataacaata tgggctctac tactaataag ttcttcaaaa agcctgctat ggacattgat   2940
   aagacacctg caaaatcaac agtcaactgt tctcatcatt cacatgtgtt tgagccagtg   3000
   caaagttccc atatgtctaa taataacctt actgcatctg gtaagcctgg tgttggctcc   3060
   gtaaatggta tgctgcaaga aaacgtacca gtaaatgctg ttctgccgca agaaaataac   3120
   gtggatcagc agctcaagat tcagcgccac catcactacc atcattacga tgtccataat   3180
   gtacagcagc taccaaaggt ttctgttcaa cataatatgt ccaaaagcaa ggatgtgaca   3240
   gcacccccac agtgtgggtc ttcaaacact tgtagatcgc caattaaagc aaatgttgcc   3300
   aattgcagtt tgaatggaag tggtagtgga agcaatcatg ggagcaattt ccttaatgga   3360
   agtagtgctg ctgtgaatgt tgaaggaaca aacatggtca atgatagtgg gatagctgca   3420
   aaagatggta ctgaaaatgg aagtggtagt ggaagtggaa gtggtagtgg tagtggtgtt   3480
   ggtgtggatc aaagtcgatc agctcaacga gaagctgcct tgaataaatt ccgtctcaag   3540
   cgtaaagaaa gatgctttga caaaaaggtg cgatatcaaa gcagaaagaa gttagcagat   3600
   caaagacctc gtgttcgtgg gcaattcgtg cgccaggtgc gagaaaacaa aggaaggaat   3660
   accgatagct aacaccaatt ctttccacaa gttgctgccg agatcattta tgccactctg   3720
   atatcagctg tcttcatatg tacaaatttc gaattttatg tgtgcatgag gtgctaaata   3780
   ctgtcaaacc tcagtgattc tgtttggttt aggctgtaga aagacatctt ttcctttgtg   3840
   ttttcatggt tcttattttg agctgtgttc actacttttt ataacatggt agccccaggt   3900
   tgcctttgga aataagcttt tccttaaagg tgtgatgcat ataatcttgt ttggtgttag   3960
   attatatgat catttcttca ggcgtttacg ggtcacattt tccgggatcc tttcaaacgc   4020
   gattccggaa acaatggctc atattttctt ttggtttcaa ggagaaggct atttaaaaca   4080
   gaaaagattt aggttacaga aatcagtgat gaagcaatga gtttcattat agaataggtg   4140
   gaagtagggg gtgttttttc cgtactcttg agatagaaag tggggataga ttctttggac   4200
   tcgtcagaaa ggaataatat aggagttgtc tacctttttc atttttagtt cttgtaagag   4260
   ttttattcca cttccatttt agtaaaattt aggagttgta aggacgtgta aagagaatct   4320
   gccatccaga ttttaaccga cggtaaattt gttcttttca tgttttctca agtaactata   4380
   atgttttcat cgaatctaga gggattttct aatgtgtacc tgatagaggc acacagtaac   4440
   aataatataa gtacatatat tctttaagaa taatgacata gtaattatat ttttaataca   4500
   aataaaagat gtccttatgt aatgaaacaa ataacttttc ctt 4543
<210> 55
   <211> 23
   <212> DNA
   <213> Artificial
<220>
   <223> oligonucleotide sequence - Primer 1r22(T1)-F
<400> 55
   gataaattct gacccgcatc aca 23
<210> 56
   <211> 28
   <212> DNA
   <213> Artificial
<220>
   <223> oligonucleotide sequence - Primer 1r22(T1)-R
<400> 56
   ggactgagtt gataataatc aactttcc 28
<210> 57
   <211> 14
   <212> DNA
   <213> Artificial
<220>
   <223> oligonucleotide sequence - Probe 1r22(T1)-VIC
<400> 57
   cctagcgcaa tttc 14
<210> 58
   <211> 15
   <212> DNA
   <213> Artificial
<220>
   <223> oligonucleotide sequence - probe 1r22(T1)-FAM
<400> 58
   agctagcgcc caatt 15